# EUROPEAN PATENT SPECIFICATION

(11) **EP 1 613 772 B1**
(45) Date of publication and mention of the grant of the patent: **03.06.2009**
(21) Application number: 04727459.2
(22) Date of filing: 14.04.2004
(51) Int. Cl.: C12Q 1/68

(54) **METHOD FOR DETERMINING SOIL CONDITION**
VERFAHREN ZUR BESTIMMUNG DES BODENZUSTANDS
PROCEDE PERMETTANT DE DETERMINER LA SANTE ECOLOGIQUE OU AGRICOLE D'UN SOL

(30) Priority: 14.04.2003 NL 1023180
(43) Date of publication of application: 11.01.2006
(73) Proprietor: Bedrijfslaboratorium voor Grond- en Gewasonderzoek B.V., 6861 WN Oosterbeek (NL)
(72) Inventor: HELDER, Johannes, NL-6707 AP Wageningen (NL); VAN DEN ELSEN, Sven, Johannes, Josephus, NL-5427 AW Boekel (NL); BONGERS, Alberthus, Maria, Theodorus, NL-6701 CW Wageningen (NL); VAN DER WURFF, Adrianus, Wilhelmus, Gerardus, NL-6708 CP Wageningen (NL); BAKKER, Jaap, NL-6704 PD Wageningen (NL); KAMMENGA, Jan, Edward, NL-6708 SP Wageningen (NL)
(74) Representative: Winckels, Johannes Hubertus F.
(86) International application number: PCT/NL2004/000247
(87) International publication number: WO 2004/090164

(56) References cited:
- US-A1- 2002 065 609
- GRANT WARWICK N: "Genetic variation in parasitic nematodes and its implications" INTERNATIONAL JOURNAL FOR PARASITOLOGY, vol. 24, no. 6, 1994, pages 821-830, XP002441283 ISSN: 0020-7519
- APTE AARON N ET AL: "Anchor-ligated cDNA libraries: A technique for generating cDNA library for the immediate cloning of the 5' ends of mRNAs" BIOTECHNIQUES, vol. 15, no. 5, 1993, pages 890-893, XP002149618 ISSN: 0736-6205
- TRIGA DIMITRA ET AL: "Gel electrophoretic restriction fragment length polymorphism analysis of DNA derived from individual nematodes, using the PhastSystem" ELECTROPHORESIS, vol. 20, no. 6, June 1999 (1999-06), pages 1274-1279, XP002441285 ISSN: 0173-0835
- REID ALEXANDER P ET AL: "Molecular taxonomy and phylogeny of entomopathogenic nematode species (Rhabditida: Steinernematidae) by RFLP analysis of the ITS region of the ribosomal DNA repeat unit" SYSTEMATIC PARASITOLOGY, vol. 37, no. 3, 1997, pages 187-193, XP002441286 ISSN: 0165-5752
- FOLKERTSMA ROLF T ET AL: "Cluster analysis of 36 Globodera pallida field populations using two sets of molecular markers" EUROPEAN JOURNAL OF PLANT PATHOLOGY, vol. 102, no. 6, 1996, pages 577-584, XP002441287 ISSN: 0929-1873
- BONGERS TOM ET AL: "Nematode community structure as a bioindicator in environmental monitoring" TRENDS IN ECOLOGY AND EVOLUTION, vol. 14, no. 6, June 1999 (1999-06), pages 224-228, XP002441288 ISSN: 0169-5347
- FLOYD ROBIN ET AL: "Molecular barcodes for soil nematode identification." MOLECULAR ECOLOGY. ENGLAND APR 2002, vol. 11, no. 4, April 2002 (2002-04), pages 839-850, XP002272397 ISSN: 0962-1083
- DE GOEDE R G M ET AL: "A nematode reference database as an instrument for biological soil assessment: A case study from the Netherlands" NEMATOLOGY, vol. 4, no. 2, 2002, page 150, XP008028294 Fourth International Congress of Nematology Programme and Abstracts;Tenerife, Canary Islands, Spain; June 08-13, 2002 ISSN: 1388-5545
- BLAXTER ET AL.: "A molecular evolutionary framework for the phylum Nematoda" NATURE, vol. 392, 5 March 1998 (1998-03-05), pages 71-75, XP002272398 cited in the application

## Description

### FIELD OF THE INVENTION

The present invention relates to a method for determining a life strategy of a nematode, to a method for determining biodiversity and to a method for determining soil health ecologically and agriculturally. In particular, the present invention relates to methods for determining the nematode fauna in the soil in which nematode taxa are identified on the basis of one or more characteristic nucleotides in the ribosomal RNA ciatron.

### BACKGROUND OF THE INVENTION

At a large number of locations in the world, polluted soils cause considerable environmental and health problems. The cleaning of polluted soils is a very expensive business. For this reason, at any moment, only a limited number of locations are eligible for sanitation and a priority indication will need to take place in which the soil needs to be characterized with regard to nature and extent of pollution..In addition, it is desired, for instance after soil sanitation, to be able to determine to what extent the measures taken have indeed had the desired effect on the soil health.

A polluted soil may be characterized in various manners. A pollution may, for instance, have a chemical nature, characterize by the presence of certain toxic substances. However, certain toxic substances may also be naturally present in a soil. The presence of a damage, for instance an anthropogenic damage of the (biological) soil environment, that is, of the physical, chemical and biological soil environment, or the presence of a threat to public health is also important for determining the necessity and urgency of sanitation.

Whether the soil environment has been damaged can often only be determined if a corresponding reference is known, or if the situation before and after pollution is known, Physical, chemical and biological parameters can be used to describe the effect of disturbances on the soil. Of these, biological parameters are the most telling because they potentially reflect the ultimate combined effect of all disturbances.

However, the soil health condition, that is, the ecological condition of the soil (micro)flora and fauna, is often difficult to determine. Yet it is desired that, at any moment, an indication of the condition of the soil environment can be obtained which represents the biological condition of this soil.

It is generally assumed that healthy soils have a certain degree of resilience, that is, that the biological community in this soil has the ability to return to its original condition after disturbance or stress (Van Bruggen and Semenov (2000) Appl. Soil Ecol. 15:13-24; O'Neill et al. (1986) In: A Hierarchical Concept of Ecosystems. Princeton Univ. Press, Princeton, USA). Also, the stability of the ecosystem is considered a measure for the resistance to multiple disturbances in the long term.

For determining the health of a soil, a number of measuring methods have been developed. For instance, various bioassays are known and various biosensors and/or bioindicators can be used. Two types of bioassays for the soil health are distinguished: one category of assays uses exotic test organisms, while the other type uses organisms which are naturally present in a soil, native (or endemic) test organisms.

With a bioassay, the biological effects of toxic substances can be made visible by measuring the effect of a particular environmentally polluting substance on an (exotic) test organism. An example is the use of the luminescent bacteria *Vibrio fischeri*; a soil disturbance-induced reduction of the respiration of this bacterial species results in a light signal (Microtox, Coastal Bioanalysts Inc., Gloucester, USA). However, bioassays using exotic test organisms are ecologically hardly relevant and - at best - only suitable for measuring acute stress. Ecological relevance is, in this context, understood to mean the relevance for the condition of the soil life as a whole, where the effect on only one single test organism by definition provides no insight into the condition of the ecosystem. The influence of soil pollution on biological features of the soil is therefore preferably determined on the basis of the state of the native flora or fauna because the diversity and condition of the native soil organisms reflect both the acute and chronic effects of any soil disturbance.

A number of biosensor systems are known for determining soil health in which native microbial populations are characterized. Examples of this are *inter alia* a system for microbial substrate use (MicroStation 3E System, Biolog, Hayward, USA) and a system for determination of a microbial fatty acid methyl ester profile (FAME profile; Microbial ID Inc., Newark, USA).

A drawback of tests based on the substrate use of microorganisms (for instance the Biolog system) is that only a small part of the microbial populations can be grown on artificial media (often about 1%). It also holds true for the MIDI-FAME method that the first step relates to the growing of the bacteria (with above-mentioned implications). In addition, the taxonomic resolution of the FAME method is not very high, and a distinction between bacterial genera is by no means always possible. A good indication of the actual ecological and biological diversity of microorganisms in a soil is therefore not achieved with this method.

In order to get an impression of the health of the environment, also, certain bioindicators can be measured. In this context, bioindicators are, for instance, certain biological species (indicator species) with a small tolerance range which disappear first when the condition of the environment changes. For instance, lichens are good bioindicators for measuring air pollution. However, as said before, the use of individual species as indicators for the soil health has little ecological relevance for the soil life as a whole. In addition, the occurrence of a species is often soil-specific so that such an indicator can only be used in a limited amount of soil types.

A complete biodiversity study does provide the possibility to determine whether soils have a certain degree of resilience, and whether the biological community in these soils has the ability to return to the original condition after disturbance or stress. In principle, a biodiversity study can be carried out for any type of soil and is in fact a biological indicator system in the most comprehensive form. In such a study, the occurrence (in number and biomass) of, preferably, all different organisms (worms, potworms, mites, nematodes, bacteria, mushrooms and toadstools, mycorrhiza, fungi, springtails and protozoa) which are found in the respective soil is determined, which results in a virtually complete description of the structure of the biological community.

Although a complete biodiversity study can provide important insight into the trophic interaction between the different organisms in the soil and therefore has considerable ecological relevance, it is not suitable for a use in which, in a simple manner, a priority for sanitation can be assigned to a particular polluted soil. The methods currently available for determining the ecological and biological diversity of the flora and fauna are too slow and too labor-intensive for this.

It is known from various studies that nematodes may very suitably be used as 'native' biosensors for measuring soil health (see for instance Doran et al. (1996) In: D.L. Sparks (ed.): Advances in Agronomy, Vol.56. Acad. Press Inc., San Diego, USA; Bongers (1990) Oecologia 83: 14-19). In this context, the trophic diversity of nematodes is important. Within the phylum, bacterivores, fungivores, carnivores (nematodes eating other nematodes), plant parasites and animal parasites are represented, although this classification is not strict; fungiphagous nematodes may be facultatively plant-parasitic. Bacterivores, fungivores and carnivores are together referred to by the term 'free-living nematodes'.

It is known that, if a soil disturbance influences only one specific group of organisms, for instance the fungi, this disturbance will have repercussions on the nematode community (in this case - initially - the fungivorous nematodes).

It has been found that the composition of the nematode fauna which is found in relation to particular disturbances, such as the acute exposure to toxic compounds including agrochemicals or an increasing availability of easily degradable organic substances (manuring), shows specific patterns. It is also known that these patterns can be quantitatively described.

In general, a pollution of the soil results in a shift of the composition of the population towards nematode species with an opportunistic life strategy, also referred to as 'colonizers', while a decrease in the pollution -results in a shift to species with a persistent life strategy, also referred to as 'persisters'.

In order to determine whether soils have a certain degree of resilience, and whether the biological community in this soil has the ability to return, to the original condition after disturbance or stress, it is, for this reason, possible to determine the composition of the population of the different nematode species. The life strategy of nematodes in the soil therefore reflects the soil health.

On the basis of morphological identification (down to family level) of the different nematodes in a soil and the subsequent classification based on their life strategies and the use of arithmetic algorithms, the composition of the free-living nematode population can be translated into a quantitative indicator for soil health, the 'Maturity Index' (Bongers (1990) Oecologia 83:14-19; Bongers & Ferris (1999) Trends Ecol. Evol. 14:224-228). Parallel to this, also, the so-called plant parasite index (PPI) has been defined. In this, only obligate plant-parasitic nematodes are included (Bongers (1990) Oecologia 83:14-19).

However, there are factors which make the use of nematodes as biosensors or indicators for soil health more difficult.

Nematodes are identified and have also been classified according to subtle morphological features. For instance, the until recently very relevant division between the classes of *Adenophorea* and *Secernentea* is *inter alia* based on the occurrence or absence of phasmids, small sensory organs in or dorsal to the lateral field on the back half of the body (sometimes perceivable under a light microscope at a magnification of 1000x). Another striking example is the family of the *Rhabditidae* (including *Caenorhabditis elegans*); for a number of genera, only the males can be identified.

However, there are very few experts-who are competent in the morphological identification of nematodes and are capable of completely analyzing a nematode population in a particular soil sample on the basis of solely morphological features. Here, it should also be noted that, in a large number of cases, nematodes in larval stages (that is, non-mature specimens) cannot be identified, not even by taxonomic experts.

A routine analysis of one single sample, in which a maximum of approximately 150 (mature) individuals are characterized, takes an expert 3-4 hours on average. Such a sampling also has a considerable sampling error. In addition, systematics on the basis of morphological features always leads to changes in the classification system (Fortuner and Raski (1987) Revue Nématol. 10:257-267). See for a taxonomic classification of different families within the phylum *Nematoda* according to morphological features for instance Maggenti, A.R (1981) General Nematology, Springer Verlag, New York, USA. Subclassification down to the species/genus level is necessary because, in certain families, 'colonizers' and 'persisters' are closely related to each other.

It is known that certain drawbacks of morphological identification methods can be obviated by use of molecular identification methods. For instance, sequence data of the so-called small subunit (SSU) gene located in the ribosomal RNA cistron (Blaxter et al. (1998) Nature 392:71-75; De Ley and Blaxter (2001) In: Biology of Nematodes (ed. D.L. Lee). London: Harwood Academic Publishers) or mitochondrial sequence data (Blouin et al., 1998, Mol. Biol. Evol. 15:1719-1727) can be used to determine the evolutionary relationship and/or the genetic diversity of nematodes. Such methods form the basis for a systematics on the basis of molecular sequence data. See for a taxonomic classification of different genera within the phylum *Nematoda* according to molecular features the public domain information of the NCBI Web pages (www.ncbi.nlm.nih.gov/taxonomy).

Certain researchers have attempted to couple information regarding the trophic type, that is, the manner of feeding of the nematode (for instance carnivory, bacterivory, fungivory, plant pathogenic, animal pathogenic) to molecular sequence data. However, it is known that different trophic types have originated multiple times during the evolution as a result of independent parallel evolution. For this reason, the coupling of a trophic type to a molecular feature has been found not well possible (Blaxter et al. (1998) Nature 392:71-75). Thus, there is no univocal relation between SSU ribosomal DNA sequences and the trophic type of nematodes. Because the taxonomic classification of different families within the phylum *Nematoda* with known trophic types and classified according to morphologically systematic methods does not necessarily correspond to the classification according to molecularly systematic methods, by means of molecular features, no ecological property can be assigned to the organisms with any reliability.

In addition, the trophic type to which a particular nematode can be said to belong bears no relationship to the 'Maturity Index' which can be a measure for the soil health.

Further, the determination of solely the genetic diversity does provide insight into the absolute amount of taxa (or molecular operational taxonomic units) occurring in a particular soil, but does not provide insight into the biological functioning of these species at different trophic levels. The reason for this is that the phylogenetic relationship between species does not provide direct information about the niche or the function of a particular species in an ecological community. An example of this can be found in Table 5, where different species from a same genus can have completely different life strategies, expressed as a *c*-*p* value. So, currently, the ecological and biological diversity cannot be determined by means of molecular methods and for this reason, to date, the soil health of a sample cannot be determined with molecular techniques.

Currently, there is a great need for a method for determining the soil health which provides a good approximation of the ecological and biological diversity of the flora and faunain a soil and which can play a role in drawing up a priority list for sanitation of soil pollution or in determining the suitability of a soil for growing a particular crop.

It is the object of the present invention to provide a biosensor or bioindicator system on the basis of nematodes by means of which the soil health can quickly and univocally be determined ecologically and agriculturally.

### SUMMARY OF THE INVENTION

It has surprisingly been found that specific molecular features which can be found in the nucleotide sequence of the small subunit (SSU) rRNA of nematodes have a correlation with particular life strategies, allowing an ecological classification of nematodes according to molecular features. On the basis thereof, the invention provides a biosensor system comprising a data file in which the nucleotide sequence of the SSU rRNA of nematodes is correlated with a specific life strategy. Such a biosensor system can therefore very suitably be used in a method for determining the ecological soil health.

Further, the present inventers have determined the ribosomal RNA sequences of a large number of nematodes and have, after comparison of these nucleotide sequences, found single nucleotide polymorphisms (SNPs) on the basis whereof nematodes at low taxonomic level, that is, lower taxonomic groups of nematodes, can be distinguished from one another and identified (see Table 7). These SNPs can be used to distinguish particular species or genera of nematodes from other, most closely related species or genera, and are characteristic of the respective species or the respective genus. For this reason, these SNPs can be used to identify specific nematode species or genera.

Herein described is the use of at least one of the single nucleotide polymorphisams (SNPs) of a ribosomal nucleic acid according to Table 7 as a marker for detecting lower taxonomic groups of nematodes, for then determining a life strategy of a nematode, for then determining the biodiversity of a soil sample, and/or for then determining the soil health.

Herein is further described a method for detecting a lower taxonomic group of nematodes in a sample, comprising the steps of:
a) providing a ribosomal nucleic acid sample of the nematodes present in this sample;
b) detecting a ribosomal nucleic acid with at least one single nucleotide polymorphism (SNP) according to Table 7 in the nucleic acid sample for demonstrating the presence of the respective lower taxonomic group of nematodes in the sample.

As a sample, a soil sample or another sample in which the presence of the respective nematode is suspected can be taken. This sample may also comprise a mixed population of different nematodes, or a sample in which one single nematode is present. In the latter case, the identification of a nematode is involved.

In a preferred embodiment of a method for detecting a lower taxonomic group of nematodes in a sample, one or more specific species of plant-parasitic nematodes are detected in a sample. This preferred embodiment has the advantage that it enables the determination, for instance in the soil, of the presence of particular species of plant-parasitic nematodes, which method is important to agriculture.

In a further preferred embodiment of a method for detecting a lower taxonomic group of nematodes in a sample, one or more specific genera of free-living nematodes are detected, and more preferably, multiple genera of free-living nematodes are detected in a sample. Such a preferred embodiment has the advantage that it enables the obtainment of a complete population composition of the population of the free-living nematode population at the level of genera of nematodes on the basis of which, in addition to the biodiversity, the soil health can also be determined.

It is further possible to determine the relative occurrence of individual lower taxonomic groups .(for instance species or genera) of nematodes in a mixed population of different nematodes by providing a ribosomal nucleic acid sample of the nematodes in a sample with a mixed population of different nematodes, followed by detecting at least one single nucleotide polymorphism (SNP) in the ribosomal nucleic acid and, then, to determine the quantitative contribution of the nucleic acid in which the at least one single nucleotide polymorphism (SNP) occurs in the total ribosomal nucleic acid of nematodes in the sample.

Also described herein is a method for determining, the biodiversity of a soil sample, comprising the steps of:
a) providing a ribosomal nucleic acid sample of the nematodes present in the sample;
b) detecting a plurality of ribosomal nucleic acids each comprising at least one single nucleotide polymorphism (SNP) according to Table 7 in the ribosomal nucleic acid sample; and
c) determining the presence of lower taxonomic groups in the sample on the basis of the detected plurality of ribosomal nucleic acids.

A method for determining the biodiversity of the nematode fauna in a soil, starting from the life strategies of a plurality of individual nematodes in a soil, can very suitably be carried out by means of a method in which a Maturity Index (MI) and a Plant Parasite Index (PPI) are determined.

By coupling the detected plurality of lower taxonomic groups of nematodes to their life strategies, a method of the present invention enables the determination of the soil health.

In one aspect, the present invention therefore provides a method for determining a life strategy of a nematode, comprising the steps of:
a) providing a database in which the single nucleotide polymorphisms (SNPs) according to Table 7 are correlated with life strategies of the respective nematodes.
b) detecting at least one of the single nucleotide polymorphisms (SNPs) in a nematode; and
c) determining, from the database, the life strategy of the nematode on the basis of the SNPs detected in step b.

In another aspect, the present invention provides a method for determining, the soil health of a soil, comprising the steps of:
a) providing a database in which the single nucleotide polymorphisms (SNPs) according to Table 7 are correlated with life strategies of the respective nematodes;
b) providing a ribosomal nucleic acid sample of the nematodes present in a sample of the soil;
c) detecting a plurality of ribosomal nucleic acids each comprising at least one single nucleotide polymorphism (SNP) according to Table 7 in the ribosomal nucleic acid sample for demonstrating the presence of a plurality of lower taxonomic groups of nematodes in the sample;
d) determining, from the database, the life strategies of a plurality of nematodes in the soil on the basis of the SNPs detected in step c; and
e) translating the plurality of life strategies thus obtained into an indication for the soil health.

In a preferred embodiment of such a method, the 'Maturity Index' is used as an indication for the soil health.

A method for determining the soil health according to the present invention has the advantage that, with molecular analysis techniques, a better differentiation can be provided in the determination of life strategies of nematodes than with morphological analysis techniques.

The invention further relates to a method for detecting a nematode with a specific life strategy, in which ribosomal nucleic aid of this nematode is contacted with one or more specific binding partners according to the invention and in which binding between the ribosomal nucleic acid and the binding partner is detected as an indication of the presence of this life strategy in this nematode.

Descibed herein is a polynucleotide isolated from nematode ribosomal nucleic acid, which polynucleotide has a length of between 6 and 50 nucleotides, preferably between 8 and 40 nucleotides, still more preferably between 10 and 35 nucleotides, and which polynucleotide contains at least one SNP according to Table 7. This polynucleotide may be RNA or DNA.

Also described herein are specific binding partners which are capable of specifically binding to the above-mentioned polynucleotide isolated from nematode ribosomal nucleic acid. In a preferred embodiment, the specific binding partners are oligonucleotide probes, such as nucleic acid probes or MLPA probes. The different nucleic acid probes which can be used as binding partners for ribosomal nucleic acid are known to a skilled person.

The present invention further provides a device for determining soil health, comprising a measuring device and a memory device, which memory device comprises a database in which the single nucleotide polymorphisms (SNPs) referred to in Table 7 are correlated with a life strategy of the respective nematode. The device further preferably comprises a contact location on which specific binding partners according to the invention can be provided.

Further described herein is a composition comprising one or more nucleic acid probes or MLPA probes according to the invention and to a kit for carrying out a method according to the invention, comprising a composition comprising one or more nucleic acid probes or MLPA probes according to the invention.

### DESCRIPTION OF THE FIGURES

Figure 1 shows the amplification diagram for the SSU rDNA of the nematodes as described in Example 1.
Figure 2 shows a device for determining soil health according to the invention.

### DETAILED DESCRIPTION OF THE INVENTION

Herein, the term "classification" or "classifying" is defined as ordering or grouping according to morphological; ecological or morphological features.

Herein, the term "taxonomic classification system" is defined as a morphological or a molecular classification system.

Herein, the term "morphological classification system" is defined as a hierarchical system in which nematodes are ordered according to phenotypic features such as form and structure and in which an ordering has been provided into different taxonomic levels such as orders, families, genera, species and subspecies.

Herein, the term "molecular classification system" is defined as a hierarchical system in which nematodes are ordered according to molecular features such as DNA and RNA nucleotide sequences and in which an ordering has been provided into different taxonomic levels such as orders, families, genera, species and subspecies.

Herein, the term "taxonomic identification" is defined as determining the corresponding position of an unidentified nematode in a classification system described in advance.

Herein, the term "morphological identification" is defined as identifying an unidentified nematode by determining morphological features and comparing these features to features in a morphological classification system described in advance.

Herein, the term "molecular identification" is defined as identifying an unidentified nematode by determining molecular features and comparing these features to features in a molecular classification system described in advance.

Herein, the term "taxon" is defined as a taxonomic group or unit, and may comprise any taxonomic level, such as order, family, genus, species and/or strain. -

Herein, the term "low taxonomic group" or "lower taxonomic group" is defined as a species, a genus or a family, preferably a species or genus.

"Phylogeny" relates to the study of the origin and ancestral succession which determines the evolutionary descent of a species or a class of species and in which the evolutionary origin and relationship are found by recognition of homology.

In the present application, "biodiversity" is understood to mean the variety, which variety with regard to life strategies may, for instance, be quantitatively expressed in the occurrence or the abundance of all taxa with different life strategies.

A "nucleic acid" or "polynucleotide" as used herein refers to a polymeric form of nucleotides of any length, both ribonucleotides and deoxyribonucleotides and comprises double and single-stranded DNA and RNA.

The term "life strategy" as used herein refers to a set of (population-)ecological properties of an organism, by means of which the rate at which the organism can regain its place in an ecosystem after a disturbance is shown. In general, the term is used to indicate whether the respective nematode is a "colonizer" or a "persister". The difference between "colonizers" and "persisters" in the sense of the life strategy is known to a skilled person. Nematodes whose numbers rapidly increase under favorable conditions are considered to belong to the colonizers. These nematodes have a short life cycle and reproduce fast; they also have a high nutrient demand, are often numerically dominant in samples, have high fluctuations in population density, have voluminous gonads, produce large amounts of small eggs and are often viviparous. Ecologically, colonizers may be considered *r*-strategists. Colonizers have low colonizer-persister (c-p) values. Persisters, on the other hand, are K-strategists (*sensu lato*), and have a low reproductive rate. They generally have a longer life cycle, a limited colonizing ability and they are sensitive to disturbance and need a longer time to recover after a disturbance; they never belong to the dominant species in a sample; they hardly fluctuate in number during the year; they have few descendants and small gonads but produce large eggs. In general, persisters live in environments which are stable during a longer period of time. Persisters have higher c-p values.

The 'Maturity Index' (MI) uses information regarding the fertility, nutrient demand and life strategy (*r* versus *K* strategist) of a nematode, and is an important indicator of the effects of disturbance on the soil ecosystem. For study of the soil health, and in the context of the method according to the present invention, the notion "life strategy" therefore particularly relates to the reproductive strategy, including the reproductive rate, the size of the reproductive system (the gonads), the size of the eggs, the number of descendants per individual and the length of the period in which the individual produces descendants; the life cycle strategy, including the duration of the life cycle, the fluctuation of the population size within a season; the ability to colonize; the sensitivity to disturbances; the usual population density or abundance; and the area of distribution.

Here, it should particularly be noted that the life strategy does not relate to the feeding type, that is, whether the nematode is a bacterivore, fungivore, carnivore, plant parasite or animal parasite. This information has no bearing on the determination of the soil health because this property does not relate to the life strategy as defined hereinabove or to whether soils have a certain degree of resilience, and whether the biological community in this soil has the ability to return to the original condition after disturbance or stress. For determining the soil health, the plant parasites and animal parasites may optionally be left out of account because they do not belong to the free-living nematodes.

The life strategy can quantitatively be expressed by means of a *c*-*p* value. Table 1 shows *c*-*p* values for different families of terrestrial and aquatic nematodes (from: Bongers (1990) Oecologia 83:14-19).

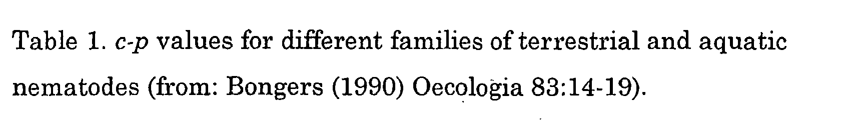

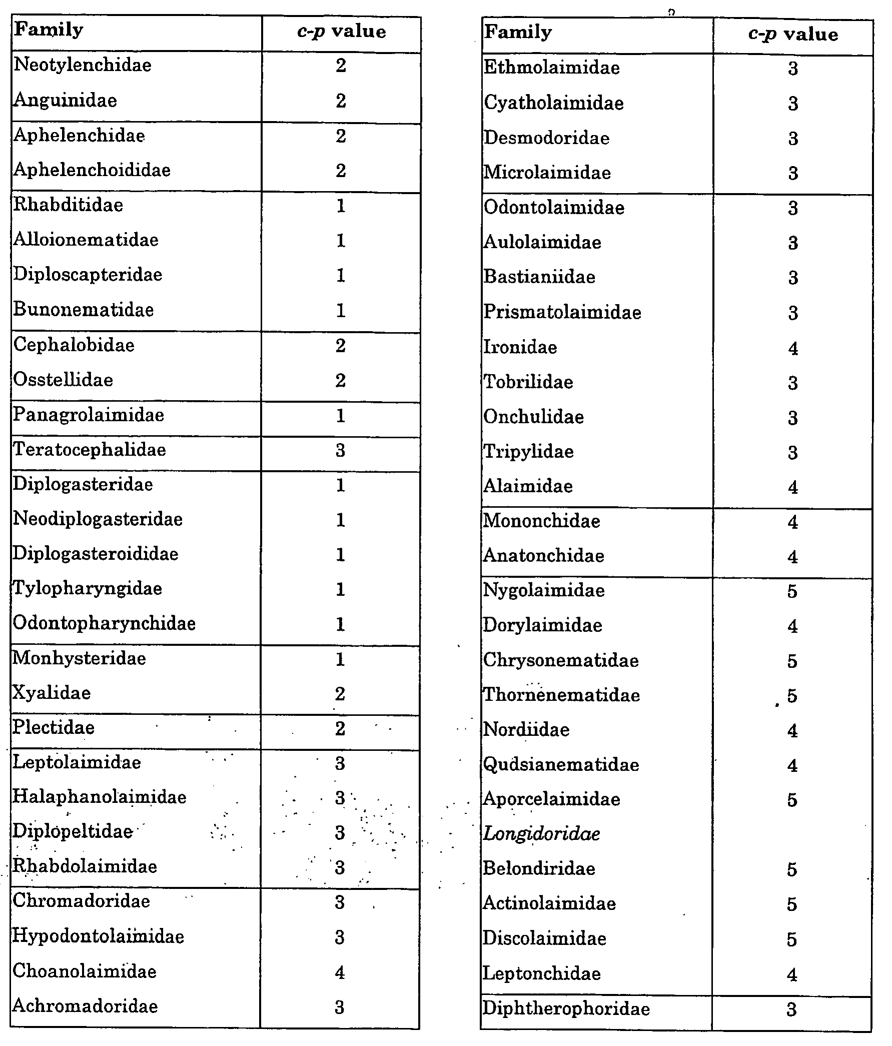

Table 2 shows *c*-*p* values for different families of plant-parasitic nematodes (from: Bongers (1990) Oecologia 83: 14-19).

**Table 2: c-p values for different families of plant-parasitic nematodes.**

| **Family** | ***c*-*p* value** |
|---|---|
| Tylenchidae | 2 |
| Psilenchidae | 2 |
| Tylodoridae | 2 |
| Ecphyadophoridae | 2 |
| Pratylenchidae | 2 |
| Anguinidae | 2 |
| Dolichodoridae | 3 |
| Hoplolaimidae | 3 |
| Pratylenchidae | 3 |
| Heteroderidae | 3 |
| Meloidogyne | 3 |
| Criconematidae | 3 |
| Hemicycliophoridae | 3 |
| Trichodoridae | 4 |
| Longidoridae | 5 |

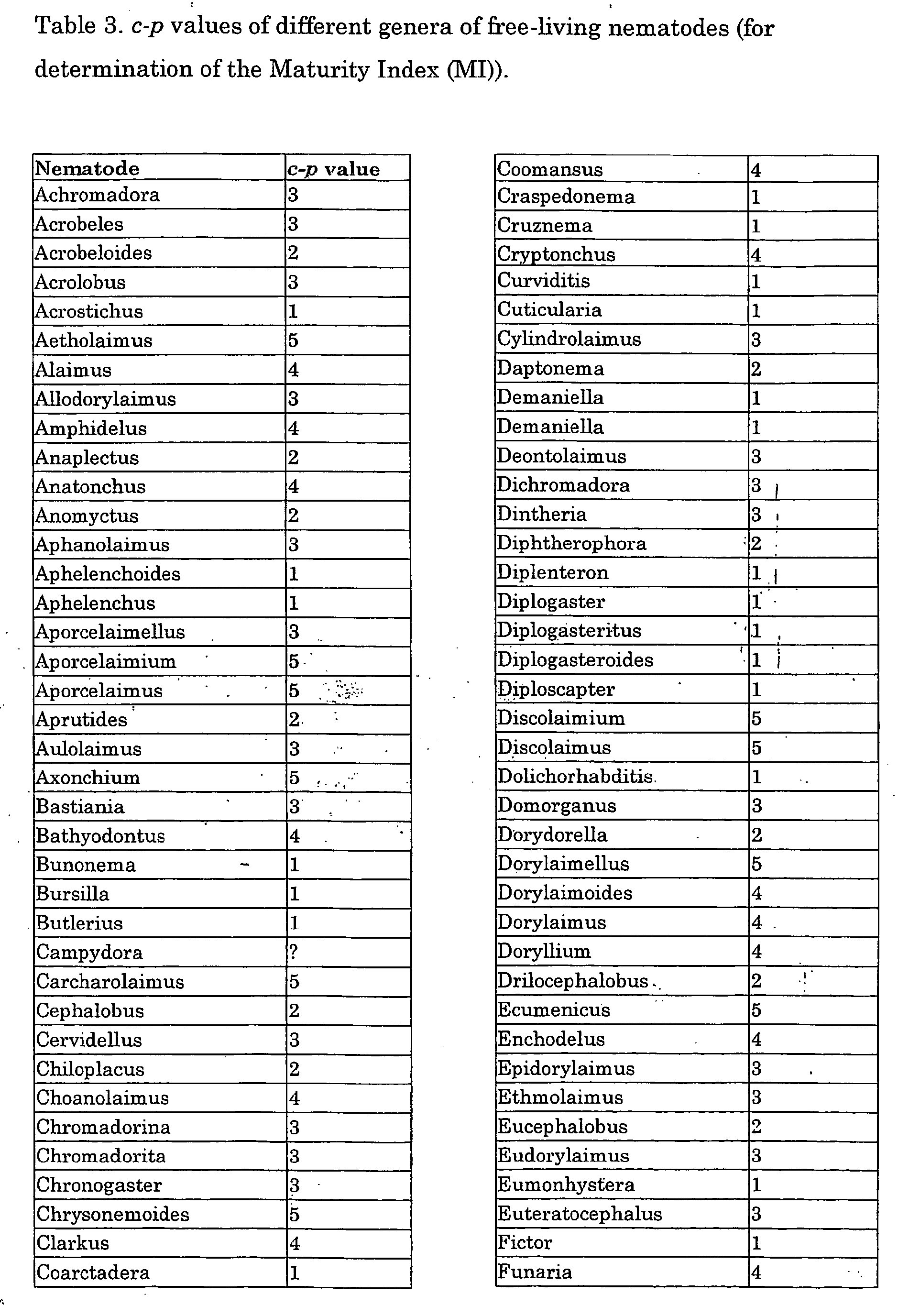

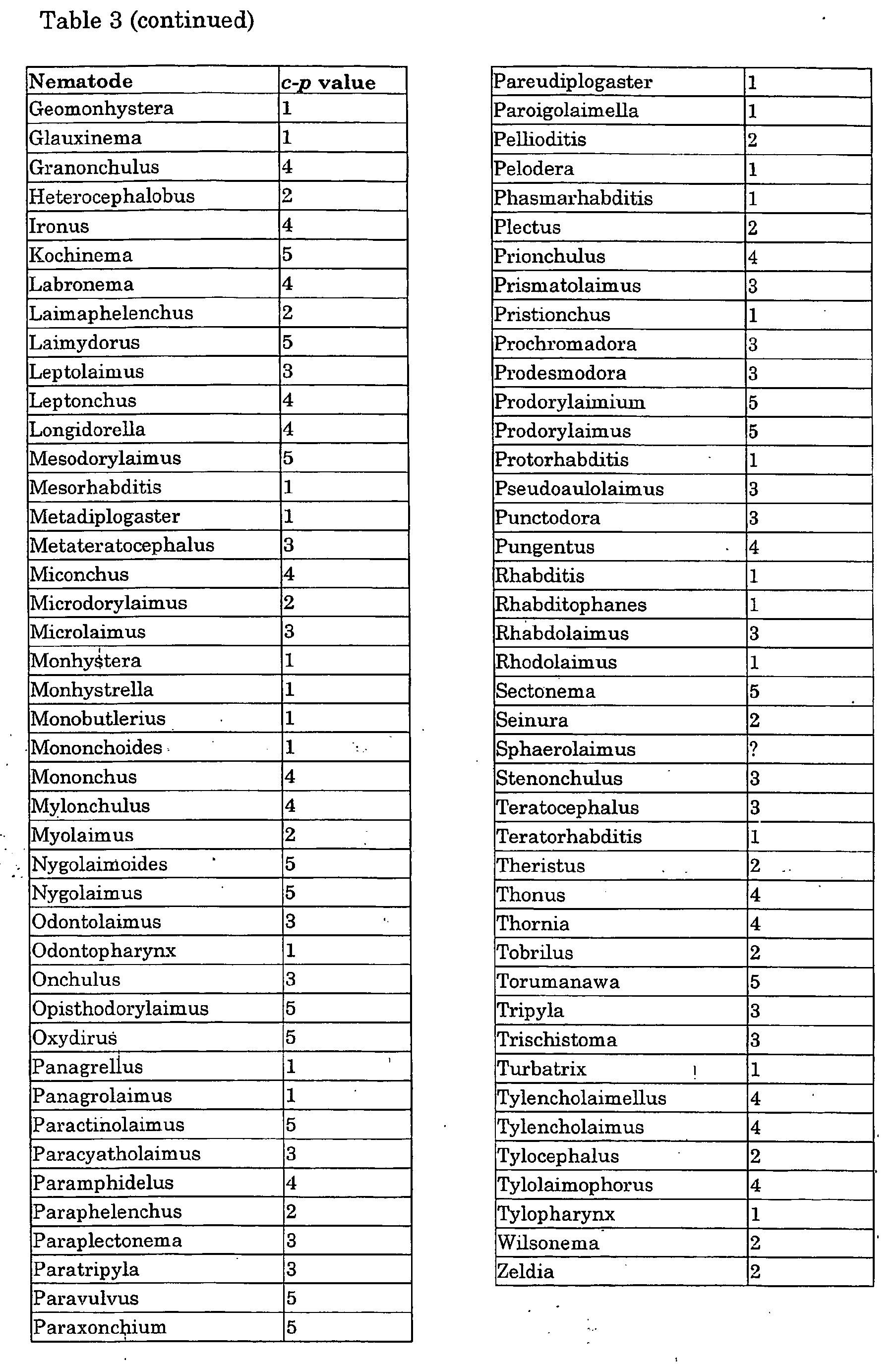

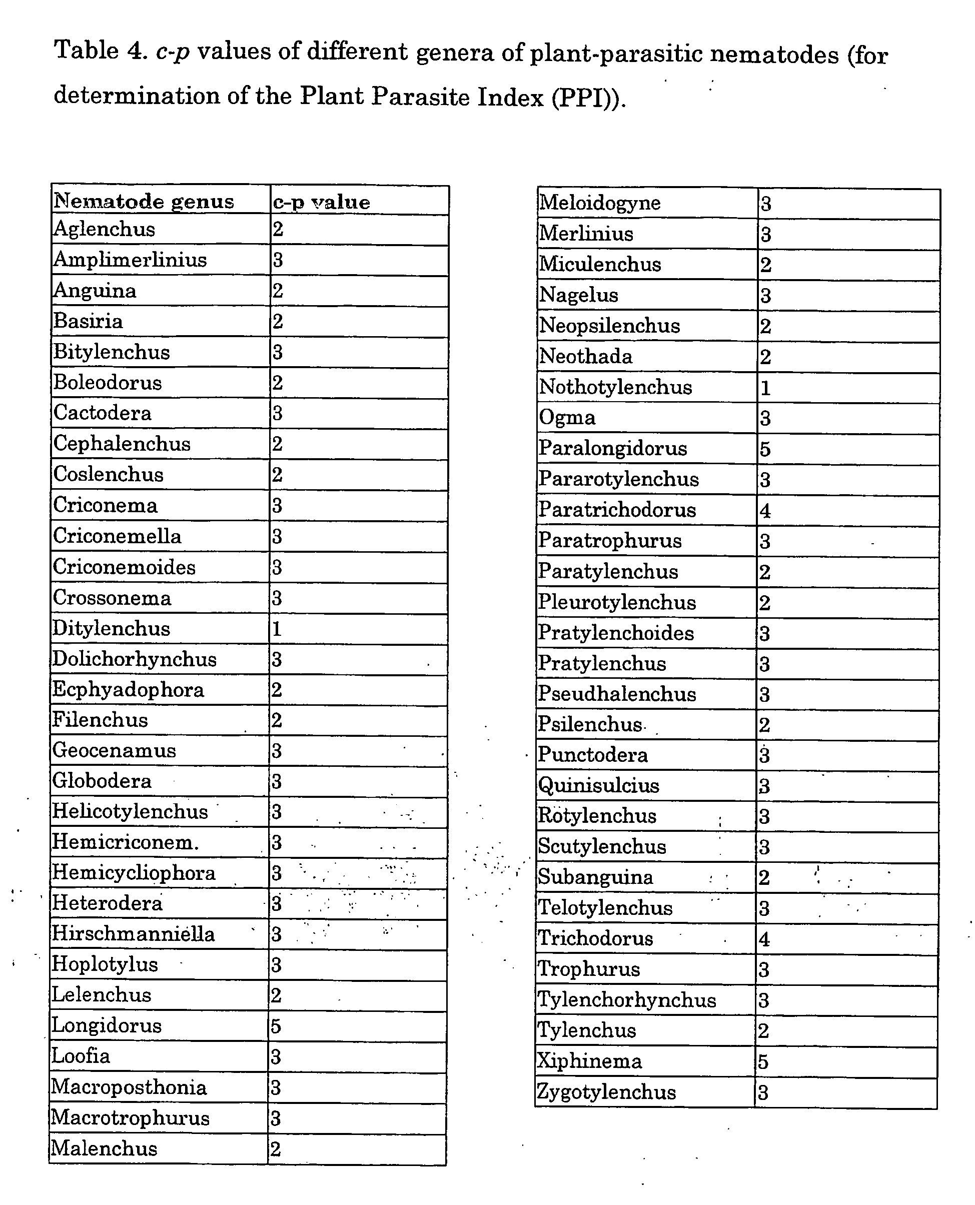

**Table 5. An example of a nematode family to which genera belong with a considerable diversity of ecological features (as appears from the c-p values). Family: Qudsianematidae (c-p value 4).**

| Family: Qudsianematidae | |
|---|---|
| **Genus** | ***c*-*p* value** |
| Thonus | 4 |
| Eudorylaimus | 3 |
| Allodorylaimus | 3 |
| Epidorylaimus | 3 |
| Dorydorella. | 2 |
| Microdorylaimus | 2 |
| Kochina | 5 |
| Labronema | 4 |

In the context of the present invention, the term "life strategy" may also be agriculturally relevant because the ability of either ob ligately or facultatively plant-parasitic nematodes to settle on cultivated crop is agriculturally important. Four life strategies in particular are of great agricultural importance: (1) the pathogenicity (where, when and at what density does a plant-parasitic nematode do demonstrable damage to a host plant, in this case a cultivate dcrop), (2) the host plant spectrum (on which (alternative) plant species can a nematode feed and propagate (monophagous versus polyphagous), (3) the vigor (how fast does the nematode density increase in the presence of a suitable host plant) and (4) the persistence (how long can a nematode population remain present in a soil in the absence of a host plant).

In the present invention, a "marker" is defined as a characteristic molecular feature of a nematode which can be measured or determined, preferably by use of molecularly biological methods, more preferably by use of specific binding partners. In aspects of the present invention, a molecular marker is a single nucleotide polymorphism referred to in Table 7. In case of use of specific binding partners, the pair, formed by the molecular feature to be measured and the binding partner, may, for instance, be chosen from the combination of polynucleotide (or nucleic acid) and complementary polynucleotide.

The terms "molecular feature", "molecular features" and "genetic marker" comprise, but are not limited to, the SNPs of Table 7.

The term "selectively hybridizing" comprises hybridizing, under stringent hybridization conditions, a nucleic acid sequence to a specified nucleic acid target sequence, to a detectably higher degree (for instance at least two times higher) than the hybridization of the same nucleic acid sequence to other, non-target sequences. Selectively hybridizing sequences typically have approximately at least 80% sequence identity, preferably 90% sequence identity, still more preferably 95% sequence identity, still more preferably 100% sequence identity (that is, complementarity) to each other.

Native populations of nematodes present in the soil offer a number of specific advantages for the determination of soil health. An important advantage is that nematodes as a group are very diverse. The phylum *Nematoda* comprises very many species, even a number of 5 million being estimated. The plant and animal parasites are generally known, but only form a minority within this phylum. The majority of nematodes belong to the free-living meiofauna of terrestrial soils and marine sediments. The number of species of nematodes which can be found in a soil sample may vary, but is characteristically between 30 and 60 species in a sample of approximately 100 ml. Another advantage is that nematodes are numerous. Nematodes are among the most numerous multicellular organisms. The nematode density in a soil may vary between 2 and 20 million individuals per square meter. They are found down to a depth of 10 m. Still another advantage of the use of nematodes is that the duration of the life cycle of nematodes is very diverse.

A plant-parasitic nematode is a nematode which obligately or facultatively uses living plants as a source of food. Facultative plant-parasitic nematodes may use living plants as a source of food (but they may also feed on, for instance, fungi). Obligate plant-parasitic nematodes can only feed on living plants.

Plant-parasitic nematodes are an integral part of the soil food web and can therefore also be included in a system for determining the soil health on the basis of the nematode population.

A number of obligate plant-parasitic nematodes cause considerable damage to agricultural crop, for instance the potato cyst nematode (*Globodera rostochiensis* and *Globodera pallida,* damage particularly in potato growing), the beet cyst nematode (*Heterodera schachtii,* damage particularly in sugar beet growing) and stem nematodes (*Ditylenchus* spp., damage *inter alia* in onion growing and a large number of bulbous plants). For this reason, a method according to the invention may also be used to determine the soil health more agriculturally, that is, by determining the presence of particular plant-parasitic nematodes in the soil.

The life cycle of nematodes varies from 3 days to several years. This implies that a nematode population can react to changes in their natural environment already within a short time.

Yet another advantage of the use of nematodes is that different species of nematodes have different sensitivities to soil pollutions. Nematodes have semi-permeable cuticles (permeable to water and a large number of ions and non-electrolytes). This means that nematodes are in direct contact with the capillary soil water and any pollution dissolved therein. In spite of this, their sensitivity to pollution varies from low (e.g. *Rhabditidae*) to high (e.g. *Thornenematidae*).

Yet another advantage of the use of nematodes is that nematodes form a trophically heterogeneous group. Nematodes may live on, for instance, fungi, bacteria, algae, and other nematodes, or are parasitic on plants.

Yet another advantage of the use of nematodes is that nematodes are relatively simple to purify from a soil sample. All soil-resident nematodes are small (0.05 to 2 mm), mobile, vermiform and positively geotropic. These features enable a simple purification or enrichment of the whole nematode population from a soil sample. After a first purification by means of a so-called Oostenbrink elutriator, the soil sample can be stored overnight on a double cotton/wool filter. All active nematodes will move downwards through the pores of this filter and can be collected in an extraction dish. The resulting relatively clean nematode suspension is directly ready for analysis. Other methods for the extraction of nematodes from the soil are described in the Examples hereinbelow.

A method for determining soil health according to the invention comprises the step of providing a database in which the single nucleotide polymorphisms (SNPs) referred to in Table 7 are correlated with life strategies of the respective nematodes. In the proposed method, animal-parasitic nematodes will be left out of account. This is because they are not informative in relation to the soil health condition.

In principle, such a database is not limited to the single nucleotide polymorphisms (SNPs) referred to in Table 7, but may comprise molecular features of individual nematodes which have been identified by means of morphological identification criteria or molecular identification criteria. The identification of an individual nematode according to the current, morphological criteria is known to a skilled person (see *inter alia* Maggenti, A.R. 1981. General Nematology. Springer Verlag, New York). The actual taxonomic identification (for instance species indication) of the nematodes in the database is not essential. However, it is important for the database that molecular features of nematodes therein are correlated with life strategies. This may optionally take place by taxonomically identifying the nematodes on the basis of molecular features, and, on the basis of the correlation with the morphological classification, annotating the known life strategies associated with this organism to the molecular feature.

In a preferred embodiment, single nucleotide polymorphisms (SNPs) are used to detect species of nematodes. In another preferred embodiment, single nucleotide polymorphisms (SNPs) are used to detect genera of nematodes.

Detecting a single nucleotide polymorphism (SNP) in the rRNA cistron of a nematode can be carried out in different manners. For instance, from individual, isolated nematodes, molecular material, such as DNA or RNA, can be isolated and a specific SNP can be determined in the ribosomal genes or in ribosomal RNA molecules. A very suitable method for detecting a single nucleotide polymorphism (SNP) in the rRNA cistron of a nematode, which can be correlated with a life strategy, comprises the use of a device according to the invention as described hereinbelow. Also, the presence of a molecular feature can be determined by use of the Multiplex Ligation-Dependent Probe Amplification (MLPA) methodology (Schouten et al. 2002. Nucleic Acids Responder. 30(12):e57) as *inter alia* described in EP 1130113, or by means of other suitable detection techniques known to a skilled person.

A life strategy which can be used in a database for use in a method of the present invention is preferably quantitatively indicated by means of a *c*-*p* value, that is, a number which indicates whether the respective nematode is a "colonizer" (*c*-*p* value = 1) or a "persister" (*c*-*p* value = 5).

A database which is used in a method according to the invention preferably comprises specific molecular features for a large number of different taxa of nematodes. Preferably, in a database, the molecular features of taxa at the genus level are correlated with life strategies. Preferably, genera of the families indicated in Table 1 and/or Table 2 are comprised in a database according to the invention. Such families are well characterized with regard to their life strategies. More preferably, the genera indicated in Table 3 and/or Table 4 are comprised in a database according to the invention. Such genera are well characterized with regard to their life strategies. A skilled person will be able to collect molecular features of suitable genera of which the life strategies are known and to enter them into a database. Also, a skilled person will be able to determine the relevant life strategies of nematodes not yet entered into the database. In most cases, a skilled person will grow the respective nematode in a Petri dish on a suitable medium. A skilled person will then determine "life -history traits" such as the growth rate, the body length in different stages of development, the optimum growth temperature and the sensitivity to suboptimal temperatures, the duration of the juvenile period (the period until the first egg laying) the duration of the reproductive period, the number of eggs laid during the reproductive period, and the duration of the senile (that is, post-reproductive) period. For the plant-parasitic nematodes, the pathogenicity, the host plant series (a list of plants on which the nematode can parasitize), the "vigor" and the persistence need to be added to these.

So, a database as described herein can very suitably be composed by determining molecular features and annotating life strategies of nematodes thereto. A database may also be provided with data such that the database applies to a limited or, conversely, a large number of different habitats or a corresponding low of high diversity of soil types. For composing a database which can be applied to a large variety of soil types, a skilled person may, for instance, include nematodes therein which have a general area of distribution. For this purpose, also, a large number of species can be classified into so-called rarity classes, varying from species which can be found generally to species which are only found very rarely in any habitat (see Table 6). By entering the life strategies and features of precisely those organisms with a low Z-value (generally distributed), the advantage is achieved that a database is provided having a range of application for a large number of different soil types.

Table 6: Occurrence and habitat of free-living and plant-parasitic nematodes found in the Netherlands, with the occurrence being expressed in different rarity classes (Z classes). In addition, it is indicated to which nematode family the different nematode species belong. The numeric values of the Z classes have the following denotations: Z class 5: 1- 5 sites known; Z class 4: 5 - 20 sites known; Z class 3: 20 -100 sites known; Z class 2: to be found in half of the indicated habitats; Z class 1: virtually constantly present in indicated habitat. In the column of soil type(s), the habitat is indicated in which the respective nematode species is found, for which the following abbreviations are used: AS: aquatic, silt / clay; AZ: aquatic, sand; AO: aquatic, organic (nutritious) material; AE: aquatic, epizoic (on water plants); A: aquatic (general); TK: terrestrial, clay; TL: terrestrial, loam, loess, sandy clay; TZ: terrestrial, sand; TV: terrestrial, peat; TO: terrestrial, organic (nutritious) material; TE: terrestrial, epizoic (on/in vegetation); T: terrestrial (general). The table has been compiled on the basis of the Dutch nematode fauna, but is representative of Northwest Europe.

| **Species** | **Family** | **Z class** | **Soil type(s)** |
|---|---|---|---|
| Achromadora pseudomicoletzkyi | Achromadoridae | 3 | - |
| Achromadora ruricola | Achromadoridae | 3 | T / A |
| Achromadora semiarmata | Achromadoridae | 3 | - |
| Achromadora tenax | Achromadoridae | 3 | T |
| Achromadora terricola | Achromadoridae | 3 | T / A |
| Acrobeles ciliatus | Cephalobidae | 2 | TZ |
| Acrobeles complexus | Cephalobidae | 2 | TZ |
| Acrobeles mariannae | Cephalobidae | 3 | TZ |
| Acrobeloides apiculatis | Cephalobidae | 3 | TZ / TV |
| Acrobeloides buetschlii | Cephalobidae | 1 | TZ / TK / TL |
| Acrobeloides nanus | Cephalobidae | 1 | TZ / TK / TL |
| Acrobeloides tricornis | Cephalobidae | 3 | TZ |
| Acrolobus emarginatus | Cephalobidae | 4 | TZ |
| Acrostichus mikuschi | Diplogasteridae | 4 | TE / TO |
| Aetholaimus rotundicauda | Nygolaimidae | 4 | TZ |
| Aglenchus agricola | Tylenchidae | 2 | TZ |
| Alaimus elongatus . | Alaimidae | 4 | TZ |
| Alaimus meyli | Alaimidae | 3 | T / A |
| Alaimus mucronatus | Alaimidae | 5 | T |
| Alaimus parvus | Alaimidae | 4 | T / A |
| Alaimus primitivus | Alaimidae | 2 | T / A |
| Alaimus proximus | Alaimidae | 3 | T |
| Alaimus simplex | Alaimidae | 4 | TZ |
| Allodorylaimus andrassyi | Qudsianematidae | 4 | TZ |
| Amphidelus elegans | Alaimidae | 3 | T / A |
| Amplimerlinius caroli | Dolichodoridae | 3 | - |
| Amplimerlinius icarus | Dolichodoridae | 3 | - |
| Anaplectus grandepapillatus | Plectidae | 3 | TZ / A |
| Anaplectus granulosus | Plectidae | 2 | TZ / A |
| Anaplectus porosus | Plectidae | 4 | T |
| Anatonchus tridentatus | Anatonchidae | 2 | T |
| Anguina agrostis | Anguinidae | 4 | TE |
| Anguina graminis | Anguinidae | 4 | TE |
| Anguina tritici | Anguinidae | 5 | TE |
| Anomyctus xenurus | Aphelenchoididae | 4 | TZ |
| Aphanolaimus aquaticus | Halaphanoloaimidae | 3 | T / A |
| Aphanolaimus attentus | Halaphanoloaimidae | 3 | A |
| Aphanolaimus deconincki | Halaphanoloaimidae | 4 | - |
| Aphanolaimus pseudoattentus | Halaphanoloaimidae | 4 | - |
| Aphelenchoides asterocaudatus | Aphelenchoididae | 5 | - |
| Aphelenchoides bicaudatus | Aphelenchoididae | 4 | TZ / AZ |
| Aphelenchoides blastophthorus | Aphelenchoididae | 3 | T |
| Aphelenchoides composticola | Aphelenchoididae | 1 | T |
| Aphelenchoides fragariae | Aphelenchoididae | 2 | TE |
| Aphelenchoides helophilus | Aphelenchoididae | 4 | AE |
| Aphelenchoides kuehni | Aphelenchoididae | 4 | T |
| Aphelenchoides limberi | Aphelenchoididae | 4 | TO? |
| Aphelenchoides parietinus | Aphelenchoididae | 2 | TE |
| Aphelenchoides ritzemabosi | Aphelenchoididae | 2 | TE |
| Aphelenchoides saprophilus | Aphelenchoididae | 1 | TO |
| Aphelenchoides sinodendroni | Aphelenchoididae | 5 | TE |
| Aphelenchoides subtenuis | Aphelenchoididae | 3 | TZ / TE |
| Aphelenchus avenae | Aphelenchidae | 2 | TZ / TO / TE |
| Aporcelaimellus paraobtusicaudatus | Aporcelaimidae | 5 | TZ |
| Aporcelaimellus obtusicaudatus | Aporcelaimidae | 2 | TZ / TL / TK |
| Aporcelaimellus simplex | Aporcelaimidae | 4 | T |
| Aporcelaimellus tritici | Aporcelaimidae | 5 | T |
| Aporcelaimium labiatum | Aporcelaimidae | 4 | TZ / A |
| Aporcelaimus eurydoris | Aporcelaimidae | 5 | T |
| Aporcelaimus superbus | Aporcelaimidae | 4 | TZ / TK / A |
| Aporcelaimus vorax | Aporcelaimidae | 4 | T / A |
| Aprutides guidetti | Aphelenchoididae | 5 | TL |
| Aulolaimus oxycephalus | Aulolaimidae | 4 | TZ |
| Axonchium coomansi | Belondiridae | 4 | TK |
| Axonchium coronatum | Belondiridae | 3 | TK |
| Axonchium nairi | Belondiridae | 5 | TK |
| Axonchium propinquum | Belondiridae | 3 | T |
| Basiria duplexa | Tylenchidae | 5 | TK |
| Basiria flandriensis | Tylenchidae | 5 | TK |
| Basiria gracilis | Tylenchidae | 5 | - |
| Basiria graminophila | Tylenchidae | 5 | TK |
| Bastiania gracilis | Bastianiidae | 3 | T |
| Bastiania longicaudata . | Bastianiidae | 5 | T |
| Bathyodontus mirus | Bathyodontidae | 4 | TZ |
| Bitylenchus bryobius | Dolichodoridae | 5 | TZ |
| Bitylenchus dubius | Dolichodoridae | 1 | TZ |
| Bitylenchus maximus | Dolichodoridae | 2 | TZ |
| Boleodorus clavicaudatus | Tylenchidae | 4 | TZ / TK |
| Boleodorus thylactus | Tylenchidae | 4 | - |
| Boleodorus volutus | Tylenchidae | 4 | TZ / TK |
| Bunonema ditlevseni | Bunonematidae | 4 | TE |
| Bunonema reticulatum | Bunonematidae | 2 | TO / TE |
| Bunonema richtersi | Bunonematidae | 2 | TO / TE |
| Bursilla monhystera | Rhabditidae | 2 | TO / AO |
| Butlerius filicaudatus | Diplogasteridae | 5 | TO |
| Butlerius micans | Diplogasteridae | 4 | AO / TO |
| Cactodera weissi | Heteroderidae | 5 | TZ |
| Campydora demonstrans | Campidoridae | 5 | T |
| Carcharolaimus banaticus | Discolaimidae | 4 | TZ |
| Cephalenchus hexalineatus | Tylodoridae | 2 | TZ |
| Cephalenchus illustris | Tylodoridae | 5 | TZ |
| Cephalenchus illustris | Tylodoridae | 5 | - |
| Cephalenchus leptus | Tylodoridae | 5 | TO |
| Cephalobus persegnis | Cephalobidae | 1 | TZ |
| Cervidellus serratus | Cephalobidae | 3 | TZ |
| Cervidellus vexilliger | Cephalobidae | 5 | - |
| Chiloplacus bisexualis | Cephalobidae | 3 | T |
| Chiloplacus demani | Cephalobidae | 3 | T |
| Chiloplacus propinquus | Cephalobidae | 3 | T |
| Chiloplacus symmetricus | Cephalobidae | 3 | T |
| Chiloplacus trifurcatus | Cephalobidae | 5 | T |
| Choanolaimus psammophilus | Choanolaimidae | 4 | TZ |
| Chromadorina bioculata | Chromadoridae | 3 | AE |
| Chromadorina germanica | Chromadoridae | 3 | AE |
| Chromadorita leuckarti | Hypodontolaimidae | 3 | AE |
| Chronogaster boettgeri | Leptolaimidae | 4 | TV |
| Chronogaster tenuis | Leptolaimidae | 4 | T |
| Chronogaster typica | Leptolaimidae | 4 | T / A |
| Chrysonemoides holsaticus | Chrysonematidae | 3 | A / T |
| Clarkus papillatus | Mononchidae | 2 | T / A |
| Coarctadera cystilarva | Rhabditidae | 4 | TZ / TO |
| Coarctadera icosiensis | Rhabditidae | 3 | TO |
| Coomansus parvus | Mononchidae | 3 | TZ / A |
| Coslenchus cancellatus | Tylenchidae | 5 | TK |
| Coslenchus costatus | Tylenchidae | 2 | TZ |
| Coslenchus franklinae | Tylenchidae | 5 | TK |
| Craspedonema styriacum | Bunonematidae | 5 | - |
| Craspedonema zeelandicum | Bunonematidae | 4 | TO |
| Criconema annuliferum | Criconematidae | 2 | TZ |
| Criconema demani | Criconematidae | 3 | TK |
| Criconema longulum | Criconematidae | 3 | TZ / TK |
| Criconema loofi | Criconematidae | 5 | TZ |
| Criconema mutabile | Criconematidae | 3 | - |
| Criconema princeps | Criconematidae | 3 | TZ |
| Criconema sphagni | Criconematidae | 3 | TV / TK |
| Criconemella macrodora | Criconematidae | 2 | TZ |
| Criconemella parva | Criconematidae | 3 | TL |
| Criconemoides amorphus | Criconematidae | 4 | TZ |
| Criconemoides informis | Criconematidae | 3 | TZ |
| Criconemoides morgensis | Criconematidae | 4 | TV |
| Crossonema menzeli | Criconematidae | 5 | TZ |
| Crossonema multisquamatum | Criconematidae | 5 | TZ |
| Cruznema tripartitum | Rhabditidae | 2 | TO |
| Cryptonchus tristis | Bathyodontidae | 3 | A |
| Curviditis curvicaudata | Rhabditidae | 3 | TO / AO |
| Cuticularia oxycerca | Rhabditidae | 2 | TO / AO |
| Cylindrolaimus communis | Diplopeltidae | 3 | TZ / A |
| Cylindrolaimus melancholicus | Diplopeltidae | 3 | TZ / A |
| Daptonema dubium | Xyalidae | 4 | A |
| Deladenus durus | Neotylenchidae | 3. | TE / TO |
| Demaniella cibourgensis | Diplogasteridae | 5 | TO |
| Demaniella microstoma | Diplogasteridae | 3 | TO |
| Deontolaimus papillatus | Leptolaimidae | 4 | T / A |
| Desmolaimus zeelandicus | Linhomoeidae | 4 | A |
| Dichromadora geophila | Hypodontolaimidae | 3 | AE |
| Dintheria tenuissima | Bastianiidae | 5 | TZ |
| Diphtherophora brevicollis | Diphtherophoridae | 5 | T |
| Diphtherophora communis | Diphtherophoridae | 3 | TK / TL |
| Diphtherophora obesa | Diphtherophoridae | 4 | T |
| Diplenteron colobocercus | Neodiplogasteridae | 3 | TZ |
| Diplogaster rivalis | Diplogasteridae | 2 | AO / AE |
| Diplogasteritus consobrinus | Diplogasteridae | 3 | TO |
| Diplogasteritus dendrophilus | Diplogasteridae | 4 | TE |
| Diplogasteritus nudicapitatus | Diplogasteridae | 3 | TO / AO |
| Diplogasteritus superbus | Diplogasteridae | 3 | TO / AO |
| Diplogasteroides spengelii | Diplogasteridae | 3 | TO |
| Diploscapter coronatus | Diploscapteridae | 2 | TO |
| Discolaimium dubium | Discolaimidae | 4 | T |
| Discolaimium filiforme | Discolaimidae | 5 | T |
| Discolaimium symmetricum | Discolaimidae | 5 | TZ |
| Discolaimus major | Discolaimidae | 3 | TZ |
| Discolaimus texanus | Discolaimidae | 3 | TZ |
| Ditylenchus brevicauda | Anguinidae | 4 | T? |
| Ditylenchus convallariae | Anguinidae | 5 | TE |
| Ditylenchus destructor | Anguinidae | 3 | T / TO |
| Ditylenchus dipsaci | Anguinidae | 3 | TZ / TK |
| Ditylenchus myceliophagus | Anguinidae | 2 | T / TO |
| Dolichorhabditis dolichura | Rhabditidae | 5 | TO / AO |
| Dolichorhynchus lamelliferus | Dolichodoridae | 4 | TK / TL |
| Dolichorhynchus microphasmis | Dolichodoridae | 3 | TZ |
| Domorganus macronephriticus | Diplopeltidae | 4 | TZ |
| Dorydorella bryophila | Qudsianematidae | 4 | TZ / A |
| Dorydorella pratensis | Qudsianematidae | 5 | T |
| Dorylaimellus globatus | Belondiridae | 3 | T |
| Dorylaimellus montenegricus | Belondiridae | 4 | T |
| Dorylaimellus parvulus | Belondiridae | 4 | T |
| Dorylaimoides bulbosus | Leptonchidae | 5 | T |
| Dorylaimoides elegans | Leptonchidae | 5 | TZ |
| Dorylaimoides limnophilus | Leptonchidae | 4 | TK |
| Dorylaimoides micoletzkyi | Leptonchidae | 4 | TK? |
| Dorylaimus stagnalis | Dorylaimidae | 2 | AS / T |
| Doryllium uniforme | Leptonchidae | 4 | TZ |
| Drilocephalobus moldavicus | Osstellidae | 4 | TZ |
| Ecphyadophora tenuissima | Ecphyadophoridae | 4 | - |
| Ecumenicus monohystera | Thornenematidae | 3 | TZ / A |
| Enchodelus macrodorus | Nordiidae | 4 | T / A |
| Epidorylaimus agilis | Qudsianematidae | 4 | T / A |
| Epidorylaimus consobrinus | Qudsianematidae | 4 | T / A |
| Epidorylaimus lugdunensis | Qudsianematidae | 5 | T / A |
| Ethmolaimus pratensis | Ethmolaimidae | 3 | A / TZ |
| Eucephalabus mucronatus | Cephalobidae | 2 | T |
| Eucephalobus oxyuroides | Cephalobidae | 1 | TZ |
| Eucephalobus paracornutus | Cephalobidae | 3 | T |
| Eucephalobus striatus | Cephalobidae | 2 | T |
| Eudorylaimus acuticauda | Qudsianematidae | 3 | TZ / A |
| Eudorylaimus carteri | Qudsianematidae | 3 | TZ / TE / A |
| Eudorylaimus centrocercus | Qudsianematidae | 3 | TZ / A |
| Eudorylaimus iners | Qudsianematidae | 5 | T / A |
| Eudorylaimus leuckarti | Qudsianematidae | 5 | T |
| Eudorylaimus similis | Qudsianematidae | 4 | T / TE / A |
| Eumonhystera barbata | Monhysteridae | 5 | AZ |
| Eumonhystera dispar | Monhysteridae | 3 | AE |
| Eumonhystera filiformis | Monhysteridae | 2 | TZ / AE |
| Eumonhystera similis | Monhysteridae | 3 | TZ / AZ / AE |
| Eumonhystera simplex | Monhysteridae | 3 | TZ / AZ |
| Eumonhystera vulgaris | Monhysteridae | 3 | TZ / AZ |
| Euteratocephalus hirschmannae | Teratocephalidae | 3 | AZ / TZ |
| Euteratocephalus palustris | Teratocephalidae | 3 | AZ |
| Fictor fictor | Neodiplogasteridae | 3 | AO |
| Filenchus baloghi | Tylenchidae | 5 | - |
| Filenchus discrepans | Tylenchidae | 2 | TK / TZ |
| Filenchus ditissimus | Tylenchidae | 1 | - |
| Filenchus helenae | Tylenchidae | 1 | - |
| Filenchus misellus | Tylenchidae | 3 | - |
| Filenchus orbus | Tylenchidae | 5 | - |
| Filenchus polyhyphnus | Tylenchidae | 5 | - |
| Filenchus quartus | Tylenchidae | 5 | - |
| Filenchus terrestris | Tylenchidae | 3 | - |
| Filenchus thornei | Tylenchidae | 3 | - |
| Filenchus vulgaris | Tylenchidae | 1 | - |
| Filenchus zaphari | Tylenchidae | 5 | - |
| Funania maryanneae | Leptonchidae | 5 | TL |
| Fungiotonchium fungorum | Iotonchiidae | 4 | TE / TO |
| Geocenamus arcticus | Dolichodoridae | 5 | TZ |
| Geomonhystera villosa | Monhysteridae | 2 | TZ / TE |
| Glauxinema armata | Neodiplogasteridae | 4 | AO / AE |
| Globodera millifolii | Heteroderidae | 5 | TZ |
| Globodera pallida | Heteroderidae | 1 | TZ / TK / TL |
| Globodera rostochiensis | Heteroderidae | 1 | TZ / TK / TL |
| Granonchulus schulzi | Mononchidae | 4 | A / TZ |
| Helicotylenchus canadensis | Hoplolaimidae | 4 | TK |
| Helicotylenchus coomansi | Hoplolaimidae | 5 | TK |
| Helicotylenchus digonicus | Hoplolaimidae | 4 | TK |
| Helicotylenchus paxilli | Hoplolaimidae | 5 | TZ |
| Helicotylenchus pseudorobustus | Hoplolaimidae | 1 | TZ |
| Helicotylenchus varicaudatus | Hoplolaimidae | 3 | TK |
| Helicotylenchus vulgaris | Hoplolaimidae | 3 | TK |
| Hemicriconem. pseudobrachyuris | Criconematidae | 3 | TZ |
| Hemicycliophora conida | Hemicycliophoridae | 2 | TZ |
| Hemicycliophora epicharoides | Hemicychophoridae | 4 | TZ |
| Hemicycliophora macristhmus | Hemicycliophoridae | 4 | TZ |
| Hemicycliophora nucleata | Hemicycliophoridae | 5 | AZ / AS |
| Hemicycliophora thornei | Hemicycliophoridae | 4 | TZ / TK |
| Hemicycliophora triangulum | Hemicycliophoridae | 3 | TZ |
| Hemicycliophora typica | Hemicycliophoridae | 2 | TZ |
| Heterocephalobus elongatus | Cephalobidae | 1 | T |
| Heterocephalobus longicaudatus | Cephalobidae | 2 | T |
| Heterocephalobus loofi | Cephalobidae | 5 | T |
| Heterodera avenae | Heteroderidae | 2 | TZ |
| Heterodera bifenestra | Heteroderidae | 3 | TZ |
| Heterodera carotae | Heteroderidae | 3 | TK |
| Heterodera cruciferae | Heteroderidae | 2 | TK |
| Heterodera daverti | Heteroderidae | 5 | TZ |
| Heterodera goettingiana | Heteroderidae | 2 | TZ |
| Heterodera hordecalis | Heteroderidae | 4 | TZ |
| Heterodera humuli | Heteroderidae | 3 | TZ |
| Heterodera iri | Heteroderidae | 4 | TZ |
| Heterodera mani | Heteroderidae | 2 | TZ |
| Heterodera schachtii | Heteroderidae | 2 | TK |
| Heterodera trifolii | Heteroderidae | 2. | TZ |
| Heterodera urticae | Heteroderidae | 4 | TZ |
| Hexatylus viviparus | Neotylenchidae | 3 | TO |
| Hirschmanniella gracilis | Pratylenchidae | 3 | TK |
| Hirschmanniella loofi | Pratylenchidae | 3 | TK |
| Hoplotylus femina | Pratylenchidae | 3 | TZ / TL |
| Ironus ignavus | Ironidae | 3 | A |
| Ironus longicaudatus | Ironidae | 3 | A / T |
| Ironus tenuicaudatus | Ironidae | 3 | T / A |
| Isolaimium multistriatum | Isolaimidae | 5 | TZ |
| Kochinema longum | Qudsianematidae | 5 | TZ |
| Labronema paesleri | Qudsianematidae | 4 | T |
| Laimaphelenchus pannocaudus | Aphelenchoididae | 4 | TE |
| Laimaphelenchus penardi | Aphelenchoididae | 3 | TE |
| Laimaphelenchus pini | Aphelenchoididae | 4 | TE |
| Laimydorus doryuris | Thornenematidae | 4 | AS |
| Laimydorus filiformis | Thornenematidae | 4 | A / T |
| Lelenchus leptosoma | Tylenchidae | 3 | TZ |
| Leptolaimus papilliger | Leptolaimidae | 3 | T / A |
| Leptolaimus setiger | Leptolaimidae | 4 | A |
| Leptonchus granulosus | Leptonchidae | 5 | TZ |
| Longidorella macramphis | Nordiidae | 4 | TZ |
| Longidorella microdorus | Nordiidae | 4 | TZ / A |
| Longidorus attenuatus | Longidoridae | 5 | TZ |
| Longidorus caespiticola | Longidoridae | 4 | TK |
| Longidorus cylindricaudatus | Longidoridae | 5 | TZ |
| Longidorus dunensis | Longidoridae | 5 | TZ |
| Longidorus elongatus | Longidoridae | 3 | TZ / TL / TK |
| Longidorus goodeyi | Longidoridae | 4 | TK |
| Longidorus intermedius | Longidoridae | 4 | TZ |
| Longidorus kuiperi | Longidoridae | 5 | TZ |
| Longidorus leptocephalus | Longidoridae | 3 | T |
| Longidorus macrosoma | Longidoridae | 4 | TK / TL |
| Longidorus profundorum | Longidoridae | 4 | TK |
| Longidorus vineacola | Longidoridae | 4 | TZ / TL |
| Loofia robusta | Hemicycliophoridae | 4 | TZ |
| Loofia thienemanni | Hemicycliophoridae | 2 | TZ / TL |
| Macroposthonia annulata | Criconematidae | 3 | TL / TK |
| Macroposthonia annulatiformis | Criconematidae | 4 | TK |
| Macroposthonia crenata | Criconematidae | 4 | TZ |
| Macroposthonia curvata | Criconematidae | 4 | TZ |
| Macroposthonia dherdei | Criconematidae | 4 | TK |
| Macroposthonia involuta | Criconematidae | 5 | TK |
| Macroposthonia irregularis | Criconematidae | 4 | TZ |
| Macroposthonia maritima | Criconematidae | 5 | TZ |
| Macroposthonia pseudosolivaga | Criconematidae | 3 | TK |
| Macroposthonia raskiensis | Criconematidae | 5 | TV |
| Macroposthonia rotundicauda | Criconematidae | 3 | TZ |
| Macroposthonia rustica | Criconematidae | 2 | TZ |
| Macroposthonia solivaga | Criconematidae | 4 | TZ |
| Macroposthonia sphaerocephala | Criconematidae | 3 | TZ |
| Macroposthonia vadensis | Criconematidae | 5 | TK |
| Macroposthonia xenoplax | Criconematidae | 2 | TZ |
| Macrotrophurus arbusticola | Dolichodoridae | 4 | TK |
| Malenchus acarayensis | Tylenchidae | 3 | - |
| Malenchus andrassyi | Tylenchidae | 4 | TZ |
| Malenchus bryophilus | Tylenchidae | 2 | - |
| Meloidogyne ardenensis | Meloidogynidae | 3 | TZ |
| Meloidogyne chitwoodi | Meloidogynidae | 3 | TZ |
| Meloidogyne graminis | Meloidogynidae | 3 | TZ |
| Meloidogyne hapla | Meloidogynidae | 2 | TZ / TK |
| Meloidogyne naasi | Meloidogynidae | 2 | TZ |
| Merlinius brevidens | Dolichodoridae | 3 | TK |
| Merlinius joctus | Dolichodoridae | 5 | - |
| Merlinius microdorus | Dolichodoridae | 3 | TZ / TL |
| Merlinius nanus | Dolichodoridae | 3 | TZ / TL |
| Merlinius nothus | Dolichodoridae | 3 | TZ |
| Merlinius processus | Dolichodoridae | 5 | TZ |
| Mesodorylaimus aberrans | Thornenematidae | 5 | TZ |
| Mesodorylaimus attenuatus | Thornenematidae | 4 | TZ / A |
| Mesodorylaimus bastiani | Thornenematidae | 2 | TZ / A |
| Mesodorylaimus litoralis | Thornenematidae | 4 | TK? |
| Mesodorylaimus mesonyctius | Thornenematidae | 4 | TL |
| Mesodorylaimus spengeli | Thornenematidae | 4 | TZ |
| Mesorhabditis belari | Rhabditidae | 5 | TO |
| Mesorliabditis juglandicola | Rhabditidae | 5 | TE /TO |
| Mesorhabditis spiculigera | Rhabditidae | 2 | TO |
| Mesorhabditis ultima | Rhabditidae | 4 | TE / TO |
| Metadiplogaster graciloides | Diplogasteridae | 4 | TO |
| Metateratocephalus crassidens | Teratocephalidae | 1 | TZ / TV / AE |
| Miconchus studeri | Anatonchidae | 4 | TZ |
| Microdorylaimus miser | Qudsianematidae | 5 | T |
| Microdorylaimus modestes | Qudsianematidae | 5 | TZ |
| Microdoiylaimus parvus | Qudsianematidae | 3 | TZ / A |
| Microlaimus globiceps | Microlaimidae | 3 | A |
| Miculenchus salvus | Tylenchidae | 5 | TK / TZ |
| Monhystera paludicola | Monhysteridae | 2 | AZ |
| Monhystera riemanni | Monhysteridae | 4 | AZ |
| Monhystera stagnalis | Monhysteridae | 2 | AZ |
| Monhystrella macrura | Monhysteridae | 3 | T / A |
| Monobutlerius degrissei | Diplogasteridae | 4 | TO |
| Monobutlerius monohystera | Diplogasteridae | 5 | TO |
| Mononchoides elegans | Neodiplogasteridae | 4 | TO |
| Mononchoides striatus | Neodiplogasteridae | 4 | TO / AO |
| Mononchus aquaticus | Mononchidae | 5 | A |
| Mononchus niddensis | Mononchidae | 5 | A / T |
| Mononchus truncatus | Mononchidae | 2 | A / T |
| Mononchus tunbridgensis | Mononchidae | 3 | A |
| Mylonchulus brachyuris | Mononchidae | 2 | T / A |
| Mylonchulus rotundicaudatus | Mononchidae | 4 | A |
| Mylonchulus sexcristatus | Mononchidae | 5 | T |
| Mylonchulus sigmaturus | Mononchidae | 2 | T / A |
| Mylonchulus subtenuis | Mononchidae | 4 | TZ |
| Myolaimus heterurus | Myolainiidae | 4 | TO / TE |
| Nagelus obscurus | Dolichodoridae | 4 | - |
| Neopsilenchus magnidens | Tylenchidae | 3 | - |
| Neopsilenchus minor | Tylenchidae | 3 | TK |
| Neothada cancellata | Tylenchidae | 5 | TZ |
| Nothotylenchus acris | Anguinidae | 5 | T? |
| Nygolaimoides borborophilus | Nygolaimidae | 4 | T |
| Nygolaimus altherri | Nygolaimidae | 5 | T |
| Nygolaimus aquaticus | Nygolaimidae | 3 | A |
| Nygolaimus brachyuris | Nygolaimidae | 4 | TZ |
| Nygolaimus clavicaudatus | Nygolaimidae | 4 | T |
| Nygolaimus intermedius | Nygolaimidae | 4 | T / A |
| Nygolaimus laevis | Nygolaimidae | 4 | T |
| Nygolaimus loofi | Nygolaimidae | 3 | T / A |
| Nygolaimus macrobrachyuris | Nygolaimidae | 4 | T |
| Nygolaimus trophurus | Nygolaimidae | 5 | TK |
| Odontolaimus chlorurus | Odontolaimidae | 4 | T |
| Odontopharynx longicaudata | Odontopharynchidae | 3 | TO / AO |
| Ogma cobbi | Criconematidae | 4 | TV |
| Onchulus nolli | Onchulidae | 2 | A |
| Opisthodorylaimus sylphoides | Thornenematidae | 5 | TK |
| Oxydirus amplicephalus | Belondiridae | 4 | TK |
| Oxydirus nethus | Belondiridae | 5 | TK |
| Oxydirus oxycephaloides | Belondiridae | 4 | TK |
| Oxydirus oxycephalus | Belondiridae | 4 | T |
| Panagrellus redivivus | Panagrolaimidae | 2 | TO / AO |
| Panagrolaimus paetzoldi | Panagrolaimidae | 2 | TO / AO |
| Panagrolaimus rigidus | Panagrolaimidae | 1 | TO / AO |
| Panagrolaimus thienemanni | Panagrolaimidae | 3 | AO |
| Paractinolaimus macrolaimus | Actinolaimidae | 4 | A / T |
| Paracyatholaimus intermedius | Cyatholaimidae | 4 | TK / A |
| Paralongidorus maximus | Longidoridae | 4 | TZ / TL |
| Paramphidelus dolichurus | Alaimidae | 2 | TL / TZ / TV |
| Paramphidelus exilis | Alaimidae | 3 | T |
| Paramphidelus hortensis | Alaimidae | 3 | T |
| Paramphidelus paramonovi | Alaimidae | 4 | A / T |
| Paramphidelus uniformis | Alaimidae | 3 | T / A |
| Paraphanolaimus behningi | Halaphanolaimidae | 4 | A |
| Paraphelenchus pseudoparietinus | Aphelenchidae | 3 | TZ |
| Paraphelenchus tritici | Aphelenchidae | 5 | TZ |
| Paraplectonema pedunculatum | Leptolaimidae | 3 | A |
| Pararotylenchus blothrotylus | Hoplolaimidae | 5 | TZ |
| Paratrichodorus anemones | Trichodoridae | 4 | T |
| Paratrichodorus nanus | Trichodoridae | 4 | TZ |
| Paratrichodorus pachydermis | Trichodoridae | 3 | TZ / TL |
| Paratrichodorus renifer | Trichodoridae | 4 | TZ |
| Paratrichodorus teres | Trichodoridae | 3 | TZ |
| Paratripyla intermedia | Tripylidae | 3 | A / T |
| aratrophurus bursifer . | Dolichodoridae | 4 | TK / TL |
| Paratylenchus bukowinensis | Paratylenchidae | 5 | - |
| Paratylenchus dianthus | Paratylenchidae | 5 | - |
| Paratylenchus goodeyi | Paratylenchidae | 3 | TK / TL |
| Paratylenchus hamatus | Paratylenchidae | 5 | - |
| Paratylenchus macrodorus | Paratylenchidae | 3 | TL |
| Paratylenchus microdorus | Paratylenchidae | 2 | - |
| Paratylenchus nanus | Paratylenchidae | 2 | TZ |
| Paratylenchus neoamblycephalus | Paratylenchidae | 4 | TK |
| Paratylenchus projectus | Paratylenchidae | 4 | TZ |
| Paratylenchus steineri | Paratylenchidae | 3 | TZ |
| Paratylenchus straeleni . | Pratylenchidae | 3 | - |
| Paratylenchus tateae | Paratylenchidae | 5 | - |
| Paravulvus hartingi | Nygolaimidae | 4 | T / A |
| Paraxonchium laetificans | Aporcelaimidae | 4 | T |
| Pareudiplogaster pararmatus | Neodiplogasteridae | 3 | TO / AO / AE |
| Paroigolaimella bernensis | Diplogasteridae | 4 | AO |
| Paroigolaimella coprophaga | Diplogasteridae | 3 | TO |
| Paroigolaimella paraspirifera | Diplogasteridae | 5 | TO |
| Pellioditis buetschlii | Rhabditidae | 2 | TO |
| Pellioditis marina | Rhabditidae | 3 | TO / AO |
| Pellioditis pellio | Rhabditidae | 3 | TO |
| Pellioditis pellioides | Rhabditidae | 2 | TO |
| Pelodera punctata | Rhabditidae | 3 | TZ |
| Pelodera strongyloides | Rhabditidae | 4 | TO |
| Pelodera teres | Rhabditidae | 3 | TO |
| Phasmarhabditis papillosa | Rhabditidae | 3 | TO |
| Plectus acuminatus | Plectidae | 3 | T / TE |
| Plectus armatus | Plectidae | 3 | T / A |
| Plectus assimilis | Plectidae | 2 | T |
| Plectus cirratus | Plectidae | 3 | T / A |
| Plectus elongatus | Plectidae | 3 | TE / T |
| Plectus exinocaudatus | Plectidae | 5 | T |
| Plectus geophilus | Plectidae | 3 | T / A |
| Plectus longicaudatus | Plectidae | 1 | T / A |
| Plectus palustris | Plectidae | 3 | A |
| Plectus parietinus | Plectidae | 2 | T / A |
| Plectus parvus | Plectidae | 2 | T / A |
| Plectus pusillus | Plectidae | 3 | TE / T |
| Plectus rhizophilus | Plectidae | 3 | T / A |
| Plectus tenuis | Plectidae | 3 | A |
| Pleurotylenchus sachsi | Tylodoridae | 5 | AS |
| Pratylenchoides crenicauda | Pratylenchidae | 3 | TZ |
| Pratylenchoides laticauda | Pratylenchidae | 4 | TZ |
| Pratylenchoides maritimus | Pratylenchidae | 5 | TZ |
| Pratylenchus convallariae | Pratylenchidae | 3 | TZ |
| Pratylenchus crenatus | Pratylenchidae | 1 | TZ |
| Pratylenchus fallax | Pratylenchidae | 1 | TL |
| Pratylenchus flakkensis | Pratylenchidae | 4 | TL |
| Pratylenchus neglectus | Pratylenchidae | 1 | TK / TL |
| Pratylenchus penetrans | Pratylenchidae | 1 | TZ |
| Pratylenchus pinguicaudatus | Pratylenchidae | 4 | TZ |
| Pratylenchus pratensis | Pratylenchidae | 1 | TL |
| Pratylenchus pseudopratensis | Pratylenchidae | 4 | TZ |
| Pratylenchus thornei | Pratylenchidae | 1 | TK |
| Pratylenchus vulnus | Pratylenchidae | 3 | TZ |
| Prionchulus muscorum | Mononchidae | 2 | TE / T |
| Prionchulus punctatus | Mononchidae | 2 | TE / T |
| Prismatolaimus dolichurus | Prismatolaimidae | 1 | TZ / TK / A |
| Prismatolaimus intermedius | Prismatolaimidae | 3 | TV / T / A |
| Pristionchus lheritieri | Neodiplogasteridae | 2 | TO |
| Prochromadora orleji | Chromadoridae | 3 | T / AE |
| Prodesmodora arctica | Desmodoridae | 5 | TZ |
| Prodesmodora circulata | Desmodoridae | 4 | A |
| Prodorylaimium brigdammense | Thornenematidae | 4 | T / A |
| Prodorylaimus acris | Thornenematidae | 3 | T |
| Prodorylaimus filiarum | Thornenematidae | 3 | T |
| Prodorylaimus mas | Thornenematidae | 4 | T |
| Prodorylaimus rotundiceps | Thornenematidae | 3 | TK |
| Prodorylaimus uliginosus | Thornenematidae | 3 | TK |
| Protorhabditis filiformis | Rhabditoidae | 3 | TO |
| Protorhabditis oxyuroides | Rhabditoidae | 3 | TO |
| Pseudhalenchusminutus | Anguinidae | 3 | TZ |
| Pseudoaulolaimus anchilocaudatus | Aulolaimidae | 5 | TK / TL |
| Psilenchus aestuarius | Psilenchidae | 4 | - |
| Psilenchus hilarulus | Psilenchidae | 3 | TK |
| Psilenchus terextremus | Psilenchidae | 5 | - |
| Punctodera punctata | Heteroderidae | 1 | TZ |
| Punctodora ratzeburgensis | Chromadoridae | 3 | AE |
| Pungentus alpinus | Nordiidae | 4 | TZ |
| Pungentus engadinensis | Nordiidae | 4 | T |
| Pungentus silvestris | Nordiidae | 3 | TZ / TK / A |
| Quinisulcius capitatus | Dolichodoridae | 4 | TZ |
| Rhabditis gracilicauda | Rhabditidae | 2 | TO |
| Rhabditis intermedia | Rhabditidae | 3 | TO |
| Rhabditis longicaudata | Rhabditidae | 3 | TO |
| Rhabditis maupasi | Rhabditidae | 3 | TO |
| Rhabditis producta | Rhabditidae | 3 | TO |
| Rhabditis terricola | Rhabditidae | 1 | TO |
| Rhabditophanes schneideri | Alloionematidae | 2' | TO |
| Rhabdolaimus aquaticus | Rhabdolaimidae | 3 | A |
| Rhabdolaimus terrestris | Rhabdolaimidae | 3 | TK / TZ / A |
| Rhodolaimus goffarti | Bunonematidae | 3 | TO |
| Rhodolaimus poligraphi | Bunonematidae | 3 | TO |
| Rotylenchulus borealis | Rotylenchulidae | 4 | TK |
| Rotylenchus buxophilus | Hoplolaimidae | 3 | - |
| Rotylenchus fallorobustus | Hoplolaimidae | 2 | TK |
| Rotylenchus goodeyi | Hoplolaimidae | 3 | TK |
| Rotylenchus robustus | Hoplolaimidae | 1 | TZ |
| Scutylenchus quadrifer | Dolichodoridae | 3 | TK |
| Scutylenchus tartuensis | Dolichodoridae | 4 | TK / TL |
| Scutylenchus tessellatus | Dolichodoridae | 4 | TZ |
| Sectonema barbatoides | Aporcelaimidae | 5 | TZ |
| Sectonema pseudoventrale | Aporcelaimidae | 4 | T |
| Seinura demani | Aphelenchoididae | 3 | TZ / TK |
| Seinura tenuicaudata | Aphelenchoididae | 4 | TZ |
| Seinura winchesi | Aphelenchoididae | 3 | TK |
| Sphaerolaimus gracilis | Sphaerolaimidae | 3 | T / A |
| Sphaeronema rumicis | Sphaeronematidae | 4 | TZ |
| Stenonchulus troglodytes | Onchulidae | 5 | - |
| Subanguina askenasyi | Anguinidae | 4 | - |
| Subanguina radicicola | Anguinidae | 3 | TZ |
| Telotylenchus ventralis | Dolichodoridae | 4 | TZ |
| Teratocephalus costatus | Teratocephalidae | 3 | TZ |
| Teratocephalus tenuis | Teratocephalidae | 4 | TZ |
| Teratocephalus terrestris | Teratocephalidae | 2 | TZ / AZ |
| Teratorhabditis dentifera | Teratocephalidae | 4 | TO |
| Theristus agilis | Xyalidae | 4 | T / A |
| Thonus circulifer | Qudsianematidae | 3 | TZ |
| Thonus ettersbergensis | Qudsianematidae | 5 | TK |
| Thonus laticollis | Qudsianematidae | 5 | TZ |
| Thonus minutus | Qudsianematidae | 5 | T |
| Thonus productus | Qudsianematidae | 4 | TZ / A |
| Thonus rhopalocercus | Qudsianematidae | 4 | T |
| Thornia propinqua | Nordiidae | 4 | T / A |
| Thornia steatopyga | Nordiidae | 4 | T / A |
| Tobrilus allophysis | Tobrilidae | 3 | AS |
| Tobrilus diversipapillatus | Tobrilidae | 4 | AS |
| Tobrilus gracilis | Tobrilidae | 2 | AZ |
| Tobrilus grandipapillatus | Tobrilidae | 3 | AS |
| Tobrilus medius | Tobrilidae | 5 | AZ |
| Tobrilus pellucidus | Tobrilidae | 4 | T |
| Tobrilus stefanski | Tobrilidae | 3 | A |
| Tobrilus steineri | Tobrilidae | 3 | A |
| Torumanawa litoralis - | Aporcelaimidae | 4 | TZ |
| Trichodorus cylindricus | Trichodoridae | 3 | TK / TL |
| Trichodorus primitivus | Trichodoridae | 3 | TZ / TL |
| Trichodorus similis | Trichodoridae | 3 | TZ / TL / TV |
| Trichodorus sparsus | Trichodoridae | 3 | TZ |
| Trichodorus variopapillatus | Trichodoridae | 4 | T |
| Trichodorus viruliferus | Trichodoridae | 3 | TZ / TL |
| Tripyla affinis | Tripylidae | 3 | A / T / TE |
| Tripyla cornuta | Tripylidae | 3 | T / A |
| Tripyla filicaudata | Tripylidae | 3 | A / T |
| Tripyla glomerans | Tripylidae | 4 | A |
| Tripyla setifera | Tripylidae | 3 | T / A |
| Trischistoma arenicola | Tripylidae | 4 | T |
| Trischistoma monohystera | Tripylidae | 4 | A / T |
| Trophurus imperialis | Dolichodoridae | 4 | TK |
| Trophurus sculptus | Dolichodoridae | 5 | TK |
| Turbatrix aceti | Panagrolaimidae | 3 | TO / AO |
| Tylencholaimellus affinis | Leptonchidae | 3 | T / A |
| Tylencholaimellus striatus | Leptonchidae | 3 | T / A |
| Tylencholaimus crassus | Leptonchidae | 3 | T / A |
| Tylencholaimus formosus | Leptonchidae | 4 | TZ |
| Tylencholaimus minimus | Leptonchidae | 3 | TK |
| Tylencholaimus mirabilis | Leptonchidae | 2 | TZ / A |
| Tylencholaimus paradoxus | Leptonchidae | 5 | TZ |
| Tylencholaimus proximus | Leptonchidae | 4 | T |
| Tylencholaimus teres | Leptonchidae | 4 | T |
| Tylenchorhynchus clarus | Dolichodoridae | 5 | TK |
| Tylenchorhynchus claytoni | Dolichodoridae | 3 | TZ |
| Tylenchorhynchus striatus | Dolichodoridae | 4 | TK |
| Tylenchus arcuatus | Tylenchidae | 5 | TL |
| Tylenchus davainei | Tylenchidae | 3 | - |
| Tylenchus elegans | Tylenchidae | 3 | - |
| Tylocephalus auriculatus | Plectidae | 3 | TZ |
| Tylolaimophorus typicus | Diphtherophoridae | 2 | TZ |
| Tylopharynx foetida | Tylopharynchidae | 3 | TO |
| Wilsonema otophorum | Plectidae | 2 | TZ |
| Xiphinema coxi | Longidoridae | 4 | TZ |
| Xiphinema diversicaudatum | Longidoridae | 3 | TZ / TL / TK |
| Xiphinema pseudocoxi | Longidoridae | 5 | - |
| Xiphinema vuittenezi | Longidoridae | 5 | T |
| Zeldia feria | Cephalobidae | 4 | TZ |
| Zeldia spinula | Cephalobidae | 5 | TZ |
| Zygotylenchus guevarai | Pratylenchidae | 4 | TK |

In addition, a database which is used in a method according to the invention preferably comprises specific molecular features for nematodes with different life strategies.

A database in which molecular features of nematodes are correlated with life strategies of the nematodes may, for instance, be compiled by identifying an individual nematode on the basis of current, morphological criteria, and determining, on the basis of the result of this identification procedure, a life strategy of the respective nematode and, in addition, determining a molecular feature of the same nematode. So, it is possible to identify a nematode on the basis of molecular features, such as SSU rDNA sequence data, and to determine, on the basis of the result of this identification procedure, a life strategy of the respective nematode and to annotatedly enter both data into a database.

Table 7 shows the SNPs which can be used for identification of low taxonomic groups of nematodes. For making this table, SSU rDNA sequences of a large number of free-living and plant-parasitic nematodes were aligned. For this alignment, use was made of a sequence alignment editor "BioEdit" (version 5.0.9., 2001) (Hall, T.A. 1999. Nucl. Acids. Symp. Ser. 41:95-98). Where needed, the alignment has been manually corrected. Then, with the eye, those SNPs in the alignment were identified which can be used to be able to distinguish a particular taxonomic level (a species) from the closest taxonomic level (e.g. the most related species).

Table 7: SNPs usable for identification and detection of low taxonomic groups of nematodes. Referred to in the form of a list: *Genus*/*Species* (Family; [pp = plant parasite; blank = other]; GenBank Accession Number) Taxonomic level of identification (species or genus) and then the different SNPs. The SNPs are indicated as the nucleotide position numbered like in the respective GenBank entry, stating the different nucleotide. LSU, 5'-end indicates that the respective GenBank entry and the respective SNPs relate to the LSU rDNA; in the other cases, the SNPs relate to the SSU rDNA.
*Globoder a pallida* (Heteroderidae; [pp]; AY593874) species
   136T; 651C; 672T.
*Globodera rostochiensis* (Heteroderidae; [pp]; AY593881) species
   171T; 650T; 671C.
*Heterodera schachtii* (Heteroderidae; [pp]; AY284617) species
   139G; 414G.
*Rotylenchus sp*. (Hoplolaimidae; [pp]; AY284608) species
   318G; 419G; 463C; 616T; 145A; 155T; 168T; 173C; 227A; 248A; 254T; 256G; 302T; 303G; 304G;
   452A; 471T; 516C; 526C; 616T; 630G; 646G; 649A; 660T; 661G; 691T; 864G; 891C; 892T; 1022A;
   1024T; 1034T; 1038A; 1063T; 1197G; 1321G; 1322C; 1326A; 1332G; 1334T; 1345G; 1346G; 1450C;
   1473C; 1518G; 1531T; 1544A; 1578C; 1607G; 1660C; 1662C; 1663G; 1681G; 1684C.
*Helicotylenchus canadensis* (Hoplolaimidae; [pp]; AY284605) species
   10G; 213A; 580T; 1447A; 1558C; 1567T.
*Helicotylenchus vulgaris* (Hoplolaimidae; [pp]; AY284607) species
   410C; 530A; 664C; 1681C.
*Helicotylenchus pseudorobustus;* Hoplolaimidae; [pp]; AY284606) species;
   24C; 75C; 76T; 197C; 198A; 199T; 201T; 214C; 218A; 219C; 319T; 320G; 421G; 424G; 425C; 465C;
   614C; 616A; 618T; 620G; 628C; 631T; 633T; 642T; 670G; 684T; 685G; 686T; 687G; 688C; 689T:
   691G; 692C; 693G; 695G; 696G; 698T; 699A; 700T; 702C; 704A; 705G; 1345C; 1352C; 1353A;
   1665T; 1666C; 1669G; 1670T; 1671T; 1674C; 1680A; 1681C; 1682A; 1686G; 1687T; 1688T; 1690C;
   1692G; 1693C; 1713T.
*Rotylenchus goodeyi* (Hoplolaimidae; [pp]; AY284609) species
   99C; 226C; 307G; 378C; 379A; 380C; 503T; 506C; 508G; 513C; 522C; 536G; 540C; 554C; 556C;
   559C; 561T; 563A; 564C; 580T; 584C; 585A; 592G; 594C; 599C; 601A; 603G; 634C; 648C; 656C;
   1213A; 1540C; 1543G; 1544C; 1551A; 1563C; 1566C; 1574C.
*Rotylenchus uniformis* (Hoplolaimidae; [pp]; AY593882) species
   101C; 141T; 437T; 476T; 603T; 610T; 615A; 632A; 633T; 637C; 645T; 648C; 655C; 656C; 684A;
   687G; 688T; 692T; 695A; 1326T; 1329G; 1509T; 1532A; 1614T; 1617C; 1635T; 1638T; 1646C;
   1651C; 1661A; 1670C; 1672A; 1693A; 1694T.
*Pratylenchus thornei* (Pratylenchidae I; [pp]; AY284612) species
   154C; 162G; 226A; 236T; 251A; 269T; 295G; 420G; 609C; 612T; 613G; 627C; 628G; 629C; 642G;
   644G; 646C; 649A; 668A; 671C; 672C; 673C; 676C; 677G; 688C; 689A; 690C; 692G; 695G; 696T;
   699T; 628G; 757C; 794A; 813G; 850T; 859G; 894A; 901C; 1012T; 1022A; 1023C; 1024A; 1038A;
   1061A; 1339G; 1647T; 1652G; 1658T; 1662G; 1669C; 1670T; 1673C; 1686G.
*Pratylenchus pratensis* (Pratylenchidae I; [pp]; AY284611) species
   37T; 40A; 45T; 66T; 91A; 112C; 181A; 182C; 187A; 191C; 308A; 309A; 310T; 335A; 341A; 356T;
   363T; 389G; 501A; 502C; 503G; 505A; 506G; 532T; 545T; 560A; 564T; 565G; 566C; 567A; 574A;
   575T; 579T; 583T; 586G; 587T; 627A; 633A; 914T; 918A; 950C; 1201C; 1209T; 1216T; 1218A;
   1220T; 1221G; 1507A; 1538T; 1546A; 1549A; 1552C; 1556A; 1558C; 1561T; 1569A.
*Pratylenchus crenatus* (Pratylenchidae I; [pp]; AY284610) species
   157C; 159G; 183C; 193T; 288C; 301A; 303C; 308G; 427C; 428A; 458A; 468A; 475T; 618G; 620T;
   622T; 623G; 624C; 632G; 641C; 643T; 652C; 656G; 658A; 659C; 688T; 693A; 700A; 701C; 702A;
   704A; 705A; 706A; 707T; 713G; 770C; 774T; 775T; 805C; 808A; 864T; 869G; 872A; 900T; 903C;
   909G; 1019T; 1037T; 1040G; 1041T; 1042C; 1058A; 1330G; 1331A; 1344A; 1352G; 1353C; 1498C;
   1665G; 1668G; 1672A; 1673T; 1682A; 1683T; 1686T; 1688A; 1691C.
*Hirschmanniella sp1* (Pratylenchidae I; [pp]; AY284614) species
   98C; 100T; 149C; 151A; 157C; 166G; 175G; 198T; 206A; 208T; 209G; 210A; 298T; 424G; 425C;
   426G; 615T; 621G; 642T; 643G; 644C; 651C; 653A; 654C; 656T; 671C; 674A; 675T; 684A; 686T;
   692A; 699C; 700A; 701A; 703T; 714A; 868T; 869T; 893A; 894C; 910C; 1032T; 1034A; 1037A;
   1203A; 1220A; 1338C; 1339T; 1353C; 1354G; 1355A; 1371C; 1381T; 1382C; 1573T; 1583A; 1643G.
*Meloidogyne incognita* (Meloidogynidae; [pp]; AY284621) species
   454T; 623A; 785G; 1134A; 1335A; 1498T; 1690C.
*Meloidogyne chitwoodi* (Meloidogynidae; MeloChi7; [pp]; AY593889) species
   123T; 133A; 137C; 169C; 185C; 186T; 293T; 436A; 532T; 594T; 619A; 644A; 722A; 1114G; 1643T.
*Meloidogyne fallax* (Meloidogynidae; MeloFal1; [pp]; AY593895) species
   226C; 304G; 1631T.
*Meloidogyne hapla* (Meloidogynidae; MeloHap4; [pp]; AY593892) species
   18T; 19A; 89C; 168A; 667C; 669G; 676G; 679A; 723A; 754T; 999T; 1000C; 1295G; 1305C; 1312T;
   1314G; 1336C; 1337T; 1448T; 1612C; 1617A; 1628C.
*Meloidogyne minor* (Meloidogynidae; MeloMin; [pp]; AY593899) species
   75C; 136C; 487G; 643A; 1013G; 1060A; 1306T; 1307G; 1310A; 1623T; 1639T; 1640T; 1641T;
   1642T; 1643A; 1644A; 1645A.
*Meloidogyne naasi* (Meloidogynidae; [pp]; AY593900) species
   76T; 224C; 225A; 633T; 1007A; 1308T; 1309A; 1311A; 1312A; 1632A; 1636A; 1640A; 1644A;
   1645T; 1646T; 1647T.
*Macrotrophurus arbusticola* (Telotylenchidae I; [pp]; AY284595) species
   98T; 1321G; 1337T; 1440G; 1491C; 1654A.
*Neodolichorhynchus lamelliferus* (Telotylenchidae I; [pp]; AY284598) species
   10G; 75A; 93C; 131C; 501T; 509T; 528C; 552A; 553A; 560C; 584C; 907A; 1209T; 1214G; 1217C;
   1220A; 1223G; 1542A; 1557C.
*Sauertylenchus maximus* (Telotylenchidae I; [pp]; AY284602) species
   666T; 667T; 679T; 698T; 699A; 1021T; 1315A; 1668A.
*Bitylenchus dubius;* (Telotylenchidae I; [pp]; AY284601) species
   41A; 42T; 89A; 90T; 100C; 111C; 113G; 127C; 157G; 189A; 190A; 246C; 258T; 458A; 459C; 628C;
   635C; 656T; 660T; 666A; 668G; 672A; 676T; 687G; 689A; 690A; 692C; 693G; 697C; 699C; 701A;
   705T; 711C; 727A; 1008T; 1032A; 1185C; 1186T; 1317G; 1565T; 1606A; 1625T; 1654A; 1663C;
   1710T.
*Trophurus imperialis* (Telotylenchidae I; [pp]; AY284600) species
   49G; 55A; 75A; 214C; 217A; 225C; 245A; 258T; 295C; 420T; 421G; 422C; 423G; 456C; 457T; 526C;
   613A; 628T; 651C; 664A; 671G; 680T; 693A; 700T; 701T; 705C; 706A; 707G; 710C; 711T; 992A.
*Paratylenchus straeleni* (Paratylenchidae I; [pp]; AY284630) species
   34C; 40G; 99G; 203G; 211A; 233T; 248A; 485C; 619T; 625C; 626G; 663C; 1013A; 1022C; 1027T;
   1323G; 1330G; 1331T; 1333T; 1649T; 1666T; 1675C.
*Paratylenchus microdorus* (Paratylenchidae I; [pp]; AY284632) species
   39C; 105C; 111C; 219T; 635C; 674G; 679C; 680A; 692G; 997A; 1020C; 1319A; 1335T; 1664C;
   1673T.
*Loofia thienemanni* (Hemicycliophoridae; [pp]; AY284629) species
   95A; 96C; 98C; 99A; 105A; 136T; 157A; 229C; 250G; 353C; 368G; 484A; 610C; 624G; 640T; 679T;
   743T; 748A; 1321A; 1322T; 1329A; 1330T; 1653T; 1657C; 1658A; 1676T.
*Mesocriconema xenoplax* (Criconematidae (Siddiqi); [pp]; AY284625) species
   26C; 32T; 37C; 40G; 138A; 140G; 150A; 151T; 152T; 154A; 156T; 157T; 160A; 161T; 232G; 253C;
   329G; 380C; 449T; 450G; 466C; 467A; 630A; 644G; 669C; 673G; 688C; 693G; 778C; 784C; 785A;
   1021A; 1023C; 1024A; 1313A; 1316A; 1444A; 1645T; 1654T; 1665A; 1678C; 1700A.
*Hemicriconemoides pseudobrachyurus* (Criconematidae (Siddiqi); [pp]; AY284622) species
   88G; 95A; 142G; 171G; 203T; 206C; 217C; 222C; 254A; 255C; 259C; 361T; 447T; 463A; 670C;
   671C; 1017C; 1018T; 1019A; 1020C; 1329T; 1331T; 1453C; 1654C; 1670T.
*Malenchus andrassyi* (Tylenchidae I; [pp]; AY284587) species
   25G; 27A; 29G; 47C; 66G; 80C; 89G; 123G; 305G; 454C; 455A; 495T; 499C; 512A; 514C; 535G;
   541C; 558G; 560G; 561G; 564C; 565A; 566A; 573G; 576G; 577C; 585G; 654G; 688C; 697G; 713G;
   732C; 774C; 777G; 1051A; 1057G; 1083C; 1086G; 1096T; 1184G; 1185T; 1195G; 1196A; 1198C;
   1211G; 1212C; 1215C; 1239T; 1240C; 1337G; 1345G; 1351G; 1389G; 1402C; 1525G; 1529A;
   1534C; 1536T; 1545G; 1550C; 1553C; 1556C; 1557C.
*Scutylenclaus quadrifer* (Telotylenchidae II; [pp]; AY284599) species ,
   81C; 89C; 91G; 103A; 163G; 172T; 502T; 1127A; 1158C; 1204C; 1217G; 1218C; 1395G; 1576G.
*Nagelus obscurus* (Telotylenchidae II; [pp]; AY593904) species
   186A; 226A; 591A; 592T; 631T; 1007A; 1299T; 1303A; 1304T; 1312A; 1315T; 1316G; 1640C.
*Telotylenchus ventralis* (Telotylenchidae II; [pp]; AY593905) species
   9T; 20C; 23G; 79A; 136G; 139A; 185C; 188G; 382A; 393A; 439A; 440C; 442A; 595C; 596T; 624T;
   625G; 626A; 627G; 637C; 643C; 645C; 649G; 650T; 651G; 652T; 654A; 658C; 669G; 674A; 675T;
   677T; 678A; 679C; 680A; 681T; 683T; 684G; 688A; 866T; 1006C; 1298A; 1319G; 1320G; 1621A;
   1625C; 1633C; 1637T; 1639G; 1650G; 1662G; 1666T; 1667T.
*Merlinius brevidens* (Telotylenchidae III; [pp]; AY284597) species
   96T; 203C; 245A; 611T; 1226G; 1322T; 1330A; 1393A; 1503T; 1653A; 1677A.
*Cephalenchus hexalineatus* (Tylodoridae; [pp]; AY284594) species
   38C; 41G; 42C; 76G; 96A; 115A; 125T; 226T; 268C; 419T; 420G; 450T; 460T; 468A; 610C; 616T;
   622G; 625T; 629C; 630T; 632A; 633A; 636T; 637C; 643A; 644C; 645T; 646T; 647C; 657G; 660T;
   670T; 671C; 672T; 681T; 691A; 692T; 693T; 706C; 707G; 719T; 807C; 1015A; 1029T; 1136T;
   1314G; 1316T; 1319G; 1326T; 1327T; 1328G; 1329C; 1330G; 1332A; 1334C; 1425T; 1452T; 1457C;
   1635C; 1654A; 1657G; 1664C.
*Ditylenchus dipsaci* (Anguinidae II; [pp]; AY593906) species
   21C; 129C; 146G; 597C; 598T; 659T; 662C; 664T; 816C; 833C; 901G; 1167A; 1202T; 1307C;
   1312A; 1449A; 1463A; 1530A; 1618C; 1628C; 1656T; 1669C.
*Ditylenchus destructor* (Anguinidae II; [pp]; AY593912) species
   23T; 50C; 64G; 79A; 80A; 173G; 179C; 181C; 184G; 230C; 255G; 287G; 301T; 407T; 424G; 445T;
   446A; 597C; 609T; 610G; 619C; 620A; 628A; 632T; 634T; 647T; 659T; 675C; 678A; 777C; 798G;
   815T; 970A; 984T; 992A; 1002A; 1009A; 1016T; 1026T.
*Ditylenchus sp1* (Anguinidae II; [pp]; AY284637) genus
   32T; 51A; 85C; 171C; 175C; 176T; 183C; 195G; 373T; 454G; 488T; 501G; 526T; 540T; 557C; 579T;
   734T; 778A; 1194C; 1337C; 1347C; 1394A; 1411T; 1541T; 1542C; 1552C; 1553G; 1593G.
*Anguina tritici* (Anguinidae II; [pp]; AY593913) species
   195C; 196C; 197T; 226C; 238C; 641A; 648T; 683A; 753C; 757C; 993A; 1016T; 1025T; 1275G;
   1484C.
*Nothotylenchus acris* (Anguinidae II; [pp]; AY593914) species
   93C; 99A; 142T; 143A; 144C; 146T; 151G; 190G; 227T; 331G; 418G; 436A; 439G; 490C; 601A;
   616T; 618G; 629C; 642C; 644A; 646C; 657G; 658G; 684T; 687T; 689T; 690G; 693G; 694C; 697C;
   698G; 699A; 737C; 790T; 794C; 795T; 796G; 813T; 834A; 835G; 838T; 892C; 900A; 925A; 930T;
   985T; 1192G; 1264T; 1308T; 1317A; 1336G; 1337T; 1338T; 1339G; 1340T; 1341G; 1347C; 1350A;
   1375A; 1462A; 1464T; 1470G; 1472T; 1485C; 1656G; 1657A; 1664C; 1665C; 1666T; 1669A; 1670T;
   1671T; 1672G; 1677C; 1680A; 1681C; 1693G.
*Filenchus thomei* (Tylenchidae II; [pp]; AY284591) species
   92C; 528G; 604T; 661C; 768A; 1230C; 1512C.
*Filenchus filiformis* (Tylenchidae II; [pp]; AY284592) species
   131G; 472C; 701A; 1047A; 1315C; 1322A.
*Tylenchus sp.* (Tylenchidae II; [pp]; AY284589) species
   114T; 210C; 631G; 632C; 633A; 637T; 640A; 694C; 1181C; 1232C; 1351C; 1452T; 1685C.
*Ottolenchus discrepans* (Tylenchidae II; [pp]; AY284590) species
   24C; 34G; 38C; 40C; 41A; 42G; 43C; 97C; 141G; 143A; 145G; 151G; 152T; 154G; 155C; 160C;
   193C; 197C; 201G; 202C; 235G; 332C; 388G; 414G; 416C; 418T; 435G; 485G; 607C; 608C; 610C;
   611G; 613G; 618T; 620T; 624C; 625C; 626A; 627C; 632G; 633A; 634G; 635C; 643C; 644C; 645G;
   647G; 648G; 656G; 663C; 665C; 666G; 675T; 676T; 677G; 678C; 679A; 680A; 681A; 683G; 684G;
   689A; 690T; 691T; 692C; 693G; 698A; 703G; 741C; 746C; 813G; 824T; 889C; 892G; 914C; 1017C;
   1018A; 1183C; 1184G; 1201C; 1206C; 1299G; 1309C; 1311A; 1312G; 1313T; 1317C; 1318C; 1319C;
   1320G; 1325A; 1329A; 1330C; 1331T; 1335G; 1351C; 1352A; 1357C; 1439G; 1440G; 1449C; 1459C;
   1470A; 1489C; 1554C; 1641C; 1643G; 1645C; 1647C; 1648T; 1664C; 1665G; 1666C; 1667T; 1668C;
   1669A; 1671C; 1674C; 1675T.
*Tylenchus davainei* (Tylenchidae II; [pp]; AY284588) species
   24G; 40C; 78G; 81C; 95G; 103A; 116A; 141T; 150G; 151A; 163G; 164C; 165G; 166G; 185G; 192G;
   197G; 198T; 199T; 201T; 206G; 207T; 208A; 209A; 210A; 211A; 212A; 214C; 215T; 217C; 218A;
   220G; 222C; 231G; 232C; 255C; 261G; 271A; 279G; 304C; 315T; 431G; 433T; 462A; 463G; 464A;
   465G; 468C; 470C; 478T; 480T; 481T; 482C; 483T; 486T; 517T; 520A; 522C; 524A; 570T; 582T;
   583A; 584C; 621T; 641A; 642A; 654T; 655T; 656G; 660C; 662T; 666A; 667A; 668T; 670A; 671A;
   678C; 680C; 681A; 684T; 686G; 698G; 699T; 707T; 709A; 710T; 711A; 714T; 720A; 724G; 735G;
   764A; 816G; 825G; 830C; 853G; 871T; 903A; 910C; 913C; 969A; 1029T; 1036T; 1037G; 1038T;
   1039G; 1051A; 1081G; 1185C; 1195G; 1218A; 1246T; 1333G; 1341A; 1343A; 1345T; 1349C; 1350G;
   1351T; 1353C.
*Coslenchus franklinae* (Tylenchidae II; [pp]; AY284583) species
   39A; 203G; 1324C.
*Coslencltus costatus* (Tylenchidae II; [pp]; AY284581) species
   535C; 654C; 1324C; 1479G; 1539T.
*Lelenchus leptosoma* (Ecphyadophoridae I; [pp]; AY284584) species
   10G; 161C; 344T; 542T; 1209T.
*Boleodorus thylactus* (Tylenchidae IV; [pp]; AY593915) species
   12C; 19C; 22G; 175G; 176C; 177G; 179C; 182A; 183C; 184G; 185G; 186T; 189T; 235C; 365G; 412C;
   430G; 441G; 601G; 603C; 617G; 619C; 621C; 622A; 626C; 631C; 639C; 640A; 644G; 645C; 651C;
   653G; 664G; 674T; 683C; 691C; 773G; 774G; 780C; 781G; 788G; 848C; 999C; 1018C; 1019A;
   1030T; 1039G; 1320C; 1323C; 1336G; 1450C; 1481A; 1651G; 1652A; 1655C; 1656G; 1660C; 1661A;
   1665T; 1667C; 1670C; 1672C; 1706T; 1710C.
*Aglenchus sp.* (Tylenchidae III; [pp]; AY284586) species
   157A; 460C; 630A; 646T; 676G; 685C; 982A.
*Neopsilenchus magnidens* (Tylenchidae III; [pp]; AY284585) species
   41T; 82C; 157A; 193A; 204C; 423T; 462T; 553G; 615T; 616G; 619T; 659C; 660A; 671C; 673C;
   690T; 694G; 742T; 1018T; 1030C; 1038A; 1217G; 1329C; 1330T; 1331A; 1332C; 1335C; 1340T;
   1347T; 1349G; 1371C; 1444G; 1466T; 1467C; 1468C; 1569T; 1636T; 1657C; 1664C; 1670A; 1673C;
   1677A; 678C; 1688C.
*Pseudhalenchus minutus* (Anguinidae III; [pp]; AY284638) species
   30T; 31C; 41T; 98G; 99T; 160A; 245A; 277C; 317A; 319T; 439G; 452T; 469A; 610T; 616G; 619T;
   629G; 630T; 631T; 632A; 636C; 646A; 650T; 654A; 672T; 674G; 676A; 680T; 692T; 694T; 696A;
   818A; 845C; 850A; 851C; 893G; 894T; 915G; 999T; 1007A; 1012T; 1017A; 1022A; 1026G; 1032A;
   1042T; 1049T; 1054A; 1061A; 1062C; 1065A; 1228C; 1229G; 1230A; 1323T; 1324G; 1335T; 1337A;
   1338T; 1339A; 1459A; 1462T; 1463C; 1471A; 1493C; 1551A; 1626T; 1661C; 1703A.
*Ecphyadophora sp.* (Ecphyadophoridae III; [pp]; AY593917) species
   12G; 18G; 19C; 20G; 21C; 22A; 24G; 58G; 80C; 136C; 163G; 164C; 165G; 183G; 184C; 185A; 186A;
   187G; 188G; 202T; 204G; 219C; 220T; 599C; 600C; 610C; 612A; 622C; 623G; 625G; 645G; 646G;
   647C; 1007T; 1008A; 1009A; 1010A; 1011A; 1309C; 1311C; 1316G; 1317C; 1319A.
*Aphelenchus avenae* (Aphelenchidae; [pp]; AY284639) species
   163C; 179G; 575G; 592T; 594C; 629T; 638T; 648A; 764C; 1144G; 1268C; 1274T; 1284A; 1290G;
   1421A.
*Paraphelenchus* (Paraphelenchidae; [pp]; AY284642) species
   30T; 43C; 98T; 99T; 106A; 155A; 162T; 333A; 619T; 626A; 633T; 634C; 642A; 645A; 650T; 669T;
   674A; 679T; 682A; 689C; 691T; 698C; 699A; 808A; 1012T; 1023A; 1315T; 1335A; 1625A; 1653A;
   1655T; 1666A; 1671C; 1675A; 1677C; 1696T.
*Heterocephalobus* (Cephalobidae; [ ]; AY284668) genus
   107C; 169C; 466T; 467C; 617T; 620T; 638T; 657G; 690G; 692T; 837G; 1331G; 1332C; 1334T;
   1340T; 1344C; 1652T; 1655A; 1656G; 1658C; 1659G; 1667A; 1676T; 1685T.
*Eucephalobus* (Cephalobidae; [ ]; AY284666) genus
   630T; 666T; 667C; 710A.
*Cephalobus* (Cephalobidae; [ ]; AY284662) genus
   626T; 631C; 637A; 1013T; 1037A; 1320G; 1336G; 1670A; 1676A.
*Acrobeles* (Cephalobidae; [ ]; AY284671) genus
   152C; 208A; 248T;'619T; 628T; 661A; 662C; 705T; 1328T; 1329A; 1460A; 1474G; 1624T; 1648A;
   1651A; 1655A; 1660T; 1662T; 1669A; 1673T; 1678T.
*Acrobeloides* (Cephalobidae; [ ]; AY284672) genus
   462A; 857T; 889A.
*Zeldia* (Cephalobidae; [ ]; AY284675) genus
   75G; 99C; 117C; 184G; 465C; 635T; 636T; 637T; 644C; 1333C; 1682A; 1686T.
*Chiloplacus* (Cephalobidae; [ ]; AY284677) genus
   210C; 241C; 647T; 688T; 840C; 1654A; 1683T.
*Ceruidellus* (Cephalobidae; [ ]; AY284674) genus
   25G; 40T; 335G; 337A; 340C; 344A; 514C; 519C; 527G; 543C; 545A; 561G; 567A; 570C; 589T;
   633A; 635T; 639G; 694C; 896T; 920A; 1075G; 1209C; 1211T; 1212A; 1220C; 1330A.
*Drilocephalobus* (Ostellidae; [ ]; AY284678; genus
   41T; 203A; 266C; 452A; 496C; 498A; 502T; 527T; 528A; 533T; 535G; 541A; 542A; 547A; 573C;
   574G; 622C; 742G; 770C; 1091G; 1204A; 1209A; 1216T; 1221G; 1337T; 1344A; 1354A; 1357C;
   1358G.
*Aphelenchoides* 1 (Aphelenchoididae I; [pp]; AY284646) species
   146G; 147C; 148A; 161T; 165T; 187C; 188T; 191C; 451A; 461G; 487G; 501C; 604T; 608A; 609A;
   613T; 628A; 634T; 675A; 1010C; 1286G; 1287A; 1288A; 1310T; 1389A; 1618C; 1624A; 1636A.
*Aphelenchoides* blastophtorus (Aphelenchoididae I; [pp]; AY284644) species
   145G; 146C; 147A; 160T; 164T; 186C; 187T; 190C; 450A; 459A; 460G; 486G; 500C; 603T; 607A;
   608A; 612T; 627A; 633T; 650A; 651A; 672G; 674A; 1009C; 1285G; 1286A; 1309T; 1388A; 1616C;
   1622A; 1634A.
*Aphelenchoides fragariae* (Aphelenchoididae I; [pp]; AY284645) species
   131G; 135T; 139T; 142T; 144T; 157A; 158A; 161A; 166C; 183T; 208G; 226C; 230T; 233A; 234C;
   244A; 438C; 457A; 483A; 595T; 603G; 613T; 620A; 630C; 647C; 648G; 671G; 986A; 996T; 1006A;
   1135C; 1284T; 1294C; 1300A; 1385G; 1613T; 1631C; 1635G; 1636C.
*Aphelenchoides bicaudatus* (Aphelenchoididae I; [pp]; AY284643) species
   30C; 80C; 84A; 92A; 136C; 142G; 143G; 159C; 161T; 163C; 214C; 215A; 227G; 229T; 233C; 236A;
   246A; 253G; 264C; 270T; 278A; 298C; 299A; 470C; 471C; 472G; 482G; 486C; 487G; 488G; 611T;
   612G; 634T; 635G; 653A; 654G; 655T; 656T; 819C; 821G; 1228G; 1230C; 1280C; 1281A; 1586G;
   1613C; 1620G.
*Bursaphelenchus mucronatus* (Acugutturidae ; [pp]; AY284648) species
   152C; 187C; 205A; 218A; 292T; 452A; 456A; 481A; 593C; 594A; 599A; 602T; 614C; 617T; 635T;
   636T; 655T; 661C; 668G; 677A; 715T; 772T; 875T; 956T; 1025T; 1315T; 1420G; 1639G; 1658T;
   1660G.
*Seinura* (Seinuridae; [ ]; AY284651) genus
   21G; 26G; 31G; 32T; 33T; 34A; 43T; 45G; 73T; 75T; 109T; 110A; 111G; 138C; 175G; 176C; 483G;
   484A; 486A; 488C; 489G; 495G; 509T; 510T; 511C; 568G; 570G; 1191T; 1192T; 1333G; 1334G;
   1542A.
*Laimaphelenchus* penardi (Aphelenchoididae II; [pp]; AY593918) species
   35G; 41G; 90T; 94A; 98A; 141C; 149C; 152G; 162T; 184G; 187A; 191C; 192T; 212A; 225C; 233A;
   238A; 303C; 305A; 319G; 328C; 441C; 475G; 499A; 500G; 542C; 623T; 624T; 630G; 632A; 644A;
   645C; 648G; 652G; 654A; 663G; 684G; 685C; 996C; 1184C; 1278G; 1290C; 1346A; 1364C; 1443A;
   1444C; 1455G; 1594C; 1631G; 1635C; 1653G; 1654A; 1670G.
*Plectonchus* (Brevibuccidae I; [ ]; AY593920) genus
   37A; 51G; 58C; 144C; 248A; 417T; 479G; 485A; 485C; 487G; 491A; 492A; 536T; 537T; 591A; 603G;
   608C; 655G; 666T; 669C; 766A; 1000G.
*Panagrolaimus* (Panagrolaimidae II; [ ]; AY284681; genus
   42T; 57T; 78A; 97T; 147A; 152A; 153T; 172A; 179A; 188T; 189T; 198G; 200A; 201T; 202T; 210A;
   211T; 217G; 224A; 234A; 237A; 266C; 299T; 304A; 308A; 312A; 314T; 318T; 341G; 343T; 352A;
   368T; 471C; 472G; 498T; 506T; 508A; 617G; 622T; 626A; 633T; 636A; 642T; 643G; 645A; 646T;
   647T; 651A; 655A; 662A; 674A; 698A; 786T; 791A; 817T; 854C; 863A; 891T; 1029A; 1033T; 1047A;
   1224T; 1333C; 1334A; 1339T; 1341C; 1344T; 1345T; 1367G; 1368C; 1369G; 1370T; 1371G; 1373T;
   1374T; 1375C; 1376T; 1377T; 1378T; 1379T; 1404C; 1496A; 1565A; 1663G.
*Steinernema glaseri* (Steinernematidae; [ ]; AY284682) species
   36G; 67C; 74A; 81G; 90A; 93T; 100T; 102A; 138A; 151T; 154C; 160T; 163C; 193G; 203T; 209A;
   210T; 223T; 253T; 274T; 299T; 311A; 425G; 456T; 472A; 565A; 571T; 575C; 624G; 633A; 648C;
   649T; 654G; 657G; 658C; 691C; 727T; 799G; 800A; 834G; 841T; 883G; 956G; 987C; 994C; 1001C;
   1019T; 1020C; 1028A; 1049A; 1228C; 1234A; 1322G; 1325T; 1334T; 1338G; 1342G; 1346C; 1363C;
   1369A; 1422T; 1463G; 1490T; 1520T; 1528T; 1537A; 1574A; 1646A; 1651G; 1668C; 1690C.
*Rhabditella* (Rhabditidae I; [ ]; AY284654) genus
   37T; 38C; 69T; 71T; 74C; 82A; 87T; 137A; 138T; 148T; 186A; 214G; 227C; 243G; 296C; 394C;
   396T; 433T; 535A; 581G; 591C; 598G; 604C; 626A; 672A; 696C; 719C; 725G; 754A; 986C; 1285A;
   1420A; 1619C; 1620A.
*Diploscapter* (Diploscapteridae; [ ]; AY593921) genus
   8C; 9A; 12A; 29G; 48C; 56C; 57G; 118A; 171T; 198C; 216T; 259C; 267C; 279G; 304T; 385C; 461G;
   577G; 578A; 580A; 593T; 610C; 611A; 615G; 616C; 618A; 629A; 632C; 634G; 636G; 637C; 639A;
   641G; 646A; 647A; 648A; 651A; 653A; 655A; 664T; 677A; 687A; 689A; 699T; 723T; 773G; 814T;
   846A; 887G; 981A; 1273A; 1276A; 1281A; 1293A; 1294G; 1301G; 1319T; 1619T; 1632A.
*Cruznema* (Rhabditidae III; [ ]; AY284655) genus
   25T; 31A; 39A; 71T; 75A; 78C; 87A; 129A; 135T; 142C; 143G; 180T; 182G; 202G; 206T; 209T;
   212G; 274G; 279G; 378T; 389T; 391A; 433C; 455T; 456A; 457C; 462G; 469T; 473G; 475A; 476A;
   535C; 536T; 541A; 553A; 574A; 576A; 591A; 601C; 603A; 614A; 616G; 617T; 627A; 632T; 633C;
   641C; 642A; 643C; 644T; 645T; 650C; 661G; 663G; 704C; 714C; 726G; 732A; 735G; 796A; 800G;
   829C; 830C; 839G; 840G; 875A; 886C; 889T; 890C; 976T; 980T; 995G; 999A; 1282A; 1291A;
   1293G; 1294T; 1299A; 1324C; 1328A; 1392C; 1414G; 1426C; 1427T; 1435G; 1528C; 1575G; 1604T;
   1611C; 1613A; 1617G; 1619T; 1630G; 1641T.
*Mesorhabditis* (Rhabditidae III; [ ]; AY284660) genus
   22C; 24T; 25G; 70C; 80T; 178C; 179G; 372A; 517T; 528C; 682C; 718T; 724A; 1035G; 1128C;
   1129C; 1181T; 1190C; 1297C; 1477G; 1520C.
*Diplogaster* (Diplogastridae; [ ]; AY284688) genus
   16A; 21G; 22T; 26A; 51T; 64C; 65T; 100A; 127G; 128T; 130A; 134A; 140T; 141T; 147A; 178T;
   179T; 187A; 266G; 274G; 288G; 331A; 380T; 418G; 440C; 463A; 469C; 470C; 474A; 593C; 594A;
   608G; 609T; 616T; 625T; 628T; 629T; 630T; 641A; 652G; 665C; 677T; 713G; 721A; 727T; 759G;
   987A; 994C; 1297A; 1330T; 1442A; 1467A; 1609T; 1623A; 1634G; 1642C.
*Pristionchus* (Diplogastridae; [ ]; AY593923) genus
   16T; 17G; 19G; 22A; 25A; 48T; 54C; 56C; 68A; 69G; 81A; 97T; 139T; 142T; 171T; 204T; 207G;
   236C; 237T; 239G; 247G; 248G; 262A; 284T; 332C; 333T; 351T; 459C; 464C; 470G; 471A; 481A;
   489G; 531C; 535C; 591C; 595A; 600T; 608A; 617T; 662A; 667T; 673A; 681T; 690T; 694A; 723A;
   724T; 752C; 772T; 788A; 797C; 798T; 954T; 1005T; 1050T; 1128T; 1138A; 1148A; 1167T; 1215C;
   1221T; 1275G; 1341T; 1399A; 1408A; 1415T; 1442T; 1444T; 1454T; 1457T; 1467A; 1472A; 1474G;
   1515A; 1524T; 1590C; 1609T; 1613G; 1615G; 1616T; 1624C
*Mononchoides* (Diplogastridae; [ ]; AY593924) genus
   21C; 62C; 76G; 129G; 131A; 138T; 182C; 219A; 293T; 585T; 586G; 645A; 648T; 662A; 668T; 714A;
   752C; 766G; 788T; 993T; 995G; 1425G; 1431G; 1433A; 1443T; 1448C; 1455C; 1624A; 1625A;
   1629A; 1632A; 1633A; 1644T.
*Bunonema* (Bunonematidae; [ ]; AY284661) genus
   34G; 37C; 39A; 43T; 46C; 51T; 74A; 75G; 83G; 92A; 99C; 112T; 138C; 141G; 143T; 169G; 188A;
   202G; 203T; 206T; 209T; 223C; 226T; 261T; 267A; 268C; 285C; 306C; 317G; 434A; 437G; 445A;
   447A; 448G; 454G; 462T; 468C; 492A; 495C; 496A; 558T; 576T; 605C; 609G; 629T; 634C; 637G;
   638C; 666C; 681G; 702A; 713C; 739G; 750A; 778G; 806C; 853C; 870A; 1006C; 1010A; 1011G;
   1012C; 1013A; 1021T; 1026G; 1027C; 1028C; 1035C; 1121C; 1194C; 1253C; 1290C; 1327G; 1345T;
   1346C; 1351C; 1355T; 1369G; 1418G; 1429C; 1430T; 1431T; 1432T; 1433G; 1434C; 1446C; 1447C;
   1448G; 1478A; 1479A; 1480A; 1481G; 1516G; 1537G; 1538A; 1539C.
*Teratocephalus* (Teratocephalidae; [ ]; AY284683) genus
   31T; 32G; 37A; 59A; 149A; 204C; 256T; 296A; 304T; 401T; 412A; 417A; 604C; 605A; 606A; 607A;
   608G; 610C; 649G; 650G; 678G; 705G; 746G; 762G; 781A; 787T; 1326C; 1328T; 1337T; 1338C;
   1372A; 1373T; 1453T; 1454G; 1672T; 1702C.
*Tylocephalus* (Plectidae; [ ]; AY284707) genus
   247C; 264A; 699C; 794C; 822G; 1321G; 1323A; 1335T; 1337C; 1557C; 1652C; 1655C; 1679G.
*Wilsonema* (Plectidae; [ ]; AY593927) genus
   206G; 1488C; 1663A; 1664G; 1671C; 1672T.
*Plectits rhizophilus* (Plectidae; [ ]; AY593928) species
   140T; 737T; 740C; 762T; 1320A; 1334T.
*Plectus aquatilis* (Plectidae; [ ]; AY284700) species
   141T; 208G; 738T; 741C; 763T; 793C; 1487T.
*Plectus cirratus* (Plectidae; [ ]; AY593930) species
   208G; 738T; 741C; 763T; 793C; 1467A.
*Anaplectus* (Plectidae; [ ]; AY284697) genus
   42C; 94T; 155G; 156G; 158A; 206C; 208A; 209C; 622T; 660C; 789T; 796C; 902A; 1016T; 1040A;
   1068A; 1142T; 1169G; 1314C; 1331G; 1341T; 1431T; 1447A; 1452A; 1488A; 1496T; 1651T; 1682A.
*Chronogaster* (Leptolaimidae; [ ]; AY593931) genus
   58T; 179T; 212A; 422A; 454A; 470T; 612T; 618C; 627A; 649G; 663G; 683C; 763G; 819C; 853C;
   1008C; 1018T; 1038A; 1209C; 1331C; 1366C; 1368T; 1446G; 1660A; 1680T.
*Aphanolaimus* (Halaphanolaimidae; [ ]; AY593932) genus
   41T; 45C; 46A; 94C; 141C; 143G; 158G; 192A; 195C; 202G; 203T; 205T; 240T; 256A; 304C; 455G;
   457A; 458G; 488G; 503C; 615G; 616C; 617T; 622C; 635G; 636C; 639C; 655C; 659C; 662G; 667C;
   680G; 697C; 708C; 709G; 752C; 785C; 799G; 840T; 1025C; 1026A; 1027C; 1029G; 1031C; 1032G;
   1036T; 1323G; 1334C; 1343A; 1358T; 1359T; 1361A; 1362C; 1365C; 1374G; 1375T; 1382A; 1457A;
   1458T; 1460C; 1642G; 1643T; 1655A; 1656C; 1660G; 1661T; 1662C; 1668C; 1675T; 1676C; 1677G;
   1679C; 1683G; 1684C; 1686C; 1688A; 1689G; 1696A; 1697C; 1699G.
Metateratocephalus (Euteratocephalidae; [ ]; AY284686) genus
   227T; 614A; 632T; 789G; 819C; 1302A; 1340T; 1442A; 1471T; 1493T; 1660T; 1669A.
*Euteratocephalus* (Euteratocephalidae; [ ]; AY284684) genus
   49G; 139G; 141A; 190C; 192A; 201G; 226C; 258T; 306T; 642C; 646C; 670A; 696A; 749T; 1321C;
   1335G; 1340C; 1486C.
Haliplectus (Haliplectidae; [ ]; AY593935) genus
   39G; 87G; 94C; 151C; 155G; 159A; 161G; 193A; 194G; 228A; 370T; 386A; 451A; 463C; 472T; 559A;
   561T; 564C; 571T; 572A; 574A; 579T; 611G; 620T; 621A; 631G; 632G; 633G; 634A; 635A; 636A;
   650T; 651T; 673A; 681G; 697A; 701T; 702C; 703A; 705C; 707G; 709A; 715A; 716C; 824G; 825C;
   906G; 946A; 1038C; 1039A; 1040T; 1054C; 1321T; 1330A; 1345C; 1346A; 1351A; 1352C.
*Eumonhystera* (Monhysteridae; [ ]; AY284692) genus ' .
   48C; 91C; 226T.
*Monhystera* (Monhysteridae; [ ]; AY593938) genus
   34G; 104T; 107C; 134T; 156T; 161A; 191A; 310T; 413T; 549T; 571A; 608A; 616T; 625A; 628A;
   648T; 659G; 663A; 677C; 774A; 781T; 783A; 977C; 987T; 1007G; 1025C; 1037A; 1039G.
*Theristus* (Xyalidae; [ ]; AY284693) genus
   41C; 96A; 97A; 191T; 200G; 240C; 613C; 614C; 649A; 650C; 902C; 1018C; 1019A; 1041T; 1042G;
   1342G.
*Aulolaimus* (Aulolaimidae; [ ]; AY284724) genus
   18T; 30T; 31G; 40C; 41T; 82C; 85C; 111C; 143A; 172C; 192G; 265T; 370A; 391A; 540T; 550G;
   551C; 628A; 642A; 688C; 710A; 733C; 734G; 741A; 744G; 768C; 771T; 778C; 791G; 883T; 949A;
   1116A.
*Cylindrolaimus* (Diplopeltidae; [ ]; AY593939) genus
   58T; 59C; 78A; 84C; 86T; 140C; 202T; 213G; 263C; 286T; 412A; 488T; 489C; 614A; 615G; 646C;
   647T; 885C; 886T.
*Choanolaimus* (Choanolaimidae; [ ]; AY284714) genus
   24G; 38T; 39T; 40A; 58A; 59A; 92G; 93T; 147T; 148T; 153A; 162A; 192T; 202T; 225C; 249A; 299C;
   300A; 452A; 567T; 570A; 607G; 608T; 615T; 645A; 653C; 656A; 665C; 739G; 798C; 1001T.
*Achromadora* (Achromadoridae; [ ]; AY593941) genus
   150A; 160C; 195C; 458C; 494C; 634C; 693C; 1325C; 1326T; 1354A; 1460G.
*Ethmolaimus* (Ethmolaimidae; [ ]; ; AY593942) genus
   31A; 38C; 39A; 43C; 145C; 150T; 152G; 255G; 450G; 463T; 642C; 649T; 703T; 704G; 742A; 743A;
   744A; 836C; 994C; 1165C; 1184G; 1185A; 1214C; 1219T; 1220T; 1225T.
*Dorylaimus* (Dorylaimidae; [ ]; (LSU, 5'-end); AY592994) genus
   144A; 407T; 491T; 541G; 563G; 577A; 596A; 647T; 655G; 715T; 727A; 730C; 748T; 749C; 875A;
   891C; 892G; 920C; 952A.
*Labronema* (Qudsianematidae; [ ]; (LSU, 5'-end); AY592996) genus
   111A; 465G; 491G; 514G; 541T; 542A; 587C; 637A; 682C; 759T; 911T; 920C.
*Paractinolaimus* (Actinolaimidae; [ ]; (LSU, 5'-end); AY592999) genus
   94C; 135A; 142T; 143G; 207T; 217A; 430T; 441T; 476A; 482A; 491A; 504A; 544A; 569A; 592A;
   593T; 597C; 598C; 604A; 637T; 667T; 668T; 692A; 695A; 700T; 710A; 712A; 717T; 857T; 863A;
   948T.
*Paraxonchium* (Aporcelaimidae; [ ]; (LSU, 5'-end); AY593001) genus
   47C; 67C; 68G; 70T; 72T; 77C; 78C; 79G; 143C; 386C; 400A; 404G; 423T; 424C; 450G; 432T; 441T;
   454G; 463C; 467A; 507T; 518G; 523C; 524G; 527T; 552T; 563G; 572T.
*Dorylaimoides* (Leptonchidae; [ ]; (LSU, 5'-end); AY593003) genus
   115A; 135T; 185T; 237T; 423G; 424T; 433C; 441A; 451C; 548C; 550A; 554T; 566A; 579A; 614A;
   649G; 650T; 658C; 667A; 668T; 670G; 692C; 716C; 757C; 833T; 891A; 900A.
*Mesodorylaimus* (Thornenematidae; [ ]; (LSU, 5'-end); AY593005) genus
   44C; 79T; 88C; 91T; 93T; 98C; 134T; 138T; 204G; 430T; 434C; 435G; 442A; 447A; 450G; 458T;
   459T; 461A; 463T; 472C; 498G; 503T; 562A; 580A; 589C; 594T; 596A; 598T; 625T; 633A; 635T;
   657T; 659T; 665G; 682A; 684T; 720T; 723A; 724T; 726A; 732C; 736G; 843A; 902T; 904T; 914A;
   916G; 918A.
*Eudorylaimus* (Qudsianematidae; [ ]; (LSU, 5'-end); AY593007) genus
   79C; 94G; 133A; 138C; 156T; 173G; 174T; 355A; 358T; 421C; 432G; 436C; 450A; 461G; 465T;
   468C; 469G; 472A; 488A; 526C; 527G; 528T; 534G; 565C; 609A; 614A; 615A; 635C; 648T; 650G;
   676C; 684C; 721C; 724G; 727T; 744G; 745C; 755C; 901G; 1108G; 475T; 588T; 595C; 597C; 637G.
*Opisthodorylaimus* (Thornenematidae; [ ]; (LSU, 5'-end); AY593008) genus
   421A; 422T; 437A; 471T; 595C; 596A; 630G; 634T; 635C; 637A; 668C; 693C; 725A; 756T; 780T.
*Oxydirus* (Belondiridae; [ ]; (LSU; 5'-end); AY593011) genus
   131C; 170T; 225C; 258C; 437G; 458T; 459T; 468C; 492T; 604C; 669T; 653A; 891G; 893A
*Oxydirus* (Belondiridae; [ ]; (LSU, 5'-end); AY593012) genus
   68C; 437A; 588T; 669C; 687C; 732G.
*Ecumenicus* (Thornenematidae; [ ]; (LSU, 5'-end); AY593013) genus
   89G; 93C; 134T; 140T; 142T; 204G; 337T; 399A; 406C; 408T; 421A; 423T; 429A; 435T; 469G;
   470C; 481T; 486A; 487C; 489G; 504G; 514T; 564T; 572T; 593C; 624A; 632C; 635A; 653C; 710C;
   711T; 727T; 750C; 755T; 762G; 765A; 785G; 835C; 855G; 908G; 914C; 918G; 991G.
*Allodorylaimus* (Qudsianematidae; [ ]; (LSU, 5'-end); AY593015) genus
   134C; 207G; 258T; 373G; 420T; 440T; 445T; 453A; 467A; 468A; 469A; 474T; 478A; 485T; 541A;
   544C; 558T; 564T; 594G; 596A; 605T; 622C; 633A; 640A; 652A; 663C; 664G; 674C; 678T; 723A;
   727A; 733C; 734G; 738A; 750T; 762T; 900T; 915C; 916T; 920A.
*Aporcelaimellus* (Aporcelaimidae; [ ]; (LSU, 5'-end); AY593017) genus
   143A; 257C; 420C; 421G; 427G; 438G; 442A; 452C; 461T; 464T; 465T; 470G; 480T; 485C; 564C;
   588A; 592C; 593G; 594T; 596G; 621T; 631T; 633T; 634A; 635T; 636T; 644C; 660C; 677T; 711A;
   726G; 907T; 911C; 913T; 914T; 920A.
*Aporcelaimellus* (Aporcelaimidae; [ ]; (LSU, 5'-end); AY593020) genus
   141A; 200A; 201C; 202G; 436C; 440A; 571A; 576T; 596T; 597T; 638C; 672A; 734C; 756T.
*Axonchium* (Belondiridae; [ ]; (LSU, 5'-end); AY593022) genus
   17T; 67G; 72C; 85T; 91T; 94T; 97A; 98C; 103C; 106A; 134C; 140G; 142G; 164C; 170C; 200C; 208G;
   213A; 224C; 279G; 399T; 402C; 428C; 434T; 435A; 436G; 437A; 438T; 443G; 453A; 461T; 465C;
   469T; 470C; 471G; 472A; 475G; 489A; 508T; 512G; 513T; 561G; 566T; 572T; 573T; 576T; 587C;
   588A; 594C; 595T; 596G; 628T; 633T; 646G; 647T; 648A; 651C; 657T; 671T; 678T; 685T; 693C;
   707T; 723A; 726C; 732C; 738A; 743C; 745C; 746C; 762C; 767C; 773C; 785G; 875T; 885A; 888C;
   889G; 901C; 903C; 908C; 909G; 910A; 914A; 916T; 918C; 919G; 930C; 949A; 965C; 970C; 1078G.
*Carcharodiscus* (Discolaimidae; [ ]; (LSU, 5'-end); AY593023) genus
   12A; 32T; 62G; 77A; 99A; 102A; 114T; 120A; 143A; 149T; 171T; 215C; 216A; 218A; 224T; 259T;
   320T; 374T; 387A; 390T; 397A; 419C; 424T; 430T; 439T; 436G; 444A; 445C; 446G; 449A; 455T;
   459C; 461T; 465T; 466A; 467G; 470C; 493T; 517T; 522A; 543A; 557G; 562A; 567A; 571A; 575C;
   576C; 590T; 595A; 619T; 628G; 629A; 631A; 638A; 640T; 656T; 658G; 675C; 676T; 689T; 694T;
   701T; 703C; 707T; 711A; 719C; 720T; 738A; 744A; 745T; 750C; 756T; 768A; 771T; 780A; 825G;
   826A; 834A; 839T; 846T; 869T; 870C; 875T; 880G; 881A; 884A; 890T; 898G; 904A; 905G; 906T;
   909T; 923T; 933G; 937T; 945A.
*Discolaimus* (Discolaimidae; [ ]; (LSU, 5'-end); AY593025) genus
   12G; 44G; 142C; 217C; 289C; 344G; 380T; 423A; 438A; 440T; 444G; 445T; 465T; 466C; 473A;
   482A; 495G; 557C; 565C; 574G; 576C; 600A; 623T; 628A; 629T; 635A; 640C; 661C; 672T; 676T;
   681A; 695C; 702C; 704T; 705A; 714A; 717C; 718A; 719T; 721C; 723A; 731G; 744G; 750A; 774C;
   848C; 870T; 875C; 880A; 884G; 897A; 898A; 904C; 907A; 908A; 911G; 912G; 915T; 916T; 934A;
   938A; 942G; 946C; 950C; 953T; 957T.
*Tylencholaimus* (Leptonchidae; [ ]; (LSU, 5'-end); AY593027) genus
   9C; 44C; 79C; 93C; 98C; 102C; 112G; 134A; 136A; 141C; 143A; 161C; 204C; 218A; 280G; 356A;
   381G; 403A; 406T; 407C; 411A; 424C; 426C; 428G; 438G; 439T; 441C; 442G; 443C; 445A; 446G;
   463G; 466A; 467G; 468T; 471A; 472C; 473C; 475T; 476G; 477T; 478T; 479A; 491C; 492A; 508G;
   509A; 537T; 538G; 547G; 549C; 579T; 580C; 592G; 593A; 608C; 633C; 638G; 639G; 644T; 660C;
   668A; 669C; 679A; 683G; 686C; 687A; 688T; 689A; 690C; 691G; 693C; 695C; 698A; 699T; 705T;
   710A; 711T; 716G; 720A; 722C; 723T; 724C; 725G; 728T; 729G; 730G; 731C; 741A; 742T; 750G;
   751C; 752A; 759T; 764C; 771C; 774G; 852A; 884C; 885A; 887C; 888C; 889T; 890C; 893T; 894G;
   897C; 898A; 911G; 914T; 917A; 918C; 921C; 922T; 943C; 944C; 952T.
*Chrysonema* (Chrysonematidae; [ ]; (LSU, 5'-end); AY593029) genus
   47A; 50C; 75A; 95A; 115A; 122A; 138T; 176A; 179T; 181A; 187G; 195C; 201T; 203T; 323G; 377G;
   380C; 389A; 397T; 400T; 401C; 402G; 404A; 413G; 416T; 420A; 423C; 424G; 425G; 426A; 427A;
   428T; 433T; 438C; 442T; 445A; 446G; 447C; 448G; 449T; 451G; 466T; 467A; 469T; 476T; 481A;
   485T; 490T; 491C; 513T; 520A; 521T; 523C; 532G; 543T; 550T; 551A; 556A; 573T; 581A; 598G;
   601A; 603T; 605T; 606A; 608A; 611T; 612T; 613A; 614A; 618A; 622A; 628A; 639A; 646G; 647C;
   653T; 662C; 665A; 668A; 669A; 670C; 671G; 672C; 686T; 691T; 692A; 693G; 696C; 701C; 702G;
   714G; 718T; 724T; 730T; 735A; 738T; 746A; 753G; 825C; 835A; 855T; 858T; 861G; 865A; 868T;
   872G; 903C; 914T; 944C; 946T.
*Sectonema* (Aporcelaimidae; [ ]; (LSU, 5'-end); AY593030) genus
   72C; 85T; 95C; 102A; 122A; 125A; 128T; 134T; 153A; 169T; 207G; 208A; 215A; 217T; 218A; 219T;
   220T.
*Prodorylaimus* uliginosus (Thornenematidae; [ ]; (LSU, 5'-end); AY593034) genus
   86C; 93C; 102G; 136T; 169C; 207A; 515A; 582G; 633C; 634G; 641A; 683A; 708T; 773T; 849C;
   888A; 911A; 928C.
*Epidorylaimus lugdunensis* (Qudsianematidae; [ ]; (LSU, 5'-end); AY593036) genus
   93G; 136C; 155A; 168T; 489G; 567T; 581A; 632T; 633A; 682G; 864T; 907C; 908G; 924C; 925G.
*Eudorylaimus sp.* (Qudsianematidae; [ ]; (LSU, 5'-end); AY593037) genus
   135T; 351G; 407T; 456A; 465G; 483T; 484T; 504A; 567A; 632A; 635T; 639T; 640T; 641A; 652A;
   740T; 853C; 919C; 920G; 922A; 924T; 959A; 1009G; 1040G.
*Thonus circulifer* (Qudsianematidae; [ ] (LSU, 5'-end); AY593038) species
   127A; 173A; 206T; 209A; 215C; 225C; 441T; 448T; 472T; 473G; 474T; 573G; 585A; 587T; 596A;
   603C; 668T; 695C; 696C; 704T; 733G; 776T; 784G; 831A; 866T; 866G; 890G; 914T; 922G; 939C;
   954T; 955T; 971G; 978T.
*Longidorella cf. macramphis* (Nordiidae; [pp] (?) (LSU, 5'); AY593042) species
   72A; 77C; 90G; 120C; 129A; 143D; 178A; 181C; 194T; 221G; 259G; 382A; 385G; 418C; 419G; 454C;
   455G; 456C; 462A; 471G; 475T; 487C; 497T; 518A; 567G; 585T; 613A; 615G; 638T; 648T; 673T;
   675A; 693T; 705C; 712A; 726C; 751A; 825G; 864C; 902G; 948C.
*Longidorella sp.2;* (Nordiidae; [pp] (?) (LSU, 5'); AY593044) species
   114T; 117A; 118T; 120C; 177A; 178A; 194T; 267C; 385T; 407G; 421C; 438C; 518G; 532G; 579C;
   580T; 587C; 588G; 662A; 665A; 666T; 697G; 701G; 703T; 704C; 705A; 714T; 717C; 769A; 858G;
   869G; 881T; 899C; 942T; 1097A.
*Microdorylaimus miser* (Qudsianematidae; [ ] (LSU, 5'-end); AY593046) species
   194C; 205C; 274A; 407A; 492G; 543A; 552T; 581C; 619T; 637G; 638A; 642T; 704T; 709A; 712T;
   720T; 739C; 874C; 881A; 884G; 905G; 963T; 1101G.
*Thonus* □*imiles* (Qudsianematidae; [ ] (LSU, 5'-end); AY593047) species
   200A; 421T; 449T; 454C; 456T; 457T; 465C; 474C; 476T; 491A; 549A; 631C; 632G; 635T; 666T;
   667G; 726G; 758T; 907T; 926C; 928A; 948A; 691T; 725C.
*Microdorylaimus modestus* (Qudsianematidae; [ ] (LSU, 5'-end); AY593049) species
   14G; 23C; 156G; 168C; 178C; 403A; 406T; 416C; 424A; 426A; 451A; 453G; 462G; 502G; 533A;
   546T; 547A; 567C; 589T; 601C; 605T; 606A; 610T; 611T; 613G; 618A; 623T; 633A; 635T; 652C;
   691A; 692T; 698A; 700C; 701G; 747T; 758C; 766A; 827C; 836C; 859T; 864C; 867G; 870G; 871C;
   873A; 898T; 899G; 923C; 950G.
*Pungentus engadinensis* (Nordiidae; [pp] (LSU, 5'); AY593050) species
   296A; 465T; 629A; 633C; 634G; 660T; 675G; 712G; 718C; 719T; 741A; 817G.
*Pungentus silvestris* (Nordiidae; [pp] (LSU, 5'); AY593052) species
   500T; 540T; 562T; 570A; 627T; 628T; 632A; 644T; 674A; 715A.
*Tylencholaimellus sp.* (Leptonchidae; [ ] (LSU, 5'-end); AY593055) genus
   61C; 62G; 67T; 68C; 89C; 90G; 91G; 93C; 97A; 98G; 99A; 100C; 103C; 104C; 105G; 143T; 151A;
   200G; 201C; 208A; 221A; 249G; 287A; 295C; 296T; 326T; 387G; 406G; 407T; 408A; 417A; 428G;
   429G; 434T; 437T; 441C; 443A; 444T; 445G; 446C; 447C; 449C; 453C; 454A; 456T; 457A; 459A;
   460G; 469C; 472A; 475C; 478G; 480T; 481A; 483C; 486A; 491G; 499A; 504C; 505G; 507G; 517C;
   524T; 530T; 532T; 579G; 580A; 585C; 587T; 592C; 593T; 594C; 611T; 620T; 622G; 641A; 642A;
   648G; 649C; 654T; 658A; 659T; 668C; 669G; 670C; 674G; 682T; 685T; 686A; 700C; 701G; 707C;
   729A; 730G; 734C; 735G; 754T; 755A; 757T; 768C; 769T; 785C; 788A; 802C; 857T; 858C; 876G;
   886G; 892G; 899A; 901C; 902C; 903G; 904T; 905C; 906A; 907A; 908A; 972A; 973G; 975G; 1016C.
*Longidorus dunensis* (Longidoridae; [pp] (LSU, 5'); AY593056) species
   76T; 94C; 100A; 107A; 118T; 135C; 136G; 137A; 139T; 141A; 143T; 144G; 154T; 156G; 165T;
   170G; 171T; 179G; 188A; 191C; 199A; 202C; 238A; 249T; 294G; 443G; 445C; 464T; 569T; 603G;
   677T.
*Longidorus intermedius* (Longidoridae; [pp] (LSU, 5'); AY593058) species
   569A; 570C; 571G; 581T; 599G.
*Mylonchulus* (Mylonchulidae I; [ ]; AY284751) genus
   43C; 75T; 90C; 91A; 97T; 144A; 154C; 163A; 202G; 236T; 422T; 430A; 453A; 455A; 477T; 608T;
   638T; 657C; 666T; 669C; 671T; 681C; 703C; 708T; 1002T; 1033A; 1072A; 1332T; 1333T; 1362G;
   1369T; 1457C; 1459T; 1462C; 1463G; 1493G; 1509A; 1562T; 1578A; 1638G; 1645A; 1665A; 1666C;
   1667G; 1679T; 1684T.
*Prionchulus* (Mononchidae I; [ ]; AY284745) genus
   43T; 78C; 90G; 91T; 104C; 135G; 192A; 291T; 307T; 313A; 317G; 319C; 337A; 421C; 422T; 453C;
   469G; 578C; 621T; 751T; 851A; 880A; 895T; 1065C; 1066T; 1273A; 1318A; 1361G; 1569C; 1625G;
   1626A; 1650A; 1657T; 1679T; 1694A.
*Clarleits* (Mononchidae I; [ ]; AY284748) genus
   23C; 43C; 78T; 90T; 91C; 104T; 192G; 201C; 227C; 337T; 454A; 468T; 484C; 490A; 563T; 574A;
   628C; 658C; 659G; 708G; 710A; 711T; 751C; 783C; 793C; 813G; 840C; 851T; 895A; 1015C; 1019C;
   1036G; 1126A; 1165A; 1316T; 1317G; 1323C; 1445A; 1488C; 1503A; 1506C; 1543T; 1554G; 1567T;
   1569C; 1576A; 1623A; 1624G; 1644C; 1648G; 1676G.
*Coomansus* (Mononchidae I; [ ]; AY284766) genus
   175A; 192A; 201T; 603A; 627A; 998T; 1333T.
*Granonchulus* (Mylonchulidae II; [ ]; AY593953) genus
   87A; 149A; 157G; 179A; 207A; 227T; 258A; 461C; 603G; 617G; 619A; 620A; 628C; 645T; 646C;
   672C; 699G; 945A; 1014A; 1037T; 1209G; 1319C; 1336T; 1338T; 1413T; 1456C; 1469C; 1517G;
   1556G; 1566C; 1654G; 1661T; 1666C; 1691T; 1692C; 1700C.
*Anatonchus* (Anatonchidae; [ ]; AY284768) genus
   142T; 257T; 461A; 466C; 494A; 495A; 1024G.
*Mononchus* (Mononchidae II; [ ]; AY284764) genus
   78T; 90T; 187G; 193T; 204T; 206G; 421T; 451G; 631G; 643G; 666G; 667G; 700T; 800C; 894G;
   1011T; 1022A; 1034A; 1036A; 1187G; 1363T; 1448C; 1453A; 1455T; 1460G; 1483G; 1552T; 1568A;
   1570C; 1652G; 1659G; 1660C; 1671G; 1673T; 1675T.
*Bathyodontus* (Bathyodontidae; [ ]; AY284744) genus
   92C; 117C; 131G; 505A; 510T; 547G; 554A; 555C; 559T; 574C; 900A; 1208A; 1224T; 1248T;
   1249G; 1326G; 1351C; 1354C; 1359G; 1362G; 1502G; 1549T; 1565T; 1567A; 1578G.
*Paramphidelus* (Alaimidae; [ ]; AY284741) genus
   33C; 46C; 94C; 163C; 237T; 257A; 279T; 327A; 350A; 428A; 618G; 621G; 622G; 632G; 633C; 641A;
   646C; 648A; 662G; 663A; 664C; 673A; 674G; 683T; 689A; 696C; 752C; 754T; 823T; 1017A; 1022A;
   1027T; 1028T; 1030G; 1041T; 1065A; 1324C; 1325G; 1330C; 1333C; 1335G; 1336G; 1339T; 1340C;
   1341G; 1342G; 1343C; 1344G; 1345T; 1365G; 1377C; 1447A; 1463G; 1482C; 1638C; 1656G;
   1658T; 1664T; 1665C; 1666T; 1670G; 1672A; 1680A; 1688A; 1695G; 1697T; 1698C; 1699T.
*Trischistoma* (Tripylidae I; [ ]; AY284735) genus
   33A; 34G; 40C; 47A; 93A; 168G; 205A; 206T; 230C; 610T; 617A; 660T; 661T; 662G; 663T; 688T;
   689C; 691G; 703A; 711G; 750T; 759G; 1067C; 1330C; 1334A; 1336C; 1340C; 1362A; 1374T;
   1457C; 1462T; 1657T; 1658C; 1659A; 1661A; 1675T; 1681G; 1682A; 1685A; 1696A; 1700T; 1705;
   1706C.
*Rhabdolaimus* (Rhabdolaimidae; [ ]; AY284710) genus
   22C; 35A; 48T; 71A; 131A; 132T; 345G; 351C; 357G; 451A; 477C; 480C; 493A; 498A; 499T; 501T;
   513T; 523C; 539T; 548C; 549T; 551T; 552A; 556C; 557G; 559T; 562A; 584A; 579A; 705A; 706T;
   830G; 835T; 890T; 906A; 957A; 967G; 1211A; 1243T; 1258T; 1331T; 1335G; 1338A; 1341G; 1343T;
   1344A; 1345A; 1346C; 1351G; 1357G; 1359A; 1372T; 1273A; 1444T; 1516G; 1521C; 1522C; 1539T;
   1554C; 1562A; 1565C; 1567G; 1582T; 1587A; 1589C; 1597G.
*Trichodorus variopapillatus* (Trichodoridae; [ ]; AY284841) species
   141G; 194T; 488G.
*Trichodorus* □*imiles* (Trichodoridae; [pp]; AY284840) species
   24A; 38C; 41G; 44C; 47A; 50A; 58C; 68T; 79A; 86T; 93G; 97A; 140C; 142A; 154G; 157A; 158G;
   189G; 206A; 209T; 216A; 225A; 237T; 243T; 248C; 255G; 268A; 269G; 300C; 301T; 314G; 315A;
   331G; 356G; 367T; 371C; 377G; 380T; 386T; 437G; 450C; 467G; 493C; 505T; 512G; 513A; 539A;
   555T; 556C; 558A; 560A; 561T; 563C; 567G; 568A; 570T; 571T; 572A; 57GT; 613C; 614T; 615C;
   516C; 623A; 627T; 634T; 641G; 643G; 663T; 672C; 673A; 678C; 679T; 783A; 784T; 785T; 809T;
   810A; 811T; 812A.
*Diphterophora* (Diphterophoridae; [pp]; AY284838) genus
   86A; 96A; 110G; 139G; 141C; 177T; 198T; 469T; 578T; 612C; 613T; 614G; 618T; 630T; 633G;
   636G; 641A; 646G; 647G; 747T; 748A; 757C; 777G; 790C; 812G; 1004A; 1005G; 1006A; 1013T;
   1023C; 1024T; 1031A; 1038G; 1039C; 1041T; 1043A; 1058C; 1059A; 1066A; 1100A; 1219A; 1311G;
   1323C; 1549A; 1622T; 1623T; 1667T; 1705C.
*Prismatolaimus* (Prismatolaimidae; [ ]; AY593957) genus
   141G; 162G; 181A; 259G; 420G; 701G; 790A; 791T; 819A; 820T; 1034T; 1046T; 1339T; 1475C;
   1630A; 1641C; 1648T; 1686A; 1693G.
*Bastiania* (Bastianiidae; [ ]; AY284725) genus
   27T; 33T; 41T; 75T; 76C; 85A; 86A; 89C; 91C; 94T; 141T; 149A; 150A; 162T; 181G; 204T; 207A;
   229G; 230A; 232A; 261G; 266C; 272A; 298A; 300C; 347T; 423T; 446C; 468A; 485A; 512A; 608T;
   619A; 620T; 637C; 663A; 664A; 671C; 675G; 676G; 679T; 688A; 700A; 701C; 702C; 708T; 711T;
   782G; 787A; 798T; 814A; 1008T; 1018A; 1026T; 1027A; 1029A; 1031C; 1035C; 1037T; 1047A;
   1095A; 1212T; 1227T; 1326C; 1329A; 1330T; 1362T; 1457T; 1458T; 1464A; 1475A; 1492C; 1505A;
   1508A; 1627G; 1628A; 1648A; 1659A; 1667T; 1668A; 1678A; 1685T; 1695T; 1696A; 1703G; 1706C.
*Paratripyla* (Tripylidae II; [ ]; AY284737) genus
   31C; 103C; 113C; 160A; 271C; 444G; 549A; 628T; 636A; 643T; 655A; 662C; 751A; 754C; 816G;
   817A; 818T; 1022A; 1031C; 1325A; 1333A; 1336T; 1337T; 1376T; 1458A; 1637C; 1641A; 1642T;
   1643A; 1670G; 1678G; 1679T; 1686T; 1687T; 1688C; 1695A; 1709T; 1710T.
*Tripyla* (Tripylidae II; [ ]; AY284731) genus
   31G; 87G; 94T; 95T; 110A; 112C; 118G; 120T; 217A; 237T; 257A; 258C; 273T; 281C; 313G; 315T;
   317T; 505A; 622A; 632T; 633T; 737G; 741A; 751T; 791C; 792C; 805A; 809T; 823A; 834A; 835T;
   917G; 925G; 1012A; 1014T; 1038G; 1071G; 1177A; 1321G; 1328A; 1329G; 1355G; 1413C; 1622G;
   1625C; 1626A; 1632A; 1633C; 1636G; 1637C; 1643T; 1645C; 1660A; 1684C; 1685A;
*Odontolaimus* (Odontolaimidae; [ ]; AY284723) genus
   10G; 26T; 65G; 78C; 79C; 87G; 304G; 308T; 312C; 461A; 538G; 638C; 650C; 673T; 684T; 698T;
   901A.

It is possible to add new information to the database or to remove information therefrom, a database with a dynamic character thus being obtained. By adding new relevant information, the size and the detail of the database will increase so that the results have an increasingly better basis.

The following GenBank Accession Numbers for the SSU or LSU rDNA sequences of individual nematodes have been generated by the present inventors and used in the alignment for identifying the SNPs according to the invention: AY593874, AY593881, AY284617, AY284608, AY284605, AY284607, AY284606, AY284609, AY593882, AY284612, AY284611, AY284610, AY284614, AY284621, AY593889, AY593895, AY593892, AY593899, AY593900, AY284595, AY284598, AY284602, AY284601, AY284600, AY284630, AY284632, AY284629, AY284625, AY284622, AY284587, AY284599, AY593904, AY593905, AY284597, AY284594, AY593906, AY593912, AY284637, AY593913, AY593914, AY284591, AY284592, AY284589, AY284590, AY284588, AY284583, AY284581, AY284584, AY593915, AY284586, AY284585, AY284638, AY593917, AY284639, AY284642, AY284668, AY284666, AY284662, AY284671, AY284672, AY284675, AY284677, AY284674, AY984678, AY284646, AY284644, AY284645, AY284643, AY284648, AY284651, AY593918, AY593920, AY284681, AY284682, AY284654, AY593921, AY284655, AY284660, AY284688, AY593923, AY593924, AY284661, AY284683, AY284707, AY593927, AY593928, AY284700, AY593930, AY284697, AY593931, AY593932, AY284686, AY284684, AY593935, AY284692, AY593938, AY284693, AY284724, AY593939, AY284714, AY593941, AY593942, AY592994, AY592996, AY592999, AY593001, AY593003, AY593005, AY593007, AY593008, AY593011, AY593012, AY593013, AY593015, AY593017, AY593020, AY593022, AY593023, AY593025, AY593027, AY593029, AY593030, AY593034, AY593036, AY593037, AY593038, AY593042, AY593044, AY593046, AY593047, AY593049, AY593050, AY593052, AY593055, AY593056, AY593058, AY284751, AY284745, AY284748, AY284766, AY593953, AY284768, AY284764, AY284744, AY284741, AY284735, AY284710, AY284841, AY284840, AY284838, AY593957, AY284725, AY284737, AY284731, AY284723, AY284618, AY284620, AY593873, AY593875, AY593876, AY593878, AY593879, AY593880, AY284613, AY284615, AY284616, AY593883, AY593884, AY593885, AY593886, AY593887, AY593888, AY593896, AY593890, AY593891, AY593893, AY593901, AY593902, AY284196, AY284603, AY284604, AY284631, AY284633, AY284634, AY284628, AY284626, AY284627, AY284623, AY284624, AY284635, AY593907, AY593908, AY593909, AY593910, AY593911, AY284582, AY284593, AY593916, AY284640, AY284641, AY284664, AY284665, AY284667, AY284669, AY284670, AY284663, AY284673, AY284676, AY284679, AY284680, AY284647, AY284649, AY284650, AY593919, AY284652, AY284656, AY284657, AY284658, AY284659, AY593922, AY284690, AY284689, AY593925, AY593926, AY593929, AY284706, AY284699, AY284704, AY284705, AY284701, AY284702, AY284703, AY284698, AY284696, AY284708, AY284709, AY593933, AY284687, AY593934, AY284685, AY284691, AY593936, AY593937, AY284695, AY284694, AY284715, AY284716, AY284719, AY284720, AY284721, AY284718, AY284717, AY593940, AY592995, AY592997, AY592998, AY593000, AY593002, AY593004, AY593006, AY593009, AY593010, AY593014, AY593016, AY593018, AY593019, AY593021, AY593024, AY593026, AY593028, AY593031, AY593032, AY593033, AY593035, AY593039, AY593040, AY593041, AY593043, AY593045, AY593048, AY593051, AY593053, AY593054, AY593057, AY593059, AY593060, AY593061, AY593062, AY284758, AY284759, AY284760, AY284761, AY284752, AY284753, AY284754, AY284755, AY284756, AY284757, AY284746, AY284747, AY284749, AY284750, AY284767, AY593953, AY284769, AY284765, AY284762, AY284763, AY593954, AY284743, AY284740, AY284742, AY284739, AY284738, AY284736, AY284711, AY284712, AY284839, AY593955, AY284727, AY284728, AY593956, AY593957, AY284729, AY284726 AY284732, AY284733, AY284734, and AY284730.

The following GenBank Accession Numbers for the SSU or LSU rDNA sequences of individual nematodes have also been used in the alignment for identifying the SNPs according to the invention: AF036592, AY043247, AY271723, AY286310, AY286312, AY286311, AY279544, AY279546, AY286308, AY286309, AY286313, AY279545, AF442189, AF442190, AF442198, U42342, AY268118, AF535867, AF248477, AF442192, AF442197, AF442200, AF442196, AF442191, U81578, AF535868, AY268120, AF442193, AY268121, AF442199, AY593894, AF442195, AF442194, AY268119, AF202157, AF202164, AF036586, AF202150, AF034390, AF202158, AF202160, AF202161, U81577, U81576, AF202148, AF202152, AF034391, AF202159, AF202162, U61760, AB067755, AB067760, AB067759, AF037369, AF202153, AF202154, AF036599, AF083007, AF202156, AF202165, U81579, AJ567385, AF202151, M84229, AJ417023, AF279916, U81581, AF036605, AJ417024, AF036604, AJ417021, AF202163, AJ417022, AF036591, U81587, AF082998, AF082995, AF083019, AF082994, U81588, U13936, AF083021, AF083020, U13934, AF083000, AF083027, U13933, U13935, AF083008, L04153, L04152, AF036597, AF036598, AY168856, AY168862, AY168859, AY168857, AY168858, AY168863, AY168864, AY168861, AY168860, AF036606, U01230, U81589, U81590, AF036593, AF083004, U13929, U13931, X03680, U13930, AF083006, AF083025, AF083026, U81586, AF083009, AF083024, AF083001, AF083012, U73449, U73452, AF083013, AF083016, AF083028, AF083014 , U13937, AF083015, AF083011, AF082999, AF083002, AF083018, U13932, AF082997, U81583, A083023, AF083022, AF083017, AF036643, AF036640, U81584, AF083010, U61759, U81582, AF082996, U81585, U94367, AF036587, U94366, M58348, U94378, U94369, U94368, U94383, U81575, U94365, U94380, U94381, U94372, U94377, U94375, U94376, U94374, U94370, U94382, AF036608, U94379, U94371, AF083005, U94373, AF227234, AF036588, AF036638, AF227233, U81574, AF083003, AF036589, AF036590, Z96948, Z96946, Z96947, AF202155, U61761, AF037628, AF036602, AF036644, AF036611, AF047889, AF202149, AF047891, Y16913, Y16923, AF036595, Y16911, Y16921, Y16917, Y16916, Y16915, Y16920, Y16922, Y16919, Y16918, Y16912, AF047888, AF036612, AF480074, AF480082, AF480081, AF480080, AF480079, AF480078, AF480077, AF480076, AF480075, AF480073, AF480072, AF480071, AF480083, U47561, AY210845, AF036596, AF036641, U60231, AF036637, U88336, Y16914, AF036642, AF047890, AF329937, AF036609, AJ438052, AF036600, AF036601, and AF036603.

A method for determining soil health according to the invention comprises the step of providing a ribosomal nucleic acid sample of the nematodes present in a sample of this soil. For this purpose, very suitably, a sample can be taken from a soil, after which nucleic acids of the nematodes occurring therein are isolated. Form and size of such a soil sample are not determinative for the present invention. Suitable soils which can be studied in embodiments according to the invention may comprise terrestrial soils, aquatic soils as well as marine soils. Preferably, a soil of which the soil health is determined by use of a method according to the invention is a soil in which nematodes naturally occur (incidentally, virtually no soil can be found on earth in which no nematodes occur).

In principle, of all possible species of nematodes occurring in the soil or in the soil sample, molecular features can be determined. The nematodes may occur in suitable soils in densities between 10 and 100,000 individuals per gram wet weight.

For determining molecular features in a nematode or in a plurality of nematodes, a device intended for this purpose according to the invention can be used. Such a device is described in more detail hereinbelow. In a method in which a device according to the invention is used, a suitable sample, which has been obtained from a soil to be studied, is, optionally after preprocessing thereof, contacted with a contact point (4) intended for this purpose of the measuring device (1).

In a method according to the invention, as molecular features of nematodes, preferably, genetic markers are used which can be detected by means of specific binding partners. Preferably, the SSU rDNA of a nematode is used as a genetic marker in a method according to the invention. Other suitable genetic markers for use in a method according to the invention are further defined hereinbelow. A skilled person himself may also, in a simple manner, identify suitable genetic markers and manufacture specific binding partners for this purpose in manners indicated herein.

Methods for identifying genetic markers connected with particular life strategies are known to a skilled person (see *inter alia* WO 01/83813). Thus, so-called polymorphisms can be detected according to methods described in US 6,300,063. Also, for this purpose, known genetic fingerprint methods can be used (see Mueller and Wolfenbarger for an overview), such as AFLP (Vos *et al*. 1995), RAPD, or RFLP (Botstein et al., 1980) or techniques derived therefrom such as Ribotyping.

In the present invention, genetic markers such as RFLP markers (see for instance U S 5,324,631), RAPDs (see for instance Aufauvre-Brown *et al*., 1992), AFLP markers (see for instance EP 0,534,858) SSR markers (see for instance US 5,075,217) and SNP (single nucleotide polymorphism) markers (McEwen et al., 2000) can be used. Preferably, single and oligonucleotide polymorphisms are used as genetic markers.

In an embodiment in which genetic markers are used, a method according to the invention preferably comprises a step in which the presence or absence of this genetic marker in a polynucleotide, such as a DNA or RNA, of a nematode is determined.

Genetic markers associated with a particular genetic feature may, for instance, be detected by means of amplification of nucleic acid sequences comprising the respective genetic marker. Such amplification methods, such as PCR or MLPA, make use of specific binding partners, such as oligonucleotide primers, and are known to a skilled person. Other amplification methods can also be used for detection of a genetic marker in a nematodes. Methods for the detection and characterization of amplified products, such as electrophoresis, chromatography, sequencing or mass spectrometry, are known to a skilled person.

Genetic markers may also be detected by the use of many different types of complementary binding partners. A skilled person will understand that, for detecting these genetic markers, nucleic acid probes which are able to detect the presence of the specific genetic marker in a nematode can very suitably be used. Preferably, for this purpose, nucleic acid or oligonucleotide probes are used which have the ability to hybridize selectively to the genetic markers associated with specific life strategies.

Genetic markers may further be detected by the use of a microarray, such as a DNA array, an oligonucleotide array or, in general terms, a nucleic acid array.

In an embodiment of a method according to the invention in which a nucleic acid array is used a contact point of a measuring device, nucleic acid of a nematode or fragments thereof is/are contacted with an array of specific binding partners for nucleic acid markers. Methods for obtaining genetic information with nucleic acid arrays are known in the literature (see *inter alia* Chee *et al*., 1996).

A method for determining soil health according to the invention further comprises the step of comparing the single nucleotide polymorphisms with corresponding features in the database for determining the life strategies of the plurality of lower taxonomic groups of nematodes in the soil.

The step of comparing molecular features in order to determine a correspondence between them should herein be understood as determining homology between two molecular features. The manner in which this can be determined depends on the type of the molecular feature. Polynucleotides, for instance, have "homologous" sequences when the sequence of the nucleotides is identical in the two sequences when they have been aligned for maximum correspondence as described herein. Sequence comparison between two or more polynucleotides is generally carried out by comparing parts of the two sequences in a comparison series ("window") to identify and compare local regions of sequence correspondence. The comparison series generally comprises approximately 20 to approximately 200 successive nucleotides. The "percentage sequence homology" for polynucleotides, such as 50, 60, 70, 80, 90, 95, 98, 99 or 100 percent sequence homology can be determined by comparing two optimally aligned sequences over a comparison series, in which the part of the polynucleotide sequence present in the comparison series may comprise additions or deletions ("indels") compared to the reference sequence (which does not comprise additions or deletions) for optimal alignment of the two sequences. The percentage is calculated by: (a) determining the number of positions where the same identical nucleic acid base occurs in both sequences, which results in the number of corresponding positions; (b) dividing the number of corresponding positions by the total number of positions in the comparison series; and (c) multiplying the result by 100, which results in the percentage sequence homology. Optimal alignment of sequences for comparison can be carried out by implementation of algorithms known for this purpose on a computer, or by visual inspection. Algorithms for comparing sequences and for aligning multiple sequences are the Basic Local Alignment Search Tool (BLAST) (Altschul, S.F. et al. 1990. J. Mol. Biol. 215:403; Altschul, S.F. et al. 1997. Nucleic Acid Res. 25:3389-3402) and ClustalW programs, respectively, both available on the Internet. Other suitable programs are GAP, BESTFIT and FASTA as part of the Wisconsin Genetics Software Package (Genetics Computer Group (GCG), Madison, WI, USA). For polypeptide sequences, similar programs are available.

If a molecular feature which has been determined for a nematode from a soil corresponds to a molecular feature (in this case SNP) in the present database, the life strategy correlating with this molecular feature can be assigned to the respective nematode. Here, it is important to recognize that, for determining correspondence between two molecular features in which two SNPs are compared it is not necessary that the respective SNP actually has the exact position (nucleotide position) as indicated in Table 7. Between different nematodes belonging to the same species, but coming from different populations, an insertion may have taken place on the basis of which the numbering has been shifted, although there is a 100°/ homology between the respective SNP positions. When determining the homologous position, a skilled person will also be able to determine whether the respective SNP, as referred to in Table 7, actually occurs in the nematode studied or in the nucleic acid sample studied.

If the molecular feature which has been determined for a nematode does not occur in the database according to the invention, it can still be added to it, or the life strategy of the molecular feature which shows the greatest homology with it can be assigned to it.

A method for determining soil health according to the invention finally comprises the step of translating the plurality of life strategies thus obtained into an indication for the soil health. A plurality of lifer strategies may *inter alia* be converted into an indication for the soil health as described in Bongers & Ferris. 1999. Trends in Ecology and Evolution 14: 224-229, where a Maturity Index and a Plant Parasite Index are assigned to the nematode fauna.

An important advantage of the present invention is that, now, nematodes in the juvenile stage (including juvenile stages J-2, J-3 and J-4) can also be included in an analysis for the determination of the soil health, so that a refinement of the analysis is achieved. Further, the speed of the analysis is increased and no expert in the field of morphological identification of nematodes is needed anymore.

A further important advantage of the present invention is that, by use of the method according to the invention, a much greater number of individual nematodes can be analyzed. While, with morphological identification by means of microscopic analysis, within a reasonable period of time (2 hours), a maximum of up to 150 individuals per sample are characterized, by means of the present invention, in a same sample, some thousands of nematodes can be analyzed within a shorter time, so that a considerable reduction of the sampling error is achieved.

In addition, the life strategies (*inter alia* to be expressed in the *c*-*p* value) may vary between genera within one family. In the morphological methods according to the state of the art, this intra-familiar variation is not taken into account for determining soil health. However, the use of molecular features correlated with life strategies according to the present invention is not limited to the family level. Relevant correlation s can be recognized down to genus level. In general, the ecological variation within genera is much smaller than within families. Because a method according to the present invention supports the identification down to the genus/species level, a determination of the soil health by means of a method of the present invention provides more reliable results.

In preferred embodiments of methods according to the invention, the molecular features are specific nucleotide sequences in the SSU rDNA of nematodes, but the LSU (large subunit) rDNA may also be used. Further, a preferred embodiment of a method according to the invention comprises determining the *c*-*p* value of nematodes as quantitative indication of the life strategy.

The present invention further relates to a device for determining soil health and/or for classifying a nematode according to life strategies.

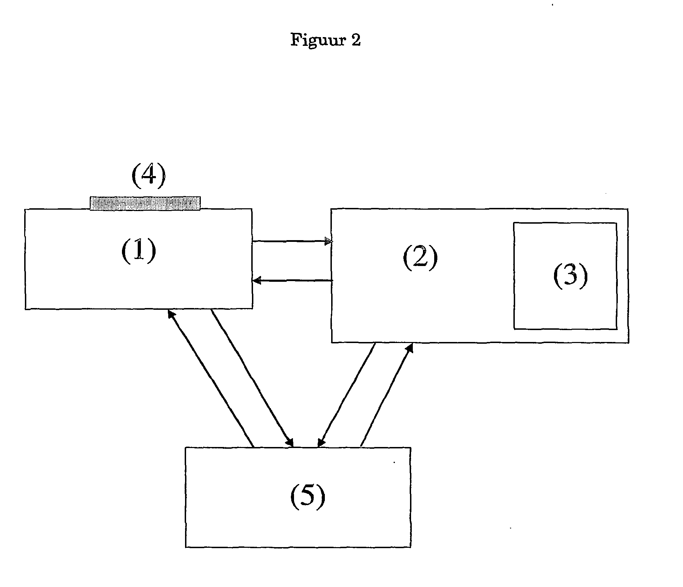

A device according to the invention comprises different parts (see Figure 2). A device according to the invention comprises a measuring device (1) for measuring a molecular feature (in this case a SNP) and a memory device (2) comprising a database (3) in which a molecular feature (in this case a SNP) of a nematode is correlated with a life strategy. The measuring device (1) and memory device (2) are provide with input and output means. The measuring device comprises at least one contact point (4) for measuring a molecular feature (in this case a SNP) of a nematode.

A device according to the invention further preferably comprises a central calculation unit (5) provided with input and output means for receiving and processing measuring results obtained by use of the measuring device (1), for comparing these measuring results with database data in the database (3), and for annotating a life strategy to a measured molecular feature (in this case a SNP).

It is not necessary that the measuring device (1), the central calculation unit (5) and the memory device (2) are physically integrated. A device according to the invention may comprise one or more detached devices.

A memory device (2) is preferably arranged for storing measuring results obtained by use of a measuring device (1) according to the invention.

A measuring device (1) for classifying a microorganism according to distinctive features according to the invention may comprise a single or multiple measuring device and may be designed such that it can be used to measure different or the same types of molecular features. This can be realized by the use of one or more types of contact points (4). A very suitable contact point (4) is formed by a microarray of specific binding partners.

If different types of molecular features of nematodes are measured by means of a device according to the invention, these features can be measured sequentially and/or simultaneously.

A device according to the invention can be used for determining a molecular feature of one single nematode, but also for determining molecular features of a plurality of nematodes. For instance, the molecular features of a nematode population as a whole can be determined from a soil sample.

A device according to the invention comprises a contact point (4) on the measuring device (1) with which a suitable sample, which has been obtained from a soil to be studied, optionally after preprocessing thereof, for determining molecular features in a nematode or a plurality of nematodes in this sample, can be contacted. Such a contact point may very suitably serve as input means on the measuring device. For this purpose, the contact point is provided with specific binding partners for binding to specific markers.

Preferably, in a measuring device for determining soil health according to the present invention, binding partners for genetic markers are used. Many genetic markers which are suitable for use in a device according to the invention are known to a skilled person. A skilled person himself may also, in a simple manner, identify suitable genetic markers and manufacture specific binding partners for this purpose.

In a preferred embodiment, a device comprises a nucleic acid array, which comprises, on a carrier surface, immobilized oligonucleotides or nucleic acid sequences which can specifically bind to genetic markers or complements thereof.

In order to improve the hybridization properties of oligonucleotides or nucleic acid sequences, specific nucleic acid analogs may be used which can enter into a sequence-specific interaction equivalent to that of the natural phosphodiester nucleic acid, such as phosphorothioate or methylphosphonate oligonucleotides, or peptide nucleic acid (PNA) oligonucleotides.

The manufacture of a nucleic acid array according to the invention can be carried out by means of methods known to a skilled person. The manufacture and the use of solid carrier nucleic acid arrays for the detection of specific nucleic acid sequences have often been described (US 5,571,639; Sapolsky et al., 1999, Genet. Anal.-Biomolecular Eng. 14, 187-192; Shena et al., 1995, Science 270, 467-470; Sheldon et al., 1993, Clinical Chem. 39, 718-719; Fodor et al., 1991, Science 251, 767-773).

A skilled person will be able to obtain arrays according to his own design and associated array reading equipment from specialized suppliers (for instance Affymetrix Corp., Santa Clara, CA, USA for nucleic acid arrays and Ciphergen Biosystems, Fremont, CA, USA for protein arrays).

A nucleic acid according to the present invention may for instance comprise between 10 and 200,00 oligonucleotides which have been immobilized on the solid carrier surface of the array and which are specific to particular sequences in the form of genetic markers.

It is advantageous if the melting point of the oligonucleotides is substantially in the same range to allow hybridization under uniform conditions.

Further, an array may comprise oligonucleotides which can detect a plurality of genetic markers such as SNP markers. Methods for designing sets of oligonucleotide probes for simultaneously analyzing nucleic acids, such as expression products of genes, have *inter alia* been described in EP 0,799,897. For the detection of SNPs by means of an optical thin-layer biosensor chip, see for instance Zhong et al., 2003 in Proc Natl Acad Sci U S A., Vol 100(20):11559-11564, which is explicitly referred to in the context of the present invention.

In particular, synthesis of the oligonucleotides can be carried out directly on the solid carrier surface of the array, such as for instance by means of a photochemical synthesis technique described in US 5, 424, 186 or with an inkjet technique. In an alternative embodiment, oligonucleotides can be synthesized *ex situ* and be connected to the solid carrier surface. In that case, it is advantageous if the carrier surface has been chemically modified prior to applying the oligonucleotides in order to allow binding between the oligonucleotides and the carrier surface, optionally by use of a hydrogel matrix or additional organic or inorganic linkers between the oligonucleotide and the carrier substrate of the array. Addressing the different oligonucleotides on the surface may be done electronically, mechanically or with an inkjet.

The hybridization conditions will depend on the nucleic acid used as sample material but can be optimized in a simple manner with methods known to a skilled person. For this purpose, *inter alia* the salt content, the pH and the temperature of the hybridization may be adjusted. Optionally, methods may be used to electronically control the stringency of the hybridization, as known from US G,017,696.

The detection of the hybridization may be carried out by means of labels such as radioisotope labels or fluorescent labels, by means of field effect measurements, by means of optoelectrochemical methods, piezoelectric methods, or ellipsometry, measurement with optical fibers or mass spectrometry. Also, telemetry may be used to study the presence of markers in the initial nucleic acid.

Prior to the hybridization, the genetic markers, such as nucleic acid fragments, can very suitably be labeled, for instance with a fluorescent label or a radioisotope or a different label, in order to facilitate the detection of these fragments hybridized to the oligonucleotides on the array. Depending on the method of detection chosen, a skilled person is able to use a suitable label.

Described herein are nucleotide positions in the SSU rRNA of nematodes which can very suitably be used as genetic markers. Preferably, the nucleotide positions according to Table 7 are used in methods according to the present invention. A skilled person himself will be able to identify, on the basis of additional sequence information of the ribosomal genes of nematodes, additional SNPs which can be used as genetic markers in the present invention for determining the life strategy of a nematode.

The present invention further provides a method for detecting a nematode with a specific life strategy. This method comprises detecting the genetically characteristic nucleotide sequences according to the invention in a nematode. This detection can be carried out by the use of one or more specific binding partners, such as nucleic acid probes, oligonucleotide probes, MLS probes or amplification primers, according to the invention.

Described herein is a composition in which one or more specific binding partners, preferably MLPA primers according to the invention, are comprised. Such a composition may very suitably be used in a method according to the invention in which a SNP according to Table 7 is detected. For designing specific probes and primers, use can suitably be made of relevant information about the occurrence of the nematode species in a particular soil type (see Table 6). This information is relevant because it indicates which nematodes can be expected in a particular soil. This is particularly valuable information with regard to cross reactivity of probes or primers: a (MLPA) probe, for instance, should be specific, but cross reactivity with nematodes which, for instance, do not occur on that soil type, in that region, or in that country is not disturbing and can be permitted in the design of the probe or primer.

Further, described herein is a kit for carrying out a method according to the invention, Such a kit comprises a composition with one or more specific binding partners, preferably MLPA primers according to the invention. Further, such a kit may comprise means for carrying out nucleic acid extractions, such as DNA extractions, DNA amplification reactions, DNA hybridization, DNA ligation and/or DNA detection.

The invention will now be illustrated in and by the following examples, which are not to be taken as being limitative.

### EXAMPLES

### Example 1. Combined morphological (digital images) and molecular (SSU rDNA sequence) characterization

The method followed comprised 9 steps in total. Steps 1 and 2 comprise the morphological characterization of the nematode. In steps 3-9, a molecular feature is determined, in this case a gene of taxonomic significance. For this purpose, the SSU rDNA is amplified, cloned and the nucleotide sequence of the cloned material is determined.

Step 1 comprises the isolation and morphological identification of the nematode. Step 2 comprises the morphological characterization of the nematode by means of digital image processing in order to be able to store these data (digital images) on a carrier and to have a (temporary) file for control and verification. Step 3 comprises a Proteinase K-based DNA extraction procedure. Step 4 comprises the amplification of the SSU rDNA by means of PCR. Step 5 comprises the cloning of the SSU rDNA fragments. During the procedure, the following parts were saved: digital images (step 2), raw DNA extract of the nematode (step 3), amplification products of the expected size (step 4) and stock solutions with the right clones in glycerol (step 5).

### Morphological characterization

Nematodes were extracted from the soil and individual nematodes were selected by hand and transferred into a small amount (50 µl) of water. Nematodes are generally very movable under the microscope and images can only be made if the organisms are temporarily anesthesized. For this purpose, the live nematode was transferred into a small amount of sodium azide solution on a slide for a microscope so that the nematodes were quickly immobilized for a period of approximately 2 hours for adult individuals and substantially shorter for juvenile individuals. The morphology of the nematode was studied and a maximum of 5 high-resolution digital photos were made under the microscope at a magnification of 1000x.

### DNA extraction of individual nematodes

Individual nematodes (comprising approximately 0.2 ng of DNA) were collected by means of a hair attached to a handle and were transferred into a 0.2-ml PCR reaction chamber which contained 25 µl of sterile water. Then, of the nematode, the DNA was isolated directly, or the reaction chamber with the nematode was stored at -20°C for later processing. An equal amount of lysis buffer (0.2 M NaCl; 0.2 M Tris-HCl pH 8.0; 1% (v/v) β-mercaptoethanol and 800 µg/ml proteinase-K) was added. The reaction chamber with the nematode therein was placed in a Thermomixer (Eppendorf, Hamburg, Germany) and incubated at 65 °C and 750 rpm for 2 hours, followed by incubation at 100 °C for 5 min.

### SSU rDNA amplification by means of PCR

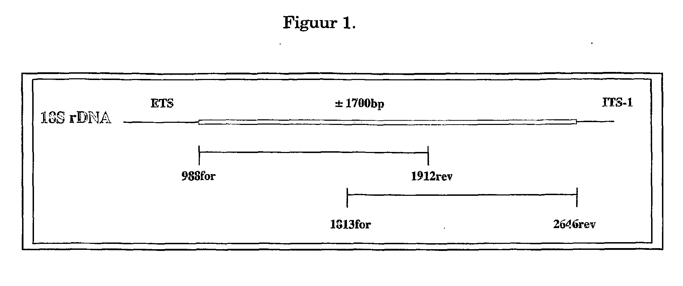

The SSU rDNA gene of the studied nematodes has a length of approximately 1700 base pairs (bp) and was amplified in two overlapping fragments by means of two primer sets: 988Forward (5'-CTCAAAGATTAAGCCATGC) in combination with 1912Reverse (5'-TTTACGGTYAGAACTAGGG); 1813Forward (5'-CKGCGYKAGAGGTGAAAT) in combination with 2646Reverse (5'-GCTACCTTGTTACGACTTTT). The primer 1912Reverse is a nematode-specific primer and was used to prevent the amplification of "adhesive" eukaryotic material, coming from, for instance, fungi and spores thereof, which adhere to the cuticles. The overlap between the two primer sets (see Figure 1) makes it possible to carry out an extra control whether the two amplified fragments belong to the same genotype.

### PCR amplification and electrophoresis

PCR amplification was carried out on a PTC-200 (MJ Research) thermal cycler. For this purpose, 3 µl of raw nematode extract was used directly or diluted 100 times with sterile water and Ready-To-Go-PCR Beads® (Amersham Pharmacia Biotech, Freiburg, Germany) were added. For the amplification of the whole length, two separate amplification reactions were carried out in which 50 ng of each primer were added per reaction. The PCR procedure comprised an initial denaturation step at 94°C for 5 min, followed by 5 cycles of 30 sec at 94 °C; 30 sec at 45 °C and 70 sec at 72 °C and 35 cycles of 30 sec at 94 °C; 30 sec at 54 °C; 70 sec at 72 °C and a last extension step of 5 min at 72 °C. Amplification products were stored at -20°C until further processing. A fraction of the PCR amplification products was loaded, together with a 1Kb⁺ marker (Gibco BRL), on a 1.2% agarose gel for electrophoresis stained with Gelstar (BMA, Rockland, ME, USA).

### Cloning and sequence determination

This step comprised standard cloning of specific SSU rDNA insertions cloned in a TOPO TA cloning vector 2.1 (Invitrogen, Groningen, The Netherlands). For sequence determination, standard primers were used (M13 Forward [5'-GTAAAACGACGGCCAG] and M13 Reverse [5'-CAGGAAACAGCTATGAC]. Sequence determination was carried out by means of the 'Sanger chain termination' method.

After collecting the sequence data, sequences were aligned by means of BioEdit version 5.0.9 (Hall, 1999). This program uses the ClustalW algorithm version 1.4 to align the sequences (Thompson et al., 1994). The standard settings of the program have been used for this. This alignment was completely visually checked afterwards and corrected where necessary.

In total, 266 SSU rDNA sequences were generated by means of the above method (GenBank Accession Numbers have been described hereinabove). These sequences were combined with 79 nematode sequences coming from the GenBank public domain database (GenBank Accession Numbers have been described hereinabove). A complete alignment was generated for all sequences. The total length of the alignment, including gaps, was 2165 bases. The complete alignment has not been inserted herein.

It was possible to recognize suborder, family, genus and species-specific sequences (SNPs). A large number of characteristic SSU rDNA nucleotide positions could be coupled to families, genera and species (see Table 7). For these families, genera and species, the specific life strategies are known.

In the case that multiplex ligation-dependent probe amplification (MLPA) technology is used for the detection of these polymorphisms, a probe consists of a set of two oligonucleotides. Each oligonucleotide consists of one PCR primer sequence, a stuffer sequence and a hybridization sequence. In one oligonucleotide, the 3' end (= the hybridization sequence) hybridizes to the target sequence; in the other oligonucleotide, the 5' end hybridizes. The outer three nucleotides of these hybridization sequences - so 6 nucleotides in total - are extremely critical to the amplification of the MLPA probe. Imperfect hybridization is the cause that no amplification product is formed. These short segments with one or a few taxon-specific nucleotides therein can therefore be used as genetic features in a database for use in a method and device according to the invention.

### Example 2. Compilation and design of the database

### (The use of SSU ribosomal DNA sequences for ecological classification of nematodes).

In total, more than 300 individual nematodes were identified, where possible down to genus level, but at least down to family level, on the basis of morphological features. Then, to the thus identified nematodes, the known life strategies were assigned (see Table 1). Then, for the molecular characterization of individual nematodes, of the same individuals, the 18S rRNA gene sequences (SSU rDNA) were determined by means of standard sequence methods as described hereinabove. For this purpose, the complete SSU rRNA genes (1700 bp) were sequenced. The thus generated molecular data of the individual nematodes were combined with corresponding publicly available sequences (GenBank; mainly of animal-parasitic nematodes and *Rhabditidae*). The combined set was aligned in order to (i) compare the newly generated data with the publicly available sequences, (ii) draw up a phylogenetic genealogical tree, and (iii) be able to identify family and genus-specific SSU rDNA domains for molecular characterization. A database was compiled in which, so far, some family and genus-specific SSU rDNA domains for molecular characterization of nematodes on the basis of the more than 300 individual nematodes were correlate with life strategies. Table 7 shows single SNPs or combination of (possibly successive) SNPs which were identified as family, genus and species-specific molecular features for the identification and detection of nematodes.

An advantage of the fact that a large number of the more than 300 individual nematodes which were characterized are plant parasites, including a number of notorious pests in agriculture, is that (a part of) the database which has been compiled can be used to make a risk analysis of crop damage caused by plant-parasitic nematodes. For this purpose, for instance, the soil could be analyzed prior to planting or sowing. In addition, it is possible to combine or integrate the data regarding the soil nematodes with corresponding data files of soil bacteria, soil fungi and other groups of soil inhabitants, in order to realize a soil life bioassay.

### Example 3. Isolation and detection of specific species, genera or families of nematodes in soil samples

For detection of nematodes in the soil, a soil sample of approximately 1.5 liters (approximately 2 kg) of soil is usually sufficient. For releasing the nematodes, generally, use is made of the fact that the settling velocity of nematodes differs from that of many other fractions in a soil sample. A skilled person is familiar with standard directions to release nematodes from soil samples.

### Sample refinement

For obtaining a nematode pellet from the soil, the following procedure can very suitably be used: the whole soil sample (approximately 1.5 1) is washed with water, after which the washing volume is reduced from 100 ml to 40-50 ml by means of siphoning off. After washing, the nematode samples are concentrated to a dry pellet. For this purpose, the samples are centrifuges for 10 minutes at 4000 rpm and the pellet with approximately 2 ml of supernatant fluid volume is dried overnight at 60°C.

### Lysis

The pellet is then resuspended in 205 µL of lysis mix (100 mM of NaCl; 100 mM of Tris-HCl (pH 8); 0.5% β-mercaptoethanol; 400 µg/mL of proteinase K), and the resuspended pellet is lysed for 2 hours at 65°C. The lysate is inactivated for 5 minutes at 100°C. The inactivated lysate with the nematode DNA therein is then directly analyzed or stored at -20°C for later analysis. *PCR*

The lysate is diluted 1:100 in sterile water, and 1.5 µL of this diluted lysate is added to 23.55 µL of PCR mix (2.5 µL 10x PCR buffer (100 mM Tris-HCl (pH 8), 500 mM KCl, 25 mM MgCl₂, 10% (v/v) Triton X-100); 1.25 µL dNTPs in water (@2.5 mM of each dATP, dCTP, dGTP en dTTP), 0.5 µL "Forward" primer (@ 5 µM); 0.5 µL "Reverse" primer (@ 5 µM); 0.1 µL SuperTaq DNA Polymerase (@5U/µL); Water (18.7 µL)). The samples are subjected to the following PCR cycle: 1x (94°C, 5 min); 38x (94°C, 20 sec; 65.5°C, 20 sec; 72°C, 40 sec); 1x (72°C, 5 min). Optionally, suitable positive, negative controls and blank controls may be included.

The nucleotide sequences of the "Forward" and "Reverse" primers used in the PCR reaction mixture can be chosen such that, under the given PCR conditions, annealing only occurs with the DNA of the desired species, the desired genus or the desired family of nematodes.

### Agarose gel electrophoresis

For analyzing the PCR products, use can be made of an agarose gel, for instance 3% agarose in TBE (Tris Borate EDTA). Concentrated TBE solutions are commercially available (for instance Roche 1.666.703). On the gel, the length of the PCR fragment can be controlled by the use of a standard marker, for instance commercial DNA Molecular Weight marker VI (0.15 to 2.1 Kbp) from Roche (1 062 590). A skilled person is familiar with the loading of PCR products on gels and the staining of DNA fragments therein.

### Example 4. Use of a thermostable DNA ligase for the detection of SNPs

DNA ligase is an enzyme which catalyzes the formation of a phosphodiester binding between a 3' hydroxyl group at the end of one DNA chain and a 5' phosphate group at the end of another (second) DNA chain. DNA ligase is not capable of connecting two single-strand DNA chains with each other; it only closes single gaps in double-stranded DNA. Bacterial DNA ligases use NAD+ as an energy source for this, while eukaryotes use ATP instead. This enzyme is used in the following manner in the detection of SNPs.

The template (the nucleic acid to be detected) with a characteristic SNP therein (in this case a T) is hybridized with two successive probes and ligated with a thermostable ligase.
5'-----G-C-A-T-A-A-----3' (Template)
3' (Probe 1)---C-G-T A-T-T-----5' (Probe 2)

A ligase usable for SNP detection should meet the requirement that a phosphodiester binding is formed between probe 1 and probe 2 only when there is a perfect match. Different types of conventional DNA ligases have a moderate to reasonable specificity (a mismatch of an end nucleotide to one of the probes does not necessarily mean that no ligation takes place), and are therefore not suitable for SNP detection. Therefore, preferably, heat-stable ligases which are active in a temperature range of 50-65°C (Ligase-65; MRC Holland, Amsterdam, The Netherlands) of 65-95°C (Ampligase; Epicentre, Madison USA) are used. Heat-stable ligases allow ligation to take place under stringent hybridization conditions (= relatively high temperature). In addition, heat-stable ligases have been found to be very specific (see for instance Luo *et al*. 1996).

A number of SNP detection technologies have been described which make use of heat-stable, specific DNA ligases. A limited number of these are suitable for the parallel detection of tens of SNPs. At this moment, two systems appear to meet the desired strongly multiplex character of the intended assay: the MLPA technique (Multiplex ligation-dependent probe amplification; Schouten *et al.* (*supra*)) and a biosensor chip as described by Zhong. *et al.* (*supra*).

In the MLPA technique, probe 1 consists of a hybridization sequence and a PCR primer sequence Y, and probe 2 consists of a hybridization sequence, a "stuffer" sequence and a PCR primer sequence X. The stuffer is variable in length and serves to separate the amplification products on the basis of differences in size. A PCR amplification product is only forme d when probe 1 and probe 2 are covalently connected with each other by means of a heat-stable ligase. Some tens of SNPs can semi-quantitatively detected in parallel.

In the biosensor chip of Zhong *et al.* (2003) (*supra*), PCR amplicons are used as a template. Then, probe 2 (with nucleotide at the end which corresponds to the SNP to be detected) is covalently bound to a substrate (the chip). Probe 1 (biotin labeled with a view to detection) and the denaturated target DNA (for instance a mixture of PCR amplicons) are spotted on the chip. The single-stranded target DNA ensures that the ends of probe 1 and probe 2 come near to each other. A piece of double-stranded DNA with single gap now forms. In the case of a perfect match, this is (covalently) closed by the heat-stable ligase. Then, the chip is washed, thus removing the target DNA. Probe 2 is now physically bound to probe 1 and the biotinylized end is detected. Some hundreds of SNPs can be detected in parallel in a qualitative manner.

## Claims

1. A method for detecting a lower taxonomic group of nematodes with a specific life strategy in a sample, comprising the steps of:
a) providing a database in which the single nucleotide polymorphisms (SNPs) according to Table 7 are correlated with the life strategy of the lower taxonomic group of nematodes;
b) providing a ribosomal nucleic acid sample of the nematodes present in said sample;
c) detecting a ribosomal nucleic acid comprising at least one single nucleotide polymorphism (SNP) according to Table 7 in said ribosomal nucleic acid sample thereby demonstrating the presence in said sample of said lower taxonomic groups of nematodes; and
d) determining, from the database, the life strategies of said lower taxonomic group of nematodes in said sample on the basis of the SNPs detected in step c.

2. The method of claim 1, wherein said life strategy relates to the reproductive strategy; the life cycle strategy; the ability to colonize; the sensitivity to disturbances; the usual population density or abundance; the area of distribution; the pathogenicity; the host plant spectrum; the vigor; or the persistence.

3. The method of claim 2, wherein said disturbances include acute exposure to toxic compounds or an increasing availability of easily degradable organic substances.

4. A method for determining the soil health of a soil, comprising the steps of:
performing the method of claim 1, wherein step c) comprises detecting a plurality of ribosomal nucleic acids each comprising at least one single nucleotide polymorphism (SNP) according to Table 7 in said ribosomal nucleic acid sample for demonstrating the presence of a plurality of lower taxonomic groups of nematodes in said sample;
wherein step d) comprises determining, from the database, the life strategies of said plurality of lower taxonomic groups of nematodes in said soil on the basis of the SNPs detected in step c; and wherein said method further comprising the step of
e) translating the plurality of life strategies thus obtained into an indication for the soil health.

5. A method according to claim 4, wherein said indication for the soil health is the 'Maturity Index'.

6. A device for determining soil health and/or for classifying a nematode according to life strategies, said device comprising a measuring device for measuring an SNP, and a memory device comprising a database wherein the single nucleotide polymorphisms (SNPs) referred to in Table 7 are correlated with life strategies of said nematode, wherein the measuring device and memory device are provided with input and output means and wherein the measuring device comprises at least one contact point for measuring an SNP of a nematode.

## Patentansprüche

1. Verfahren zum Detektieren einer niedrigen taxonomischen Gruppe von Nematoden mit einer bestimmten Lebensstrategie in einer Probe, das die folgenden Schritte umfasst:
a) Bereitstellen einer Datenbank, in der die Einzelnukleotid-Polymorphismen (SNPs) gemäß Tabelle 7 mit der Lebensstrategie der niedrigen taxonomischen Gruppe von Nematoden korreliert sind;
b) Bereitstellen einer Ribosomen-Nukleinsäure-Probe der Nematoden, die in der Probe vorhanden ist;
c) Detektieren einer Ribosomen-Nukleinsäure, die wenigstens einen Einzelnukleotid-Polymorphismus (SNP) gemäß Tabelle 7 in der Ribosomen-Nukleinsäure-Probe enthält, wodurch das Vorhandensein in der Probe der niedrigen taxonomischen Gruppen von Nematoden nachgewiesen wird; und
d) Bestimmen der Lebensstrategien der niedrigen taxonomischen Gruppe von Nematoden in der Probe anhand der in Schritt c) detektierten SNPs aus der Datenbank.

2. Verfahren nach Anspruch 1, wobei die Lebensstrategie mit der Reproduktionsstrategie; der Lebenszyklusstrategie, der Koloniebildungsfähigkeit; der Empfindlichkeit gegenüber Störungen; der gewöhnlichen Populationsdichte oder Häufigkeit; dem Verteilungsbereich; der Pathogenizität; dem Wirtspflanzenspektrum; der Vitalität; oder dem Beharrungsvermögen in Beziehung steht.

3. Verfahren nach Anspruch 2, wobei die Störungen ein plötzliches Ausgesetztsein von toxischen Verbindungen oder eine erhöhte Verfügbarkeit einfach abbaubarer organischer Substanzen umfassen.

4. Verfahren zum Bestimmen der Bodengesundheit eines Bodens, das die folgenden Schritte umfasst:
Ausführen des Verfahrens nach Anspruch 1, wobei der Schritt c) das Detektieren mehrerer Ribosomen-Nukleinsäuren umfasst, wovon jede wenigstens einen Einzelnukleotid-Polymorphismus (SNP) gemäß Tabelle 7 in der Ribosomen-Nukleinsäurenprobe enthält, um das Vorhandensein mehrerer niedriger taxonomischer Gruppen von Nematoden in der Probe nachzuweisen;
wobei der Schritt d) das Bestimmen der Lebensstrategien der mehreren niedrigen taxonomischen Gruppen von Nematoden in dem Boden anhand der im Schritt c) detektierten SNPs aus der Datenbank umfasst; und wobei das Verfahren ferner den folgenden Schritt umfasst:
e) Übersetzen der mehreren Lebensstrategien, die auf diese Weise erhalten werden, in eine Angabe der Bodengesundheit.

5. Verfahren nach Anspruch 4, wobei die Angabe der Bodengesundheit der "Maturity Index" ist.

6. Vorrichtung zum Bestimmen der Bodengesundheit und/oder zum Klassifizieren eines Nematoden in Übereinstimmung mit Lebensstrategien, wobei die Vorrichtung eine Messvorrichtung zum Messen eines SNP sowie eine Speichervorrichtung umfasst, die eine Datenbank enthält, in der Einzelnukleotid-Polymorphismen (SNPs), auf die in Tabelle 7 Bezug genommen wird, mit Lebensstrategien des Nematoden korreliert sind, wobei die Messvorrichtung und die Speichervorrichtung mit Eingabe- und Ausgabemitteln versehen sind und wobei die Messvorrichtung wenigstens einen Kontaktpunkt zum Messen eines SNP eines Nematoden umfasst.

## Revendications

1. Procédé permettant de détecter dans un échantillon un groupe taxinomique inférieur de nématodes ayant une stratégie vitale spécifique, comprenant les étapes consistant à :
a) fournir une base de données dans laquelle les polymorphismes mononucléotidiques (SNP) selon le Tableau 7 sont corrélés avec la stratégie vitale du groupe taxinomique inférieur de nématodes ;
b) fournir un échantillon d'acides nucléiques ribosomiques des nématodes présents dans ledit échantillon ;
c) détecter dans ledit échantillon d'acides nucléiques ribosomiques un acide nucléique ribosomique comprenant au moins un polymorphisme mononucléotidique (SNP) selon le Tableau 7, démontrant ainsi la présence dans ledit échantillon desdits groupes taxinomiques inférieurs de nématodes ; et
d) déterminer, à partir de la base de données, les stratégies vitales dudit groupe taxinomique inférieur de nématodes présent dans ledit échantillon sur la base des SNP détectés à l'étape c.

2. Procédé selon la revendication 1, dans lequel ladite stratégie vitale se rapporte à la stratégie reproductive ; la stratégie de cycle de vie ; la capacité de colonisation ; la sensibilité aux perturbations ; la densité habituelle de population ou abondance ; la zone de répartition ; la pathogénicité ; le spectre de plantes hôtes ; la vigueur ; ou la persistance.

3. Procédé selon la revendication 2, dans lequel lesdites perturbations comprennent l'exposition aiguë à des composés toxiques ou une augmentation de la disponibilité de substances organiques facilement dégradables.

4. Procédé permettant de déterminer la santé d'un sol, comprenant les étapes consistant à :
mettre en oeuvre le procédé selon la revendication 1, l'étape c) consistant à détecter dans ledit échantillon d'acides nucléiques ribosomiques une pluralité d'acides nucléiques ribosomiques comprenant chacun au moins un polymorphisme mononucléotidique (SNP) selon le Tableau 7 afin de démontrer la présence d'une pluralité de groupes taxinomiques inférieurs de nématodes dans ledit échantillon ;
l'étape d) consistant à déterminer, à partir de la base de données, les stratégies vitales de ladite pluralité de groupes taxinomiques inférieurs de nématodes présents dans ledit sol sur la base des SNP détectés à l'étape c ; et ledit procédé comprenant en outre l'étape consistant à
e) traduire la pluralité de stratégies vitales ainsi obtenues en une indication de la santé du sol.

5. Procédé selon la revendication 4, dans lequel ladite indication de la santé du sol est l' « Indice de Maturité ».

6. Dispositif permettant de déterminer la santé du sol et/ou de classifier un nématode en fonction des stratégies vitales, ledit dispositif comprenant un dispositif de mesure permettant de mesurer un SNP, et un dispositif de mémoire comprenant une base de données, les polymorphismes mononucléotidiques (SNP) figurant dans le Tableau 7 étant corrélés avec les stratégies vitales dudit nématode, le dispositif de mesure et le dispositif de mémoire étant dotés de moyens d'entrée et de sortie et le dispositif de mesure comprenant au moins un point de contact permettant de mesurer un SNP d'un nématode.
